# EUROPEAN PATENT SPECIFICATION

(11) **EP 3 072 887 B1**
(45) Date of publication and mention of the grant of the patent: **18.10.2017**
(21) Application number: 16162116.4
(22) Date of filing: 24.03.2016
(51) Int. Cl.: C07D 221/18, C09K 11/06, H01L 31/048, H01L 31/055, H01L 33/50, C07D 241/00, H01L 51/00, H05B 33/14, C07D 487/06, H01L 51/52, H01L 33/00

(54) **N,N'-BIS(2,6-DIISOPROPYLPHENYL)-1,7-DI(2,6-DIPHENYLPHENOXY)PERYLENE-3,4;9,10-TETRACARBOXIMIDE, N,N'-BIS(2,6-DIISOPROPYLPHENYL)-1,6-DI(2,6-DIPHENYLPHENOXY)PERYLENE-3,4;9,10-TETRACARBOXIMIDE AND THE USE THEREOF**
N,N'-BIS(2,6-DIISOPROPYLPHENYL)-1,7-DI(2,6-DIPHENYLPHENOXY)PERYLEN-3,4;9,10-TETRACARBOXIMID, N,N'-BIS(2,6-DIISOPROPYLPHENYL)-1,6-DI(2,6-DIPHENYLPHENOXY)PERYLEN-3,4;9,10-TETRACARBOXIMID UND VERWENDUNG DAVON
N, N'-BIS (2,6-DIISOPROPYLPHENYL) -1,7-DI (2,6-DIPHENYLPHENOXY) PERYLENE-3,4; 9,10-TETRACARBOXIMIDE, N, N'-BIS (2,6-DIISOPROPYLPHENYL) -1,6-DI (2,6-DIPHENYLPHENOXY) PERYLENE-3,4; 9,10-TETRACARBOXIMIDE ET SON UTILISATION

(30) Priority: 26.03.2015 EP 15161081; 01.06.2015 EP 15170126
(43) Date of publication of application: 28.09.2016
(73) Proprietor: BASF SE, 67056 Ludwigshafen am Rhein (DE)
(72) Inventor: KOENEMANN, Martin, 68199 Mannheim (DE); WAGENBLAST, Gerhard, 67157 Wachenheim (DE); IVANOVICI, Sorin, 69126 Heidelberg (DE); MITGUDE, Martina, 67269 Gruenstadt (DE); CARVALHO, Mary-Ambre, 42110 Civens (FR)

(56) References cited:
- EP-A1- 1 868 419
- WO-A1-2012/113884
- WO-A1-2012/152812
- WO-A1-2014/131628
- DE-A1-102005 037 115
- KR-A- 20140 147 037
- MEI-JIN LIN ET AL: "Bay-substituted perylene bisimide dye with an undistorted planar scaffold and outstanding solid state fluorescence properties", CHEMICAL COMMUNICATIONS, vol. 48, no. 99, 1 January 2012 (2012-01-01), page 12050, XP055205899, ISSN: 1359-7345, DOI: 10.1039/c2cc36719j

## Description

### FIELD OF THE INVENTION

The present invention relates to N,N'-bis(2,6-diisopropylphenyl)-1,7-di(2,6-diphenylphenoxy)perylen-3,4;9,10-tetracarboximide, N,N'-bis(2,6-diisopropylphenyl)-1,6-di(2,6-diphenylphenoxy)perylen-3,4;9,10-tetracarboximide, to a method for preparation and use thereof. In particular, the present invention relates to the use of said compound(s) in color converters for improving the luminous efficacy of white-light emitting diodes. The present invention also relates to the use of these color converters and to lighting devices comprising at least one LED or OLED and at least one color converter.

LEDs (light-emitting diodes, LEDs) and OLEDs (organic light-emitting diodes, OLEDs) are an important class of devices that convert electric energy into light. LEDs are attractive candidates to replace conventional light sources such as incandescent lamps and fluorescent lamps. Because of their low energy consumption, LEDs are increasingly being used as a light source for general lighting, for example in offices and residences, or for architectural lighting, in information signs, small appliances, and in the automobile and aircraft industries. Light emission is based on the recombination of electron-hole pairs (excitons) in the junction region of a pn junction poled in forward direction in a semiconductor. The size of the band gap of this semiconductor determines the approximate wavelength of the light emitted. In order to generate a particular color, LEDs with different band gaps can be combined to form a multi-LED.

Alternatively, a radiation converting luminophore (also referred to as phosphor, or fluorescent colorant or fluorescent dye; in the context of the present invention, these terms are used interchangeable to describe a material that converts light of a first wavelength into light of a second wavelength) can also be combined with a LED. In this context, the radiation emitted by the LED is partly absorbed by the radiation converting luminophore, which is thus induced to photoluminesce. The resulting light color of the LED results from the proportion of non-absorbed and transmitted LED light and the emission spectrum and intensity of the radiation converting luminophore. According to one approach called "phosphor on a chip", a polymeric material comprising a radiation converting luminophore is directly applied onto the LED light source (LED chip). The polymeric material is applied onto the LED chip, for instance, in droplet form or in hemispherical form, as a result of which particular optical effects contribute to the emission of the light. In phosphor on a chip LEDs, the radiation converting luminophores used are generally inorganic materials. Organic luminophore materials are not suitable, because the polymeric material and the radiation converting luminophore are subject to relatively high thermal stress and radiation stress.

In another approach called "remote phosphor", the color converter (also referred to as "converter" or "light converter"), which generally comprises a polymer layer and one or more radiation converting luminophore(s), is spatially separated from the LED source.

The spatial distance between the primary light source, the LED, and the color converter reduces the stress resulting from heat and radiation to such an extent that organic fluorescent dyes can also be used as radiation converting luminophores. Furthermore, LEDs according to the "remote phosphor" concept are more energy-efficient than those according to the "phosphor on a chip" concept. The use of organic fluorescent dyes in these converters offers various advantages, e.g., the composition/hue of the light can be precisely adjusted by applying optimized amounts of fluorescent dyes. Therefore, the broad emission spectra of fluorescent dyes allow to get white light with a high color rendering index.

White light-emitting LEDs are used in a wide range of applications as a light source or as a backlight in full-color displays including in flat panel display applications due to their long lifetime, high reliability and low power consumption. Two methods are commonly used to create white light with LEDs. The basis for the emission of white light is always the superimposition (mixing) of various colors. The first approach is through the combination of so called multi-LEDs, usually red, green, and blue ones. Because of the different brightnesses and operating conditions for the various light-emitting diodes, the multi-LED is technically complex and therefore expensive. Moreover, component miniaturization of the multi-LED is severely limited.

The second approach is through the combination of a blue LED with a converting material (phosphor) through luminescence converting. The light source appears white to an human observer, if a portion of the blue light generated from a blue LED is converted to yellow light. A conventional converter material is an inorganic converter material such as cerium-doped yttrium aluminum garnet (also referred to as Ce:YAG). A standard Ce:YAG-based white LED emits cold-white light, where the red component in the spectrum is absent. Generation of warm-white light is not possible with that configuration (without adding a red light emitting phosphor). Therefore, the light of a standard Ce:YAG-based white LED is often seen as unattractive.

In place of conventional inorganic phosphors, organic phosphors, namely organic fluorescent dyes, can also be used. Organic fluorescent dyes are advantageous in that they allow to create a warm-white light LED light since the fluorescent dye allows to tailor the hue of the light. In addition, organic fluorescent dyes exhibit much higher absorptivity compared to inorganic phosphors and are available at low cost. No expensive materials such as rare-earth metals are needed. Although a great variety of organic fluorescent dyes have been available, they often exhibit low fluorescence quantum yields in a polymer matrix and/or unsufficient photostabilities and/or are sensitive to moisture and oxygen. Therefore, there is an ongoing need for organic fluorescent dyes having high quantum yields in the polymer matrix and excellent stabilities under the practical application conditions.

The use of color converting layers is also a known method for generating a spectrum of a desired light color or light color mixture from the nearly monochromatic radiation that is primarily generated by the OLED. An object is also to generate white light and, in particular, light approximated to daylight, which is best suited for lighting purposes.

WO 2007/006717 relates to perylene tetracarboximides, perylene dicarboximides, the corresponding acid anhydrides and the higher homologs. The use of these compounds as fluorescent dye in color converters comprising at least one polymer as a matrix material is not described. None of the concrete compounds carries a 2,6-diphenylphenoxy substituent at the core of the perylene skeleton.

N,N'-bis(cyclohexyl)-1,7-di(2,6-diphenylphenoxy)peylene-3,4;9,10-tetracarboximide is known from Frank Würthner et al, Chem. Commun., 2012, vol. 48, pp 12050-12052. The use of this compoundsas fluorescent dye in color converters comprising at least one polymer as a matrix material is not described

KR 2014 0147037 describes the preparation of 1,7-diphenoxy -substituted N,N'-bis(2,6-diisopropylphenyl)-perylene-3,4;9,10 tetracarboximide compounds. None of the disclosed compounds bears 2,6-diphenylphenoxy substituents at the 1,7 bay positions.

FR 2 089 618 relates to benzoxanthene and benzothioxanthenedicarboxylic acid triazinyl-imide dyestuffs.

FR 2 194 827 relates to a process for the continuous dyeing of a textile material consisting of or containing synthetic fibres from an organic solvent wherein the fibrous material is impregnated with a dyeing liquor which contains as dyestuff a 1-nitro-6-alkoxy-benzothioxanthene-3,4-dicarboxylic acid imide.

In recent years, several classes of luminescent materials such as inorganic colorants and organic dyes have been described for use in color converters wherein the color converters contain a colorant/dye that is dispersed/dissolved into a polymer layer and is able to absorb the primary radiation of the LED or OLED and to generate a longer-wave secondary radiation.

EP 1 868 419 describes a concept for a color converter comprising a first perylene fluorescent dye, a second fluorescent dye and optionally, a third fluorescent dye. The first perylene fluorescent dye emits red fluorescence (wavengths of 580 to 700 nm) and the second fluorescent dye absorbs light in a ultraviolet to blue region (wavelengths of 300 nm to 500 nm) to emit light having a longer wavelength than the absorbed light (wavelength of 430 nm to 575 nm, for example), wherein the first perylene fluorescent dye absorbs light containing the fluorescence emitted by the second fluorescent dye. The optional third fluorescent dye can absorb light from the second fluorescent dye and transmit light of longer wavelength to the first perylene fluorescent dye. The first perylene fluorescent dye is a compound of formula (A) 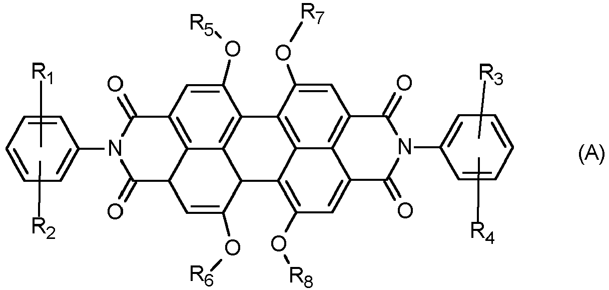 wherein R₁ to R₄ are each hydrogen, a linear alkyl group, a branched alkyl group or a cycloalkyl group, and may be substituted; R₅ to R₈ are a phenyl group, a heteroaromatic group, a linear alkyl group or a branched alkyl group, and may be substituted, and R₁ to R₈ may be the same or different. Specifically, the second fluorescent dye is a perylene fluorescent dye, coumarin dye, phthalocyanine dye, stilbene dye, cyanine dye, polyphenylene dye or rhodamine dye, preferably a core-unsubstituted perylene bisimide compound carrying on the imide nitrogen atoms a substituent selected from hydrogen, alkyl, and cycloalkyl. The third fluorescent dye is also a core-unsubstituted perylene bisimide compound, carrying on the imide nitrogen atoms a phenyl group optionally substituted by alkyl or cycloalkyl.

WO 2012/113884 and WO2012/152812 describe color converters comprising at least one perylene tetracarboximide as fluorescent dye such as N,N'-bis(2,6-diisopropylphenyl)-1,7-di(2,6-diisopropylphenoxy)perylene-3,4:9,10-tetracarboximide, N,N'-bis(2,6-diisopropylphenyl)-1,6-di(2,6-diisopropylphenoxy)perylene-3,4:9,10-tetracarboximide, or N,N'-bis(2,6-diisopropylphenyl)-1,6,7,12-tetraphenoxyperylene-3,4:9,10-tetracarboximide.

WO 2012/042438 describes color converters comprising perylene tetracarboximides in a polymeric material comprising a polyester having an aromatic moiety incorporated in the polymer backbone. The perylene tetracarboximide compounds carry up to four substituents selected from fluorine, methoxy, unsubstituted or substituted alkyl at the core of the perylene skeleton.

WO 2012/168395 describes color converters comprising at least one polymer and at least one organic fluorescent dye, wherein the organic fluorescent dye comprises at least one structural unit of the formula (A) 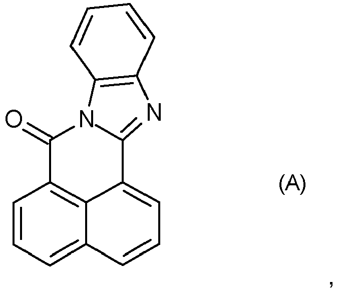 where the structural unit may be mono- or polysubstituted by identical or different substituents and where one or more CH groups in the six-membered ring of the benzimidazole structure shown may be replaced by nitrogen.

WO 2015/019270 describes cyanated naphthalene benzimidazole compounds and their use as fluorescent dye, in particular in color converters for blue LEDs.

WO 2015/169935 (PCT/EP2015/060137, unpublished at the priority date of this application) describes cyanated perylene compounds and their use as fluorescent dye, in particular in color converters for blue LEDs.

WO 2014/131628 describes lighting devices comprising (i) a blue LED as light source and (ii) a color converter comprising a polymer matrix and as organic luminescent material a non-cyanated benzoxanthene or benzothioxanthene compound.

Subject matter of unpublished EP 15161081.3 are cyanated benzoxanthene compounds and cyanated benzothioxanthene compounds and derivatives thereof and their use as organic luminescent material, in particular as fluorescent dye in color converters comprising at least one polymer as a matrix material. This reference also describes color converters comprising a combination of cyanated benzoxanthene compounds and/or cyanated benzothioxanthene compounds with further organic fluorescent dyes. N,N'-bis(2,6-diisopropylphenyl)-1,7-di(2,6-diphenylphenoxy)perylene-3,4;9,10-tetracarboximide or N,N'-bis(2,6-diisopropylphenyl)-1,6-di(2,6-diphenylphenoxy)perylene-3,4;9,10-tetracarboximide or a mixture thereof are mentioned as suitable combination partners.

Some of the known organic fluorescent dyes are unsatisfactory in terms of their light emitting properties, in terms of their poor fluorescence quantum-yield and light-fastness or in terms of their durability with respect to light emitted from a light source in the wavelength range from 400 to 500 nm or with respect to their high sensitivity towards oxygen and moisture. Furthermore, some of the known color converters comprising a combination of different organic fluorescent dyes suffer from different aging of the organic fluorescent dyes which cause prematurely undesirable color variation of the resulting total spectrum emitted. A further disadvantage of known color converters is that merely a small portion of the photons from the LED or OLED is converted into light of a longer wavelength and thus, the overall energy efficiency is not always satisfactoy.

Thus, there is a need to provide a color converter leading to a desired color temperature with high quantum conversion efficiency and high photometric efficiency. Furthermore, conventional warm-white LEDs often have reduced quantum conversion efficiency due to multi-phosphors self-absorption losses, and reduced photometric conversion efficiency because of low human eye sensitivity for deep-red light within the broad spectrum of red phosphor emission. Thus, there is an ongoing need for lighting device having high luminous efficacy.

It is therefore an object of the present invention to provide compounds having good optical properties, in particular good luminescent properties. The compounds should have narrow and distinctly separated excitation and emission spectra. In particular, the compounds should exhibit very high fluorescence quantum yields. They should preferably exhibit fluorescence quantum yields over 85%, preferably over 90%, in solid polymer matrices, especially polymer matrices selected from polycarbonate (PC), polyethylene terephtalate (PET) and polystyrene (PS), while having high thermal and photochemical stability. Furthermore, the compounds should be insensitive towards moisture and oxygen. Specially, the compounds should be stable in the presence of light having a wavelength in the range form 400 to 500 nm. The compounds are further intended for use in color converters for converting light emitted from a light source into light of a second longer wavelength, in particular for converting light emitted from a LED or an OLED. Thus, the compounds should be stable under the application conditions during the lifetime of the complete light source. For use in color converters, the compounds embedded in a polymer matrix should have high fluoresence quantum yield as well as high photostability under practical application conditions. The compounds are further intended for use in color converters for converting light emitted from a LED into light of a second longer-wave wavelength in combination with further luminescent materials, in particular yellow, yellow-green and orange organic fluorescent dyes to provide white LEDs with high photometric conversion efficacy.

These and further objects are achieved by the perylene bisimide compounds of the formulae la and Ib described below.

### SUMMARY OF THE INVENTION

According to a first aspect of the present invention there is provided a perylene bisimide compound of the formula la or Ib 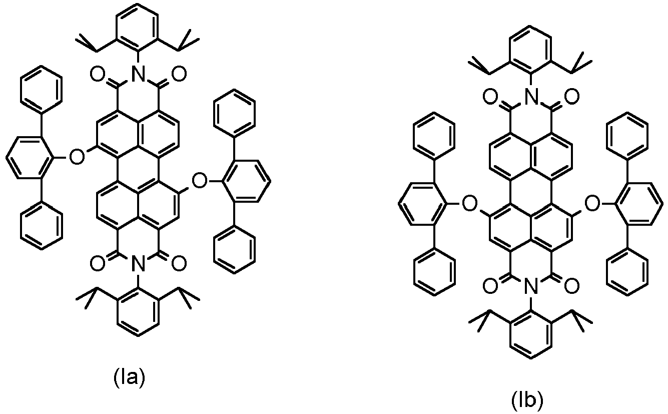 or mixtures of compounds of formula la and Ib.

According to a further aspect of the present invention there is provided a process for preparing a compound of the formula la or Ib or a mixture thereof.

According to a further aspect of the present invention, there is provided the use of a perylene bisimide compound of the formula la or Ib or a mixture thereof, in color converters for converting light emitted from a light source, in particular a light source selected from LEDs and OLEDs, into light of a second longer wavelength, for coloring coatings, printing inks and plastics, producing aqueous polymer dispersions which absorb and/or emit electromagnetic radiation, for data storage, for optical labels, for security labels in documents and for brand protection or as a fluorescent label for biomolecules.

According to a further aspect of the present invention there is provided a color converter comprising at least one polymer as a matrix and at least one compound of the formula la or Ib or a mixture of compounds of formula la and Ib, wherein the at least one polymer is selected from polystyrene, polycarbonate, polymethylmethacrylate, polyvinylpyrrolidone, polymethacrylate, polyvinyl acetate, polyvinyl chloride, polybutene, silicone, polyacrylate, epoxy resin, polyvinyl alcohol, poly(ethylene vinylalcohol)-coplymer (EVA, EVOH), polyacrylonitrile, polyvinylidene chloride (PVDC), polystyreneacrylonitrile (SAN), polybutylene terephthalate (PBT), polyethylene terephthalate (PET), polyvinyl butyrate (PVB), polyvinyl chloride (PVC), polyamides, polyoxymethylenes, polyimides, polyetherimides and mixtures thereof.

According to a further aspect of the present invention there is provided the use of a color converter as defined above and in the following for conversion of light generated by LEDs or OLEDs.

According to a further aspect of the present invention there is provided a lighting device comprising at least one LED and at least one color converter as defined above and in the following.

According to a further aspect of the present invention there is provided a device producing electric power upon illumination comprising a photovoltaic cell and the color converter as defined above and in the following, where at least a part of the light not absorbed by the photovoltaic cell is absorbed by the color converter.

The inventive perylene bisimide compounds of the formulae la and Ib are surprisingly photostable, in particular stable in the presence of blue light, and exhibit a high fluorescence quantum yield. The compounds of formula la and Ib are insensitive to moisture and oxygen. They can advantageously be used in a color converter for conversion of light generated by LEDs or OLEDs. In particular, the use of the inventive perylene bisimide compounds of the formulae la and/or Ib in combination with other luminescent materials in a color converter enables to convert light generated from a LED or OLED light source into light of a second longer wavelength with a high photometric conversion efficacy.

### DETAILED DESCRIPTION OF INVENTION

The definitions of the variables specified in the above formulae use collective terms which are generally representative of the respective substituents. The definition Cₙ-Cₘ gives the number of carbon atoms possible in each case in the respective substituent or substituent moiety:
In the context of the present invention, a "blue LED" is understood to mean a LED which emits light in the blue range of the electromagnetic spectrum, i.e. in the wavelength range from 400 to 500 nm, preferably 420 to 480 nm and especially 440 to 470 nm. Suitable semiconductor materials are silicon carbide, zinc selenide and nitrides such as aluminum nitride (AIN), gallium nitride (GaN), indium nitride (InN) and indium gallium nitride (InGaN). In the context of the present invention, a "green LED" is understood to mean an LED which emits light in the wavelength range from 501 to 560 nm, preferably 501 to 540 nm and especially 520 to 540 nm. Suitable semiconductor materials are for example based on GaInNAs.

In the context of the present invention, a "white LED" is understood to mean an LED which produces white light. Examples of a white LED are multi-LEDs or a blue LED in combination with at least one radiation converting luminophore.

In the context of the present invention, "color converter" is understood to mean all physical devices capable of absorbing light of particular wavelengths and converting it to light of second wavelengths. Color converters are, for example, part of lighting devices, especially those lighting devices which utilize UV light or LEDs or OLEDs as a light source, or of fluorescence conversion solar cells. Thus, the blue light may be (at least) partly converted into visible light of higher wavelengths than the excitation wavelengths.

A quantum dot is a nanocrystal made of semiconductor materials that is small enough to exhibit quantum mechanical properties. The quantum dot has a defined emission wavelength which is not subject to aging and is also photochemically stable. The color output of the dots can be tuned by controlling the size of the crystals. With a smaller size in quantum dots, the quantum dots emit light of a shorter wavelength.

The luminous efficacy of a source is a measure for the efficiency with which the source provides visible light and is measured in lumens per watt. Not all wavelengths of light are equally visible, or equally effective at stimulating human vision, due to the spectral sensitivity of the human eye. The overall luminous efficacy of a source is the product of how well it converts energy to electromagnetic radiation, and how well the emitted radiation is detected by the human eye.

The word "essentially" in the context of the present invention encompasses the words "completely", "wholly" and "all". The word encompasses a proportion of 90% or more, such as 95% or more, especially 99% or 100%.

The definitions of the variables specified in the above formulae use collective terms which are generally representative of the respective substituents. The definition Cₙ-Cₘ gives the number of carbon atoms possible in each case in the respective substituent or substituent moiety.

The expression "halogen" denotes in each case fluorine, bromine, chlorine or iodine, particularly chlorine, bromide or iodine.

The term "aliphatic radical" refers to an acyclic saturated or unsaturated, straight-chain or branched hydrocarbon radical. Usually the aliphatic radical has 1 to 100 carbon atoms. Examples for an aliphatic radical are alkyl, alkenyl and alkynyl.

The term "cycloaliphatic radical" refers to a cyclic, non-aromatic saturated or unsaturated hydrocarbon radical having usually 3 to 20 ring carbon atoms. Examples are cycloalkanes, cycloalkenes, and cycloalkynes. The cycloaliphatic radical may also heteroatoms or heteroatom groups.

The term "alkyl" as used herein and in the alkyl moieties of alkoxy, alkylthio, alkylsulfinyl, alkylsulfonyl, alkylcarbonyl, alkoxycarbonyl and the like refers to saturated straight-chain or branched hydrocarbon radicals having 1 to 100 ("C₁-C₁₀₀-alkyl"), 1 to 30 ("C₁-C₃₀-alkyl"),1 to 18 ("C₁-C₁₈-alkyl"), 1 to 12 ("C₁-C₁₂-alkyl"), 1 to 8 ("C₁-C₈-alkyl") or 1 to 6 ("C₁-C₆-alkyl") carbon atoms. Alkyl is preferably C₁-C₂₄-alkyl, more preferably C₁-C₂₀-alkyl. Examples of alkyl groups are especially methyl, ethyl, n-propyl, isopropyl, n-butyl, isobutyl, sec-butyl, tert-butyl, n-pentyl, 1-methylbutyl, 1-ethylpropyl, neo-pentyl, n-hexyl, 1-methylpentyl, 2-methylpentyl, 1-ethylbutyl, 2-ethylbutyl, n-heptyl, 1-methylhexyl, 2-methylhexyl, 1-ethylpentyl, 1-propylbutyl, 2-ethylpentyl, n-octyl, 1-methylheptyl, 2-methylheptyl, 1-ethylhexyl, 2-ethylhexyl, 1-propylpentyl, 2-propylpentyl, n-nonyl, 1-methyloctyl, 2-methyloctyl, 1-ethylheptyl, 2-ethylheptyl, 1-propylhexyl, 2-propylhexyl, 1-butylpentyl, n-decyl, 2-methyldecyl, 1-methylnonyl, 2-methylnonyl, 1-ethyloctyl, 2-ethyloctyl, 1-propylheptyl, 2-propylheptyl, 1-butylhexyl, 2-butylhexyl, n-undecyl, 2-ethylnonyl, 1-propyloctyl, 2-propyloctyl, 1-butylheptyl, 2-butylheptyl, 1-pentylhexyl, n-dodecyl, 2-ethyldecyl, 2-propylnonyl, 1-butyloctyl, 2-butyloctyl, 1-pentylheptyl, 2-pentylheptyl, 2-propyldecyl, n-tridecyl, 1-pentyloctyl, 2-pentyloctyl, 1-hexylheptyl, 2-butylnonyl, n-tetradecyl, 1-hexyloctyl, 2-hexyloctyl, 2-pentylnonyl, 2-hexylnonyl, 2-pentyldecyl, 2-butyldecyl,n-hexadecyl, 1-heptyloctyl, 2-heptylnonyl, 2-hexyldecyl, 2-heptyldecyl, n-octadecyl, 2-octyldecyl, n-eicosyl, 2-nonylundecyl, 2-octylundecyl, 2-heptylundecyl, 2-hexylundecyl, 2-pentylundecyl, 2-butylundecyl, 2-propylundecyl, 2-ethylundecyl, 2-methylundecyl, 2-decyldodecyl, 2-nonyldodecyl, 2-octyldodecyl, 2-heptyldodecyl, 2-hexyldodecyl, 2-pentyldodecyl, 2-butyldodecyl, 2-propyldodecyl, 2-ethyldodecyl, 2-methyldodecyl, 2-undecyltridecyl, 2-decyltridecyl, 2-nonyltridecyl, 2-octyltridecyl, 2-heptyltridecyl, 2-hexyltridecyl, 2-pentyltridecyl, 2-butyltridecyl, 2-propyltridecyl, 2-ethyltridecyl, 2-methyltridecyl, 2-undecyltetradecyl, 2-decyltetradecyl, 2-nonyltetradecyl, 2-octyltetradecyl, 2-hetyltetradecyl, 2-hexyltetradecyl, 2-pentyltetradecyl, 2-butyltetradecyl, 2-propyltetradecyl, 2-ethyltetradecyl, 2-methyltetradecyl, 2-tetradecylhexadecyl, 2-tridecylhexadecyl, 2-dodecylhexadecyl, 2-undecylhexadecyl, 2-decylhexadecyl, 2-nonylhexadecyl, 2-octylhexadecyl, 2-heptylhexadecyl, 2-hexylhexadecyl, 2-pentylhexadecyl, 2-butylhexadecyl, 2-propylhexadecyl, 2-ethylhexadecyl, 2-methylhexadecyl, 2-dodecyloctadecyl, 2-undecyloctadecyl, 2-decyloctadecyl, 2-nonyloctadecyl, 2-octyloctadecyl, 2-heptyloctadecyl, 2-hexyloctadecyl, 2-pentyloctadecyl, 2-butyloctadecyl, 2-propyloctadecyl, 2-ethyloctadecyl, 2-methyloctadecyl, 2-decyleicosanyl, 2-nonyleicosanyl, 2-octyleicosanyl, 2-heptyleicosanyl, 2-hexyleicosanyl, 2-pentyleicosanyl, 2-butyleicosanyl, 2-propyleicosanyl, 2-ethyleicosanyl, 2-methyleicosanyl, 2-octadecyldocosanyl, 2-heptadecyldocosanyl, 2-hexadecyldocosanyl, 2-pentadecyldocosanyl, 2-tetradecyldocosanyl, 2-tridecyldocosanyl, 2-undecyldocosanyl, 2-decyldocosanyl, 2-nonyldocosanyl, 2-octyldocosanyl, 2-heptyldocosanyl, 2-hexyldocosanyl, 2-pentyldocosanyl, 2-butyldocosanyl, 2-propyldocosanyl, 2-ethyldocosanyl, 2-methyldocosanyl, 2-docosanyltetracosanyl, 2-hexadecyltetracosanyl, 2-pentadecyltetracosanyl, 2-pentadecyltetracosanyl, 2-tetradecyltetracosanyl, 2-tridecyltetracosanyl, 2-dodecyltetracosanyl, 2-undecyltetracosanyl, 2-decyltetracosanyl, 2-nonyltetracosanyl, 2-octyltetracosanyl, 2-heptyltetracosanyl, 2-hexyltetracosanyl, 2-pentyltetracosanyl, 2-butyltetracosanyl, 2-propyltetracosanyl, 2-ethyltetracosanyl, 2-methyltetracosanyl, 2-dodecyloctacosanyl, 2-undecyloctacosanyl, 2-decyloctacosanyl, 2-nonyloctacosanyl, 2-octyloctacosanyl, 2-heptyloctacosanyl, 2-hexyloctacosanyl, 2-pentyloctacosanyl, 2-butyloctacosanyl, 2-propyloctacosanyl, 2-ethyloctacosanyl and 2-methyloctacosanyl.

Haloalkyl and all haloalkyl moieties in haloalkoxy: straight-chain or branched alkyl groups having usually 1 to 24, frequently 1 to 20 carbon atoms (as specified above), where some or all of the hydrogen atoms in these groups are replaced by halogen atoms as specified above.

The expression alkyl also comprises alkyl radicals whose carbon chains may be interrupted by one or more nonadjacent groups which are selected from -O-, -S- and -NR^{cc}. R^{cc} is hydrogen, C₁-C₂₀-alkyl, C₃-C₂₄-cycloalkyl, heterocycloalkyl, C₆-C₂₄-aryl or heteroaryl. The above remarks regarding alkyl also apply to the alkyl moiety in alkoxy, alkylthio (= alkylsulfanyl), monoalkylamino, dialkylamino and the alkylene moiety in aryl-C₁-C₁₀-alkylene.

Examples of alkyl groups whose carbon chains are interrupted by one or more, e.g. 1, 2, 3, 4, 5, 6., 7, 8 or more than 8, nonadjacent groups are especially 2-methoxyethyl, 2-ethoxyethyl, 2-propoxyethyl, 2-isopropoxyethyl, 2-butoxyethyl, 2- and 3-methoxypropyl, 2- and 3-ethoxypropyl, 2- and 3-propoxypropyl, 2- and 3-butoxypropyl, 2- and 4-methoxybutyl, 2- and 4-ethoxybutyl, 2- and 4-propoxybutyl, 3,6-dioxaheptyl, 3,6-dioxaoctyl, 4,8-dioxanonyl, 3,7-dioxaoctyl, 3,7-dioxanonyl, 4,7-dioxaoctyl, 4,7-dioxanonyl, 2- and 4-butoxybutyl, 4,8-dioxadecyl, 3,6,9-trioxadecyl, 3,6,9-trioxaundecyl, 3,6,9-trioxadodecyl, 3,6,9,12-tetraoxatridecyl and 3,6,9,12-tetra-oxatetradecyl; 2-methylthioethyl, 2-ethylthioethyl, 2-propylthioethyl, 2-isopropylthio-ethyl, 2-butylthioethyl, 2- and 3-methylthiopropyl, 2- and 3-ethylthiopropyl, 2- and 3-propylthiopropyl, 2- and 3-butylthiopropyl, 2- and 4-methylthiobutyl, 2- and 4-ethyl-thiobutyl, 2- and 4-propylthiobutyl, 3,6-dithiaheptyl, 3,6-dithiaoctyl, 4,8-dithianonyl, 3,7-dithiaoctyl, 3,7-dithianonyl, 2- and 4-butylthiobutyl, 4,8-dithiadecyl, 3,6,9-tri-thiadecyl, 3,6,9-trithiaundecyl, 3,6,9-trithiadodecyl, 3,6,9,12-tetrathiatridecyl and 3,6,9,12-tetrathiatetradecyl; 2-monomethyl- and 2-monoethylaminoethyl, 2-dimethylaminoethyl, 2- and 3-dimethylaminopropyl, 3-monoisopropylaminopropyl, 2-and 4-monopropylaminobutyl, 2- and 4-dimethylaminobutyl, 6-methyl-3,6-diazaheptyl, 3,6-dimethyl-3,6-diazaheptyl, 3,6-diazaoctyl, 3,6-dimethyl-3,6-diazaoctyl, 9-methyl-3,6,9-triazadecyl, 3,6,9-trimethyl-3,6,9-triazadecyl, 3,6,9-triazaundecyl, 3,6,9-trimethyl-3,6,9-triazaundecyl, 12-methyl-3,6,9,12-tetraazatridecyl and 3,6,9,12-tetramethyl-3,6,9,12-tetraazatridecyl; (1-ethylethylidene)aminoethylene, (1-ethylethylidene)-aminopropylene, (1-ethylethylidene)aminobutylene, (1-ethylethylidene)aminodecylene and (1-ethylethylidene)aminododecylene.

Alkylene represents a linear or branched saturated hydrocarbon chain having from 1 to 10 and especially from 1 to 6 carbon atoms, such as linear C₁-C₆-alkylene, e.g. methylene (-CH₂-), ethane-1,2-diyl, propane-1,3-diyl, butane-1,4-diyl, pentane-1,5-diyl or hexane-1,6-diyl and branched C₂-C₆-alkylene, such as ethane-1,1-diyl, propane-1,2-diyl, butane-1,2-diyl or butane-1,3-diyl.

The term "alkenyl" as used herein refers to straight-chain or branched hydrocarbon groups having 2 to 100 ("C₂-C₁₀₀-alkenyl"), 2 to 18 ("C₂-C₁₈-alkenyl"), 2 to 10 ("C₂-C₁₀-alkenyl"), 2 to 8 ("C₂-C₈-alkenyl"), or 2 to 6 ("C₂-C₆-alkenyl") carbon atoms and one or more, e.g. 2 or 3, double bonds in any position.

The term "alkynyl" as used herein (also referred to as alkyl whose carbon chain may comprise one or more triple bonds) refers to straight-chain or branched hydrocarbon groups having 2 to 100 ("C₂-C₁₀₀-alkynyl"), 2 to 18 ("C₂-C₁₈-alkynyl"), 2 to 10 ("C₂-C₁₀-alkynyl"), 2 to 8 ("C₂-C₈-alkynyl"), or 2 to 6 ("C₂-C₆-alkynyl") carbon atoms and one or more, e.g. 2 or 3, triple bonds in any position.

The term "cycloalkyl" as used herein refers to mono- or bi- or polycyclic saturated hydrocarbon radicals having 3 to 24 (C₃-C₂₄-cycloalkyl), 3 to 20 ("C₃-C₂₀-cycloalkyl") atoms, preferably 3 to 8 ("C₃-C₈-cycloalkyl") or 3 to 6 carbon atoms ("C₃-C₆-cycloalkyl"). Examples of monocyclic radicals having 3 to 6 carbon atoms comprise cyclopropyl, cyclobutyl, cyclopentyl and cyclohexyl. Examples of monocyclic radicals having 3 to 8 carbon atoms comprise cyclopropyl, cyclobutyl, cyclopentyl, cyclohexyl, cycloheptyl and cyclooctyl. Examples of bicyclic radicals having 7 to 12 carbon atoms comprise bicyclo[2.2.1]heptyl, bicyclo[3.1.1]heptyl, bicyclo[2.2.2]octyl, bicyclo[3.3.0]octyl, bicyclo[3.2.1]octyl, bicyclo[3.3.1]nonyl, bicyclo[4.2.1]nonyl, bicyclo[4.3.1]decyl, bicyclo[3.3.2]decyl, bicyclo[4.4.0]decyl, bicyclo[4.2.2]decyl, bicyclo[4.3.2] undecyl, bicyclo[3.3.3]undecyl, bicyclo[4.3.3]dodecyl,and perhydronaphthyl. Examples of polycyclic rings are perhydroanthracyl, perhydrofluorenyl, perhydrochrysenyl, perhydropicenyl, and adamantly.

The term heterocycloalkyl refers to nonaromatic, partially unsaturated or fully saturated, heterocyclic rings having generally 5 to 8 ring members, preferably 5 or 6 ring members, comprising besides carbon atoms as ring members, one, two, three or four heteroatoms or heteroatom-containing groups selected from O, N, NR^{cc}, S, SO and S(O)₂ as ring members, wherein R^{cc} is hydrogen, C₁-C₂₀-alkyl, C₃-C₂₄-cycloalkyl, heterocycloalkyl, C₆-C₂₄-aryl or heteroaryl. Examples of heterocycloalkyl groups are especially pyrrolidinyl, piperidinyl, imidazolidinyl, pyrazolidinyl, oxazolidinyl, morpholinyl, thiazolidinyl, isothiazolidinyl, isoxazolidinyl, piperazinyl, tetrahydrothiophenyl, dihydrothien-2-yl, tetrahydrofuranyl, dihydrofuran-2-yl, tetrahydropyranyl, 2-oxazolinyl, 3-oxazolinyl, 4-oxazolinyl and dioxanyl.

The expression C₆-C₂₄-aryl refers to an aromatic radical having 6 to 24 carbon atoms, preferably 6 to 20 carbon atoms, especially 6 to 14 carbon atoms as ring members.

Aryl is preferably phenyl, naphthyl, indenyl, fluorenyl, anthracenyl, phenanthrenyl, naphthacenyl, chrysenyl, pyrenyl, coronenyl, perylenyl, etc., and more preferably phenyl or naphthyl.

Aryl which bears one or more C₁-C₂₄-alkyl radicals is, for example, 2-, 3- and 4-methylphenyl, 2,4-, 2,5-, 3,5- and 2,6-dimethylphenyl, 2,4,6-trimethylphenyl, 2-, 3-and 4-ethylphenyl, 2,4-, 2,5-, 3,5- and 2,6-diethylphenyl, 2,4,6-triethylphenyl, 2-, 3- and 4-propylphenyl, 2,4-, 2,5-, 3,5- and 2,6-dipropylphenyl, 2,4,6-tripropylphenyl, 2-, 3- and 4-isopropylphenyl, 2,4-, 2,5-, 3,5- and 2,6-diisopropylphenyl, 2,4,6-triisopropylphenyl, 2-, 3- and 4-butylphenyl, 2,4-, 2,5-, 3,5- and 2,6-dibutylphenyl, 2,4,6-tributylphenyl, 2-, 3- and 4-isobutylphenyl, 2,4-, 2,5-, 3,5- and 2,6-diisobutylphenyl, 2,4,6-triisobutylphenyl, 2-, 3- and 4-sec-butylphenyl, 2,4-, 2,5-, 3,5- and 2,6-di-sec-butylphenyl, 2,4,6-tri-sec-butylphenyl, 2-, 3- and 4-tert-butylphenyl, 2,4-, 2,5-, 3,5- and 2,6-di-tert-butylphenyl and 2,4,6-tri-tert-butylphenyl.

The term C₆-C₂₄-aryl-C₁-C₁₀-alkylene refers to an aromatic radical having 6 to 24 carbon atoms as defined above, which is bound to the remainder of the molecule via a C₁-C₁₀-alkylene group, as defined above, in particular via a methylene, 1,1-ethylene or 1,2-ethylene group. Examples for C₆-C₂₄-aryl-C₁-C₁₀-alkylene are benzyl, 1-phenylethyl and 2-phenylethyl , phenylpropyl, naphthylmethyl, naphthylethyl etc.

C₆-C₂₄-aryloxy: C₆-C₂₄-aryl as defined above, which is bonded to the skeleton via an oxygen atom (-O-). Preference is given to phenoxy and naphthyloxy.

The term "heteroaryl" (also referred to as hetaryl) comprises heteroaromatic, mono- or polycyclic groups. In addition to the ring carbon atoms, these have 1, 2, 3, 4 or more than 4 heteroatoms as ring members. The heteroatoms are preferably selected from oxygen, nitrogen, selenium and sulfur. The heteroaryl groups have preferably 5 to 18, e.g. 5, 6, 8, 9, 10, 11, 12, 13 or 14, ring atoms.

Monocyclic heteroaryl groups are preferably 5- or 6-membered heteroaryl groups, such as 2 furyl (furan-2-yl), 3-furyl (furan-3-yl), 2-thienyl (thiophen-2-yl), 3 thienyl (thiophen-3-yl), selenophen-2-yl, selenophen-3-yl, 1H-pyrrol-2-yl, 1H-pyrrol-3-yl, pyrrol-1-yl, imidazol-2-yl, imidazol-1-yl, imidazol-4-yl, pyrazol-1-yl, pyrazol-3-yl, pyrazol-4-yl, pyrazol-5-yl, 3-isoxazolyl, 4-isoxazolyl, 5-isoxazolyl, 3 isothiazolyl, 4-isothiazolyl, 5-isothiazolyl, 2-oxazolyl, 4-oxazolyl, 5-oxazolyl, 2-thiazolyl, 4-thiazolyl, 5-thiazolyl, 1,2,4-oxadiazol-3-yl, 1,2,4-oxadiazol-5-yl, 1,3,4 oxadiazol 2 yl, 1,2,4 thiadiazol-3-yl, 1,2,4-thiadiazol-5-yl, 1,3,4-thiadiazol-2-yl, 4H-[1,2,4]-triazol-3-yl, 1,3,4-triazol-2-yl, 1,2,3-triazol-1-yl, 1,2,4-triazol-1-yl, pyridin-2-yl, pyridin-3-yl, pyridin-4-yl, 3 pyridazinyl, 4-pyridazinyl, 2-pyrimidinyl, 4-pyrimidinyl, 5-pyrimidinyl, 2 pyrazinyl, 1,3,5-triazin-2-yl and 1,2,4-triazin-3-yl.

Polycyclic heteroaryl groups have 2, 3, 4 or more than 4 fused rings. The fused-on rings may be aromatic, saturated or partly unsaturated. Examples of polycyclic heteroaryl groups are quinolinyl, isoquinolinyl, indolyl, isoindolyl, indolizinyl, benzofuranyl, isobenzofuranyl, benzothiophenyl, benzoxazolyl, benzisoxazolyl, benzthiazolyl, benzoxadiazolyl, benzothiadiazolyl, benzoxazinyl, benzopyrazolyl, benzimidazolyl, benzotriazolyl, benzotriazinyl, benzoselenophenyl, thienothiophenyl, thienopyrimidyl, thiazolothiazolyl, dibenzopyrrolyl (carbazolyl), dibenzofuranyl, dibenzothiophenyl, naphtho[2,3-b]thiophenyl, naphtha[2,3-b]furyl, dihydroindolyl, dihydroindolizinyl, dihydroisoindolyl, dihydroquinolinyl and dihydroisoquinolinyl.

Preferred embodiments and further aspects of the present invention are outlined in the following paragraphs:
The compound of formula la is also referred to as N,N'-bis(2,6-diisopropylphenyl)-1,7-di(2,6-diphenylphenoxy)perylene-3,4;9,10-tetracarboximide. The compound of the formula Ib is also referred to as N,N'-bis(2,6-diisopropylphenyl)-1,6-di(2,6-diphenylphenoxy)perylene-3,4;9,10-tetracarboximide. The compounds of formulae (Ia) and (Ib) are red-fluorescent dyes.

Preference is given to the compound of formula Ia. Likewise, preference is given to a mixture of compounds of formula Ia and Ib.

Compounds of the formula Ia and Ib can be prepared in analogy to methods described in WO 2007/006717.

Accordingly, the present invention relates to a process for preparing compounds of the formula Ia and Ib 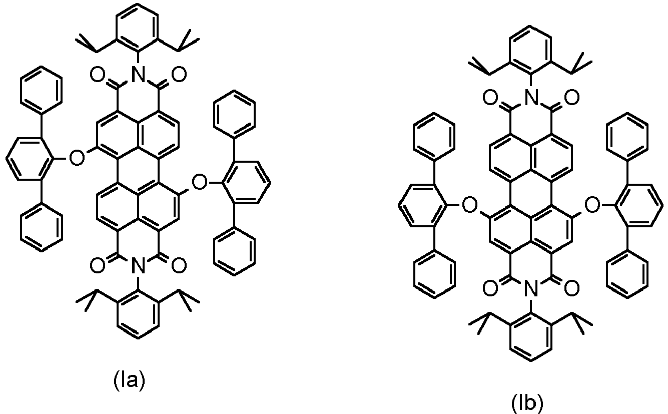 comprising
a) providing a compound of formulae (1) or (2) or a mixture thereof 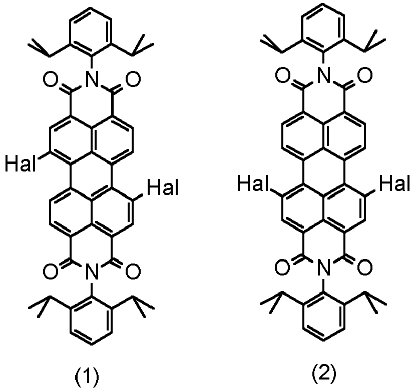 wherein
   Hal are each all chlorine or each all bromine;
b) reacting the compound of formula (1) or the compound of formula (2) or a mixture of compounds of formulae (1) or (2) provided in step a) with 2,6-diphenylphenol in the presence of an inorganic base and of a polar, aprotic solvent to obtain a compound of formula (Ia) or (Ib) or a mixture thereof.
(c) optionally, subjecting the reaction mixture obtained in step b) to at least one work-up step.

### Step a)

Perylene compounds of the formula (1) and (2) are known from WO 2007/006717, DE 19547210 and DE 19547209.

### Step b)

Step b) is carried out in the presence of a polar, aprotic solvent. Suitable solvents are especially aliphatic carboxamides, preferably N,N-di-C₁-C₄-alkyl-C₁-C₄-carboxamides, lactams such as dimethylformamide, diethylformamide, dimethylacetamide, dimethylbutyramide and N-methyl-2-pyrrolidone (NMP), nitriles such as acetonitrile. It is also possible to use mixtures of polar, aprotic solvents. Particular preference is given to NMP.

It may be advantageous to use only a small amout of solvent. The amount of solvent is in general, from 2 to 30 mL, preferably from 3 to 25 mL, in particular 4 to 25 mL of solvent per g of halogenated perylene compound.

Suitable bases are in particular inorganic alkali metal or alkaline earth metal bases, the alkali metal bases being particularly suitable. Examples of inorganic bases are the carbonates and hydrogencarbonates, hydroxides, hydrides and amides of alkali metals and alkaline earth metals. Preferred bases are the carbonates and hydrogencarbonates, particular preference being given to the carbonates. Preferred alkali metals are lithium, sodium, potassium and cesium; particularly suitable alkaline earth metals are magnesium and calcium. It will be appreciated that it is also possible to use base mixtures. Very particularly preferred bases are lithium carbonate, sodium carbonate, potassium carbonate and cesium carbonate.

In general, at least 0.4 equivalent of base is required per mole of 2,6-diphenylphenol. Particularly suitable used amounts for the bases are from 0.4 to 3 equivalents, especially from 0.4 to 1.2 equivalents per mole of 2,6-diphenylphenol.

In general, from 1 to 10 mol, preferably from 1 to 5 mol of 2,6-diphenylphenol are used per mole of halogen atom to be exchanged.

The reaction temperature is generally within the range from 0 to the boiling point of the solvent, preferably 0 to 100°C,more preferably 20 to 80°C and in particular 30 to 60°C.

The reaction time is typically from 0.5 to 48 h, preferably 0.5 to 25 h, for example 1 to 25 h, 2 to 10 h.

Usually, the reactants are first mixed and then heated to the reaction temperature for the required reaction time.

If a mixture of compounds of formula (1) and (2) is used, the isolation of the resulting perylene bisimide compounds of formulae (Ia) and (Ib) can be undertaken as follows: the precipitate obtained can be filtered off to get a filter cake and filtrate. It is assumed that the filtrate comprises at least part of the compound of formula (Ib). The filter cake comprises precipitated inorganic salts together with the precipitated perylenecarboximide of formula (Ia) and small amounts of perylenecarboximide of formula Ib. The compound of formula (Ia) is isolated by washing out the filter cake with the solvent used in step b) followed by washing with water to remove the inorganic salts and the compound of formula (Ib).

The inventive compound(s) of formula (Ia) and (Ib) may be incorporated without any problem into organic and inorganic materials and are therefore suitable for a whole series of end uses, some of which will be listed by way of example below. They may be used generally for coloring coatings, printing inks and plastics, producing aqueous polymer dispersions which absorb and/or emit electromagnetic radiation, for data storage, for optical labels, for security labels in documents and for brand protection or as a fluorescent label for biomolecules or as a fluorescent label for biomolecules. The perylene compound(s) (Ia) and/or (Ib) are notable for their fluorescence which lies within the visible region of the electromagnetic spectrum. The inventive perylene compound(s) (Ia) and/or (Ib) may be used to produce aqueous polymer dispersions which absorb and/or emit electromagnetic radiation. Fluorescent polymer dispersions can be obtained with perylene compounds la and/or Ib. The perylene compound(s) (Ia) and/or (Ib) is (are) especially suitable for data storage, for optical labels, for security labels in documents and for brand protection or as a fluorescent label for biomolecules.

The inventive perylene compound(s) (Ia) and/or (Ib) is (are) notable for use in color converters for converting light emitted from a light source, in particular a light source selected from LEDs and OLEDs, into light of a second longer wavelength.

Accordingly, the present invention further provides a color converter comprising at least one polymer as a matrix material and at least one compound of the formula la or Ib or a mixture of compounds of formulae la and Ib.

Suitable polymers are in principle all polymers capable of dissolving or homogeneously dispersing the at least one compound of the formula la or Ib or a mixture of compounds of formulae la and Ib in a sufficient amount.

Suitable polymers may be inorganic polymers or organic polymers.

In a preferred embodiment, the at least one polymer is selected from polystyrene, polycarbonate, polymethylmethacrylate, polyvinylpyrrolidone, polymethacrylate, polyvinyl acetate, polyvinyl chloride, polybutene, silicone, polyacrylate, epoxy resin, polyvinyl alcohol, poly(ethylene vinylalcohol)-coplymer (EVA, EVOH), polyacrylonitrile, polyvinylidene chloride (PVDC), polystyreneacrylonitrile (SAN), polybutylene terephthalate (PBT), polyethylene terephthalate (PET), polyvinyl butyrate (PVB), polyvinyl chloride (PVC), polyamides, polyoxymethylenes, polyimides, polyetherimides and mixtures thereof.

More preferably, the at least one polymer consists essentially of polystyrene, polycarbonate, polymethylmethacrylate, polyvinylpyrrolidone, polymethacrylate, polyvinyl acetate, polyvinyl chloride, polybutene, silicone, polyacrylate, epoxy resin, polyvinyl alcohol, poly(ethylene vinylalcohol)-copolymer (EVA, EVOH), polyacrylonitrile, polyvinylidene chloride (PVDC), polystyreneacrylonitrile (SAN), polybutylene terephthalate (PBT), polyethylene terephthalate (PET), polyvinyl butyrate (PVB), polyvinyl chloride (PVC), polyamides, polyoxymethylenes, polyimides, polyetherimides or mixtures thereof.

In particular, the at least one polymer consists essentially of polystyrene (PS), polycarbonate (PC), polymethylmethacrylate (PMMA), polyethylene terephthalate (PET) or mixtures thereof.

Most preferably, the at least one polymer consists essentially of polystyrene, polyethylene terephthalate or polycarbonate, especially of polycarbonate or polyethylene terephthalate.

Polystyrene is understood here to mean, inter alia, all homo- or copolymers which result from polymerization of styrene and/or derivatives of styrene. Derivatives of styrene are, for example, alkylstyrenes such as alpha-methylstyrene, ortho-, meta-, para-methylstyrene, para-butylstyrene, especially para-tert-butylstyrene, alkoxystyrene such as para-methoxystyrene, para-butoxystyrene, para-tert-butoxystyrene.

In general, suitable polystyrenes have a mean molar mass Mₙ of 10 000 to 1 000 000 g/mol (determined by GPC), preferably 20 000 to 750 000 g/mol, more preferably 30 000 to 500 000 g/mol.

In a preferred embodiment, the matrix of the color converter consists essentially or completely of a homopolymer of styrene or styrene derivatives.

In further preferred embodiments of the invention, the matrix consists essentially or completely of a styrene copolymer, which are likewise regarded as polystyrene in the context of this application. Styrene copolymers may comprise, as further constituents, for example, butadiene, acrylonitrile, maleic anhydride, vinylcarbazole or esters of acrylic, methacrylic or itaconic acid as monomers. Suitable styrene copolymers generally comprise at least 20% by weight of styrene, preferably at least 40% and more preferably at least 60% by weight of styrene. In another embodiment, they comprise at least 90% by weight of styrene.

Preferred styrene copolymers are styrene-acrylonitrile copolymers (SAN) and acrylonitrile-butadiene-styrene copolymers (ABS), styrene-1,1'-diphenylethene copolymers, acrylic ester-styrene-acrylonitrile copolymers (ASA), methyl methacrylate-acrylonitrile-butadiene-styrene copolymers (MABS).

A further preferred polymer is alpha-methylstyrene-acrylonitrile copolymer (AMSAN).

The styrene homo- or copolymers can be prepared, for example, by free-radical polymerization, cationic polymerization, anionic polymerization or under the influence of organometallic catalysts (for example Ziegler-Natta catalysis). This can lead to isotactic, syndiotactic or atactic polystyrene or copolymers. They are preferably prepared by free-radical polymerization. The polymerization can be performed as a suspension polymerization, emulsion polymerization, solution polymerization or bulk polymerization.

The preparation of suitable polystyrenes is described, for example, in Oscar Nuyken, Polystyrenes and Other Aromatic Polyvinyl Compounds, in Kricheldorf, Nuyken, Swift, New York 2005, p. 73-150 and references cited therein; and in Elias, Macromolecules, Weinheim 2007, p. 269-275.

Polyethylene terephthalate is obtainable by condensation of ethylene glycol with terephthalic acid.

Polycarbonates are polyesters of carbonic acid with aromatic or aliphatic dihydroxyl compounds. Preferred dihydroxyl compounds are, for example, methylenediphenylenedihydroxyl compounds, for example bisphenol A.

One means of preparing polycarbonates is the reaction of suitable dihydroxyl compounds with phosgene in an interfacial polymerization. Another means is the reaction with diesters of carbonic acid such as diphenyl carbonate in a condensation polymerization.

The preparation of suitable polycarbonates is described, for example, in Elias, Macromolecules, Weinheim 2007, p. 343-347.

In a preferred embodiment, polymers which have been polymerized with exclusion of oxygen are used. Preferably, the monomers during the polymerization comprised a total of not more than 1000 ppm of oxygen, more preferably not more than 100 ppm and especially preferably not more than 10 ppm.

Suitable polymers may comprise, as further constituents, additives such as flame retardants, antioxidants, light stabilizers, UV absorbers, free-radical scavengers, antistats. Stabilizers of this kind are known to those skilled in the art.

Suitable antioxidants or free-radical scavengers are, for example, phenols, especially sterically hindered phenols such as butylhydroxyanisole (BHA) or butylhydroxytoluene (BHT), or sterically hindered amines (HALS). Stabilizers of this kind are sold, for example, by BASF under the Irganox^{®} trade name. In some cases, antioxidants and free-radical scavengers can be supplemented by secondary stabilizers such as phosphites or phosphonites, as sold, for example, by BASF under the Irgafos^{®} trade name.

Suitable UV absorbers are, for example, benzotriazoles such as 2-(2-hydroxyphenyl)-2H-benzotriazole (BTZ), triazines such as (2-hydroxyphenyl)-s-triazine (HPT), hydroxybenzophenones (BP) or oxalanilides. UV absorbers of this kind are sold, for example, by BASF under the Uvinul® trade name.

In a preferred embodiment, TiO₂ is used as the sole UV absorber.

In a preferred embodiment of the invention, suitable polymers do not comprise any antioxidants or free-radical scavengers.

In a further embodiment of the invention, suitable polymers are transparent polymers.

In another embodiment, suitable polymers are opaque polymers.

The polymers mentioned above serve as matrix material for the inventive organic fluorescent dye selected from a compound of formula la, a compound of formula Ib and mixtures thereof.

The inventive fluorescent dye(s), i.e. the compound of the formula la or the compound of the formula Ib or a mixture of compounds of formulae la and Ib, may either be dissolved in the polymer or may be in the form of a homogeneously distributed mixture. The fluorescent dyes are preferably dissolved in the polymer.

Preferably, the color converter comprises as fluorescent dye a compound of the formula (Ia). Likewise preferably, the color converter comprises as fluorescent dye a mixture of compounds of formulae (Ia) and (Ib). The compounds of formulae la and Ib are red-fluorescing fluorescent dyes.

In a preferred embodiment, color converters comprise, in addition to the at least one organic red-fluorescent dye according to the invention, further organic fluorescent dyes. Suitable further organic fluorescent dyes may be any organic fluorescent dye that absorbs light in the wavelength range from 400 to 500 nm and emits light having a longer wavelength than that of the absorbed light, especially in the wavelength range from 450 to 600 nm.

Different organic fluorescent dyes may be combined to optimize energy efficiency and - in particular - photometric efficacy. Suitable further organic fluorescent dyes are green-fluorescent dyes, yellow-green fluorescent dyes, yellow-fluorescent dyes, orange-fluorescent dyes and red-fluorescent dyes. Preferably, fluorescent dyes are combined with one another such that color converters with high energy and photometric efficacy are obtained.

In a preferred embodiment, color converters comprise, as well as the at least one inventive fluorescent dye of the compound of the formulas la or Ib or mixtures thereof, further organic fluorescent dyes.

In particular, further organic fluorescent dyes are selected from
(i) a cyanated naphthalene benzimidazole compound of the formula II 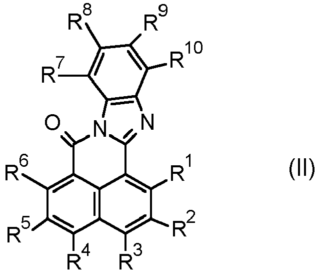 wherein
   R¹, R², R³, R⁴, R⁵, R⁶, R⁷, R⁸, R⁹ and R¹⁰ are each independently hydrogen, cyano or aryl which is unsubstituted or has one or more identical or different substituents R^{Ar},
   where
   each R^{Ar} is independently selected from cyano, hydroxyl, mercapto, halogen, C₁-C₂₀-alkoxy, C₁-C₂₀-alkylthio, nitro, -NR^{Ar2}R^{Ar3}, -NR^{Ar2}COR^{Ar3}, -CONR^{Ar2}R^{Ar3}, -SO₂NR^{Ar2}R^{Ar3}, -COOR^{Ar2}, -SO₃R^{Ar2},
   C₁-C₃₀-alkyl, C₂-C₃₀-alkenyl, C₂-C₃₀-alkynyl, where the three latter radicals are unsubstituted or bear one or more R^{a} groups,
   C₃-C₈-cycloalkyl, 3- to 8-membered heterocyclyl, where the two latter radicals are unsubstituted or bear one or more R^{b} groups,
   aryl, U-aryl, heteroaryl and U-heteroaryl, where the four latter radicals are unsubstituted or bear one or more R^{b} groups,
   where
   each R^{a} is independently selected from cyano, hydroxyl, oxo, mercapto, halogen, C₁-C₂₀-alkoxy, C₁-C₂₀-alkylthio, nitro, -NR^{Ar2}R^{Ar3}, -NR^{Ar2}COR^{Ar3}, -CONR^{Ar2}R^{Ar3}, -SO₂NR^{Ar2}R^{Ar3}, -COOR^{Ar2}, -SO₃R^{Ar2}, C₃-C₈-cycloalkyl, 3- to 8-membered heterocyclyl, aryl and heteroaryl, where the cycloalkyl, heterocyclyl, aryl and heteroaryl radicals are unsubstituted or bear one or more R^{b} groups;
   each R^{b} is independently selected from cyano, hydroxyl, oxo, mercapto, halogen, C₁-C₂₀-alkoxy, C₁-C₂₀-alkylthio, nitro, -NR^{Ar2}R^{Ar3}, -NR^{Ar2}COR^{Ar3}, -CONR^{Ar2}R^{Ar3}, -SO₂NR^{Ar2}R^{Ar} -COOR^{Ar2}, -SO₃R^{Ar2}, C₁-C₁₈-alkyl, C₂-C₁₈-alkenyl, C₂-C₁₈-alkynyl, C₃-C₈-cycloalkyl, 3- to 8-membered heterocyclyl, aryl and heteroaryl, where the four latter radicals are unsubstituted or bear one or more R^{b1} groups,
   each R^{b1} is independently selected from cyano, hydroxyl, mercapto, oxo, nitro, halogen, -NR^{Ar2}R^{Ar3}, -NR^{Ar2}COR^{Ar3}, -CONR^{Ar2}R^{Ar3}, -SO₂NR^{Ar2}R^{Ar3}, -COOR^{Ar2}, -SO₃R^{Ar2}, -SO₃R^{Ar2}, C₁-C₁₈-alkyl, C₂-C₁₈-alkenyl, C₂-C₁₈-alkynyl, C₁-C₁₂-alkoxy, C₁-C₁₂-alkylthio,
   U is an -O-, -S-, -NR^{Ar1-}, -CO-, -SO- or -SO₂- moiety;
   R^{Ar1}, R^{Ar2}, R^{Ar3} are each independently hydrogen, C₁-C₁₈-alkyl, 3- to 8-membered cycloalkyl, 3- to 8-membered heterocyclyl, aryl or heteroaryl, where alkyl is unsubstituted or bears one or more R^{a} groups, where 3- to 8-membered cycloalkyl, 3- to 8-membered heterocyclyl, aryl and heteroaryl are unsubstituted or bear one or more R^{b} groups;
   with the proviso that the compound of the formula II comprises at least one cyano group
   or mixtures thereof;
(ii) a cyanated perylene compound of the formula (III) 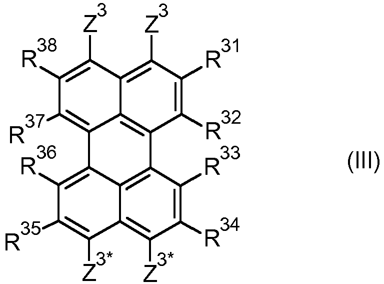 in which
   one of the Z³ substituents is cyano and the other Z³ substituent is CO₂R³⁹, CONR³¹⁰R³¹¹, C₁-C₁₈-alkyl, C₂-C₁₈-alkenyl, C₂-C₁₈-alkynyl, C₃-C₁₂-cycloalkyl or C₆-C₁₄-aryl, where
      C₁-C₁₈-alkyl, C₂-C₁₈-alkenyl, C₂-C₁₈-alkynyl are unsubstituted or bear one or more identical or different Z^{3a} substituents,
      C₃-C₁₂-cycloalkyl is unsubstituted or bears one or more identical or different Z^{3b} substituents, and
      C₆-C₁₄-aryl is unsubstituted or bears one or more identical or different Z^{3Ar} substituents;
   one of the Z^{3*} substituents is cyano and the other Z^{3*} substituent is CO₂R³⁹, CONR³¹⁰R³¹¹, C₁-C₁₈-alkyl, C₂-C₁₈-alkenyl, C₂-C₁₈-alkynyl, C₃-C₁₂-cycloalkyl or C₆-C₁₄-aryl, where
      C₁-C₁₈-alkyl, C₂-C₁₈-alkenyl, C₂-C₁₈-alkynyl are unsubstituted or bear one or more identical or different Z^{3a} substituents,
      C₃-C₁₂-cycloalkyl is unsubstituted or bears one or more identical or different Z^{3b} substituents, and
      C₆-C₁₄-aryl is unsubstituted or bears one or more identical or different Z^{3Ar} substituents;
   R³¹, R³², R³³, R³⁴, R³⁵, R³⁶, R3⁷ and R³⁸ are each independently selected from hydrogen, cyano, bromine and chlorine,
      with the proviso that 1, 2, 3, 4, 5, 6, 7 or 8 of the R³¹, R³², R³³, R³⁴, R³⁵, R³⁶, R³⁷ or R³⁸ substituents are cyano;
   where
   R³⁹ is hydrogen, C₁-C₁₀-alkyl, C₂-C₁₀-alkenyl, C₂-C₁₀-alkynyl, C₃-C₁₂-cycloalkyl or C₆-C₁₄-aryl, where
      C₁-C₁₀-alkyl, C₂-C₁₀-alkenyl, C₂-C₁₀-alkynyl are unsubstituted or bear one or more identical or different R^{3a} substituents,
      C₃-C₁₂-cycloalkyl is unsubstituted or bears one or more identical or different R^{3b} substituents and
      C₆-C₁₄-aryl is unsubstituted or bears one or more identical or different R^{3Ar} substituents;
   R³¹⁰ and R³¹¹ are each independently hydrogen, C₁-C₁₀-alkyl, C₂-C₁₀-alkenyl, C₂-C₁₀-alkynyl, C₃-C₁₂-cycloalkyl or C₆-C₁₄-aryl, where
      C₁-C₁₀-alkyl, C₂-C₁₀-alkenyl, C₂-C₁₀-alkynyl are unsubstituted or bear one or more identical or different R^{3a} substituents,
      C₃-C₁₂-cycloalkyl is unsubstituted or bears one or more identical or different R^{3b} substituents and
      C₆-C₁₄-aryl is unsubstituted or bears one or more identical or different R^{3Ar} substituents;
   each Z^{3a} is independently halogen, hydroxyl, NR^{310a}R^{311a}, C₁-C₁₀-alkoxy, C₁-C₁₀-haloalkoxy, C₁-C₁₀-alkylthio, C₃-C₁₂-cycloalkyl, C₆-C₁₄-aryl, C(=O)R^{39a};
      C(=O)OR^{39a} or C(O)NR^{310a}R^{311a}, where
      C₃-C₁₂-cycloalkyl is unsubstituted or bears one or more identical or different R^{3b} substituents and
      C₆-C₁₄-aryl is unsubstituted or bears one or more identical or different R^{3Ar} substituents;
   each Z^{3b} and each Z^{3Ar} is independently halogen, hydroxyl, NR^{310a}R^{311a}, C₁-C₁₀-alkyl, C₁-C₁₀-alkoxy, C₁-C₁₀-haloalkoxy, C₁-C₁₀-alkylthio, C(=O)R^{39a}; C(=O)OR^{39a} or C(O)NR^{310a}R^{311a};
   each R^{3a} is independently halogen, hydroxyl, C₁-C₁₀-alkoxy, C₃-C₁₂-cycloalkyl or C₆-C₁₄-aryl;
   each R^{3b} is independently halogen, hydroxyl, C₁-C₁₀-alkyl, C₁-C₁₀-alkoxy, C₁-C₁₀-haloalkoxy, C₁-C₁₀-alkylthio, C₂-C₁₀-alkenyl, C₂-C₁₀-alkynyl, C₃-C₁₂-cycloalkyl or C₆-C₁₄-aryl;
   each R^{3Ar} is independently halogen, hydroxyl, C₁-C₁₀-alkyl, C₁-C₁₀-alkoxy, C₁-C₁₀-haloalkoxy, C₁-C₁₀-alkylthio, C₂-C₁₀-alkenyl, C₂-C₁₀-alkynyl, C₃-C₁₂-cycloalkyl or C₆-C₁₄-aryl;
   R^{39a} is hydrogen, C₁-C₁₀-alkyl, C₂-C₁₀-alkenyl, C₂-C₁₀-alkynyl, C₃-C₁₂-cycloalkyl or C₆-C₁₄-aryl; and
   R^{310a} R^{311a} are each independently hydrogen, C₁-C₁₀-alkyl, C₂-C₁₀-alkenyl, C₂-C₁₀-alkynyl, C₃-C₁₂-cycloalkyl or C₆-C₁₄-aryl,
   and mixtures thereof.
(iii) a cyanated compound of the formula (IV) 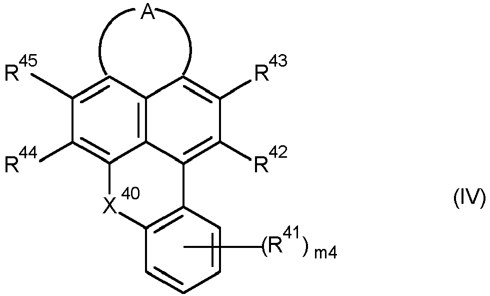 wherein
   - m4: is 0, 1, 2, 3 or 4;
   - each: R⁴¹ independently from each other is selected from bromine, chlorine, cyano, -NR^{4a}R^{4b}, C₁-C₂₄-alkyl, C₁-C₂₄-haloalkyl, C₁-C₂₄-alkoxy, C₁-C₂₄-haloalkoxy, C₃-C₂₄-cycloalkyl, heterocycloalkyl, heteroaryl, C₆-C₂₄-aryl, C₆-C₂₄-aryloxy, C₆-C₂₄-aryl-C₁-C₁₀-alkylene, where the rings of cycloalkyl, heterocycloalkyl, heteroaryl, aryl, aryloxy in the six last-mentioned radicals are unsubstituted or substituted with 1, 2, 3, 4 or 5 identical or different radicals R^{41a} and where C₁-C₂₄-alkyl, C₁-C₂₄-haloalkyl, C₁-C₂₄-alkoxy, and the alkylene moiety of C₆-C₂₄-aryl-C₁-C₁₀-alkylene may be interrupted by one or more groups selected from O, S and NR^{4c};
   at least one of the radicals R⁴², R⁴³, R⁴⁴ and R⁴⁵ is CN, and the remaining radicals, independently from each other, are selected from hydrogen, chlorine and bromine;
   - X⁴⁰: is O, S, SO or SO₂;
   - A: is a diradical selected from diradicals of the general formulae (A.1), (A.2), (A.3), and (A.4) 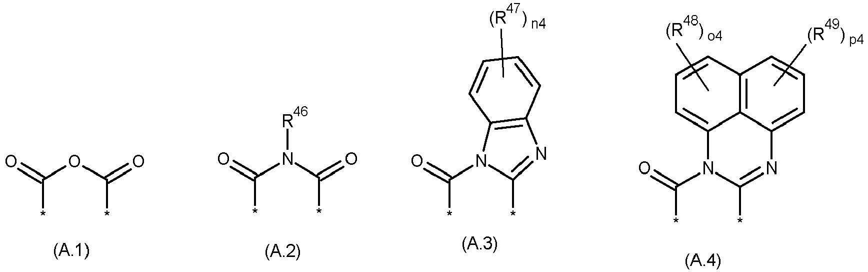
   wherein
   - *: in each case denotes the point of attachments to the remainder of the molecule;
   - n4: is 0, 1, 2, 3 or 4;
   - o4: is 0, 1, 2 or 3;
   - p4: is 0, 1, 2, or 3;
   - R⁴⁶: is hydrogen, C₁-C₂₄-alkyl, C₁-C₂₄-haloalkyl, C₃-C₂₄-cycloalkyl, C₆-C₂₄-aryl or C₆-C₂₄-aryl-C₁-C₁₀-alkylene, where the rings of cycloalkyl, aryl, and aryl-alkylene in the three last-mentioned radicals are unsubstituted or substituted with 1, 2, 3, 4 or 5 identical or different radicals R^{46a}, and where C₁-C₂₄-alkyl, C₁-C₂₄-haloalkyl and the alkylene moiety of C₆-C₂₄-aryl-C₁-C₁₀-alkylene may be interrupted by one or more heteroatoms or heteroatomic groups selected from O, S and NR^{4c};
   - each R⁴⁷: independently from each other is selected from bromine, chlorine, cyano, -NR^{4a}R^{4b}, C₁-C₂₄-alkyl, C₁-C₂₄-haloalkyl, C₁-C₂₄-alkoxy, C₁-C₂₄-haloalkoxy, C₃-C₂₄-cycloalkyl, heterocycloalkyl, heteroaryl, C₆-C₂₄-aryl, C₆-C₂₄-aryloxy, C₆-C₂₄-aryl-C₁-C₁₀-alkylene, where the rings of cycloalkyl, heterocycloalkyl, heteroaryl, aryl and aryl-alkylene in the six last-mentioned radicals are unsubstituted or substituted with 1, 2, 3, 4 or 5 identical or different radicals R^{47a} and where C₁-C₂₄-alkyl, C₁-C₂₄-haloalkyl, C₁-C₂₄-alkoxy, C₁-C₂₄-haloalkoxy, and the alkylene moiety of C₆-C₂₄-aryl-C₁-C₁₀-alkylene may be interrupted by one or more groups selected from O, S and NR^{4c};
   - each R⁴⁸: independently from each other is selected from bromine, chlorine, cyano, NR^{4a}R^{4b}, C₁-C₂₄-alkyl, C₁-C₂₄-haloalkyl, C₁-C₂₄-alkoxy, C₁-C₂₄-haloalkoxy, C₃-C₂₄-cycloalkyl, heterocycloalkyl, heteroaryl, C₆-C₂₄-aryl, C₆-C₂₄-aryloxy, C₆-C₂₄-aryl-C₁-C₁₀-alkylene, where the rings of cycloalkyl, heterocycloalkyl, heteroaryl, aryl and aryl-alkylene in the six last-mentioned radicals are unsubstituted or substituted with 1, 2, 3, 4 or 5 identical or different radicals R^{48a} and where C₁-C₂₄-alkyl, C₁-C₂₄-haloalkyl, C₁-C₂₄-alkoxy, C₁-C₂₄-haloalkoxy, and the alkylene moiety of C₆-C₂₄-aryl-C₁-C₁₀-alkylene may be interrupted by one or more groups selected from O, S and NR^{4c};
   - each R⁴⁹: independently from each other is selected from bromine, chlorine, cyano, NR^{4a}R^{4b}, C₁-C₂₄-alkyl, C₁-C₂₄-haloalkyl, C₁-C₂₄-alkoxy, C₁-C₂₄-haloalkoxy, C₃-C₂₄-cycloalkyl, heterocycloalkyl, heteroaryl, C₆-C₂₄-aryl, C₆-C₂₄-aryloxy, C₆-C₂₄-aryl-C₁-C₁₀-alkylene, where the rings of cycloalkyl, heterocycloalkyl, heteroaryl, aryl and aryl- alkylene in the six last-mentioned radicals are unsubstituted or substituted with 1, 2, 3, 4 or 5 identical or different radicals R^{49a} and where C₁-C₂₄-alkyl, C₁-C₂₄-haloalkyl, C₁-C₂₄-alkoxy, C₁-C₂₄-haloalkoxy, and the alkylene moiety of C₆-C₂₄-aryl-C₁-C₁₀-alkylene may be interrupted by one or more groups selected from O, S and NR^{4c};
   - R^{41a}, R^{46a}, R^{47a}, R^{48a}, R^{49a}: are independently of one another selected from C₁-C₂₄-alkyl, C₁-C₂₄-fluoroalkyl, C₁-C₂₄-alkoxy, fluorine, chlorine and bromine;
   - R^{4a}, R^{4b}, R^{4c}: are independently of one another are selected from hydrogen, C₁-C₂₀-alkyl, C₃-C₂₄-cycloalkyl, heterocycloalkyl, heteroaryl and C₆-C₂₄-aryl;
   and mixtures thereof;
(iv) a benzoxanthene compound of the formula (V) 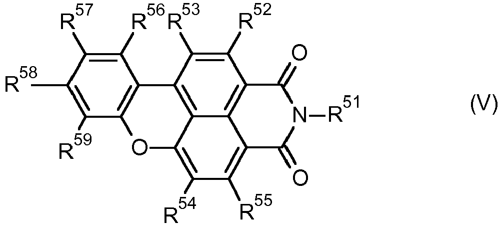 wherein
   - R⁵¹: is phenyl which is unsubstituted or carries 1, 2, 3, 4, or 5 substituents selected from halogen, R⁵¹¹, OR⁵⁵², NHR552 and NR⁵⁵²R⁵⁵⁷;
   - R52, R⁵³, R⁵⁴, R⁵⁵, R⁵⁶, R⁵⁷, R⁵⁸ and R⁵⁹: are independently of each other selected from hydrogen, halogen, R⁵⁵³, OR⁵⁵³, NHR⁵⁵³ and NR553R554,
   wherein
   - R⁵¹¹: is selected from C₂-C₁₈-alkyl, C₆-C₂₄-aryl and heteroaryl;
   - R⁵⁵² and R⁵⁵⁷: are independently of each other selected from C₁-C₁₈-alkyl, C₆-C₂₄-aryl and heteroaryl; and
   - R⁵⁵³ and R⁵⁵⁴: are independently of each other selected from C₁-C₁₈-alkyl, C₆-C₂₄-aryl and heteroaryl;
   and mixtures thereof;
(v) a fluorescent compound comprising at least one structural unit of formula (VI) 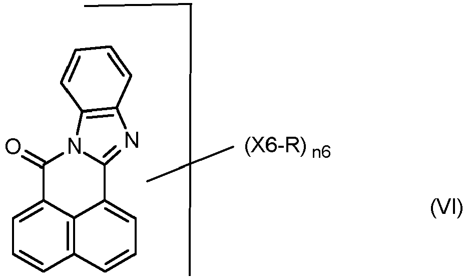 where one or more CH groups of the six-membered ring of the benzimidazole structure shown may be replaced by nitrogen and where the symbols are each defined as follows:
   - n6: is a number from 0 to (10-p6) for each structural unit of the formula (VI); where p6 is the number of CH units which have been replaced by nitrogen in the six-membered ring of the benzimidazole structure shown
   - X6: is a chemical bond, O, S, SO, SO₂, NR⁶¹; and
   - R: is an aliphatic radical, cycloaliphatic radical, aryl, hetaroaryl, each of which may bear any desired substituents,
   an aromatic or heteroaromatic ring or ring system, each of which is fused to other aromatic rings of the structural unit of the formula (VI) is F, Cl, Br, CN, H when X6 is not a chemical bond;
   where two R radicals may be joined to give one cyclic radical and
   where X6 and R, when n6 > one, may be the same or different;
   - R⁶¹: is each independently hydrogen, C₁-C₁₈-alkyl or cycloalkyl, the carbon chain of which may comprise one or more -O-, -S-, -CO-, -SO- and/or -SO₂- moieties and which may be mono- or polysubstituted; aryl or heteroaryl which may be mono- or polysubstituted;
   and mixtures thereof;
   and
(vi) a perylene compound of the formulae (VII), (VIII) or (IX) 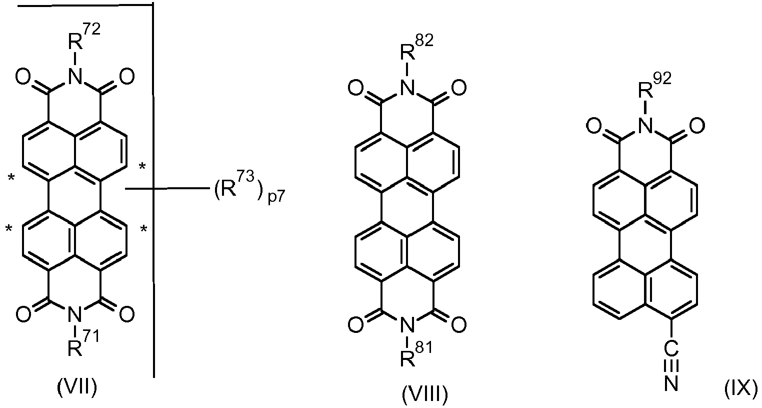 where
   - p7: is 1 to 4;
   - R⁷¹, R⁷²: are each independently C₁-C₃₀-alkyl, C₃-C₈-cycloalkyl, aryl, heteroaryl, aryl-C₁-C₁₀-alkylene, where the aromatic ring in the three latter radicals is unsubstituted or mono- or polysubstituted by C₁-C₁₀-alkyl;
   - R⁷³: is aryloxy which is unsubstituted or mono- or polysubstituted by C₁-C₁₀-alkyl, where the R⁷³ radicals are at one or more of the positions indicated by *;
   - R⁸¹, R⁸²: are each independently C₁-C₃₀-alkyl, C₃-C₈-cycloalkyl, aryl, heteroaryl, aryl-C₁-C₁₀-alkylene, where the aromatic ring in the three latter radicals is unsubstituted or mono- or polysubstituted by C₁-C₁₀-alkyl;
   - R⁹²: is C₁-C₃₀-alkyl, C₃-C₈-cycloalkyl, aryl, heteroaryl, aryl-C₁-C₁₀-alkylene, where the aromatic ring in the three latter radicals is unsubstituted or mono- or polysubstituted by C₁-C₁₀-alkyl;
and mixtures thereof.

Surprisingly, when the color converter comprises a combination of a compound of formulae (Ia) or (Ib) or a mixture thereof and at least one further organic fluorescent dye selected from a compound of formulae (II), (III), (IV), (V), (VI), (VII), (VIII), (IX) and mixtures thereof, a color converter is provided with high overall conversion efficiency. In addition, the color converter according to the invention together with blue light of a LED is able to provide white-light having any color temperature in the range between 2000 K and 7500 K. In addition, the light of the LED source is converted to longer wavelength with a high overall energy efficiency.

Fluorescent dyes of the formula (II) are known from WO 2015/019270. Among the compounds of formula (II), particular preference is given to those of the general formula II-A 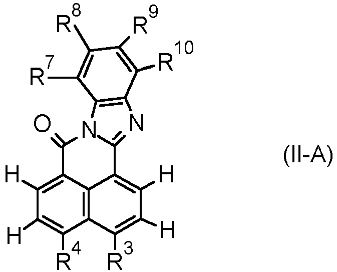 and mixtures thereof in which
R³ and R⁴ are each independently cyano, phenyl, 4-cyanophenyl or phenyl which carries 1, 2 or 3 substituents selected from C₁-C₁₀-alkyl, especially cyano, phenyl or 4-cyanophenyl; and
R⁷, R⁸, R⁹ and R¹⁰ are each independently hydrogen, cyano, phenyl, 4-cyanophenyl or phenyl which carries 1, 2 or 3 substituents selected from C₁-C₁₀-alkyl, especially hydrogen, cyano, phenyl or 4-cyanophenyl.

Compounds in turn preferred among the compounds of the formula II-A are those in which
R³ is cyano;
R⁴ is phenyl, 4-cyanophenyl or phenyl which carries 1, 2 or 3 substituents selected from C₁-C₁₀-alkyl; and
two of the radicals R⁷, R⁸, R⁹ and R¹⁰ are each independently, phenyl, 4-cyanophenyl or phenyl which carries 1, 2 or 3 substituents selected from C₁-C₁₀-alkyl and the other radicals R⁷, R⁸, R⁹ and R¹⁰ are hydrogen.

Likewise, compounds in turn preferred among the compounds of the formula II-A are also those which correspond to the formulae II-Ab and II-Ab' 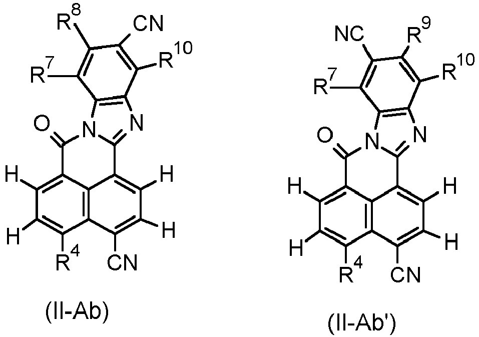 in which
R⁴ is phenyl, 4-cyanophenyl or phenyl which carries 1, 2 or 3 substituents selected from C₁-C₁₀-alkyl; and
zero, one or two of the radicals R⁷, R¹⁰, if present, R⁸ and R⁹ are each independently, phenyl, 4-cyanophenyl or phenyl which carries 1, 2 or 3 substituents selected from C₁-C₁₀-alkyl and the other radicals R⁷, R¹⁰, R⁸, R⁹, if present, are hydrogen.

Compounds in turn preferred among the compounds of the formula II-A are also those which correspond to the formula II-Ac 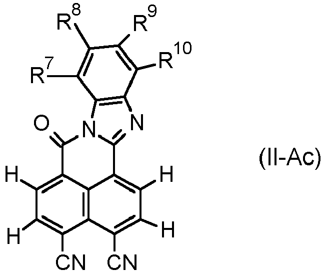 in which
one or two of the radicals R⁷, R⁸, R⁹ and R¹⁰ are each independently, phenyl, 4-cyanophenyl or phenyl which carries 1, 2 or 3 substituents selected from C₁-C₁₀-alkyl and the other radicals R⁷, R⁸, R⁹ and R¹⁰ are hydrogen.

Compounds in turn preferred among the compounds of the formula II-A are also those which correspond to the formula II-Ad and II-Ad' 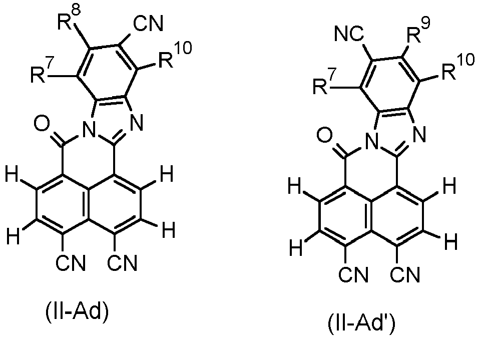 in which
one or two of the radicals R⁷, R⁸, R⁹ and R¹⁰ are each independently, phenyl, 4-cyanophenyl or phenyl which carries 1, 2 or 3 substituents selected from C₁-C₁₀-alkyl and the other radicals R⁷, R⁸, R⁹ and R¹⁰ are hydrogen.

Especially preferably, the at least one cyanated naphthalenebenzimidazole compound of the formula II is selected from the compounds of the formulae (II-1), (II-2), (II-3), (II-4), (II-5), (II-6), (II-7), (II-8), (II-9), (II-10), (II-11), (II-12), (II-13), (II-14), (II-15), (II-16), (II-17), (II-18), (II-19), (II-20), (II-21), (II-22), (II-23), (II-24), (II-25), (II-26), (II-27), (II-28), (II-29), (II-30), (II-31), (II-32), (II-33), (II-34), (II-35), (II-36), (II-37), (II-38), (II-39), (II-40), (II-41), (II-42), (II-43), (II-44), (II-45), (II-46), (II-47), (II-48), (II-49), and (II-50) and mixtures thereof

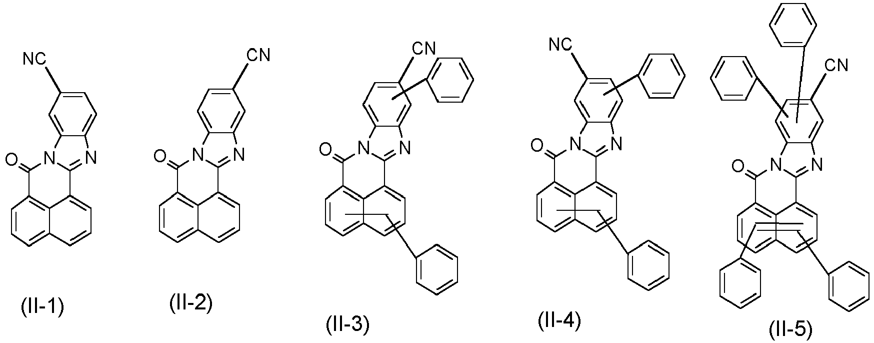

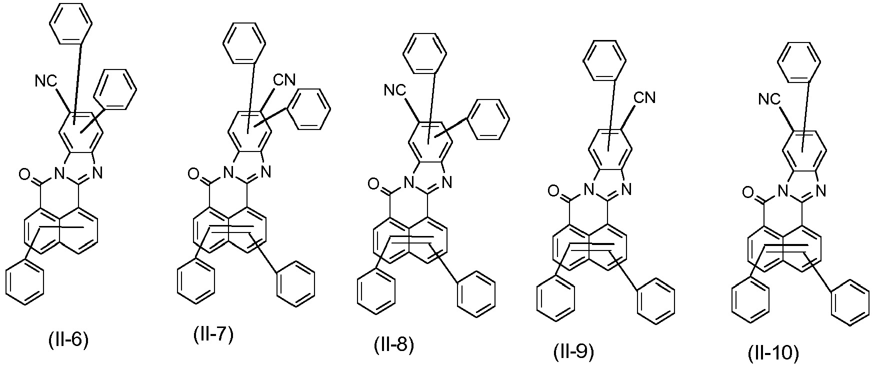

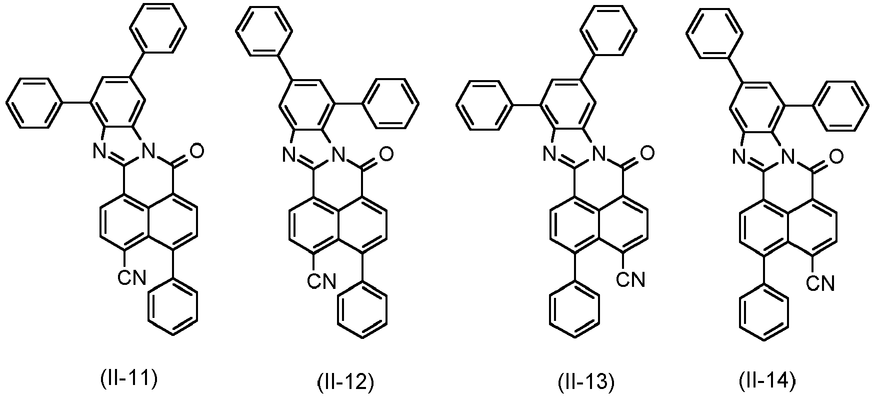

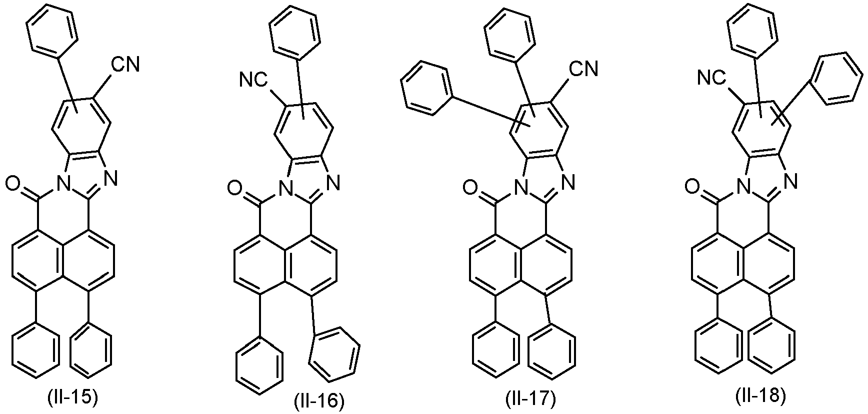

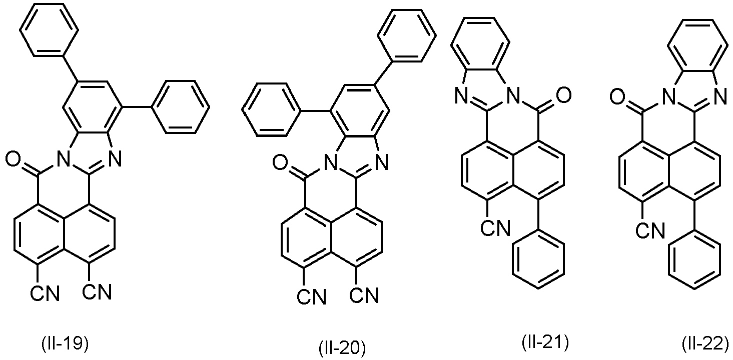

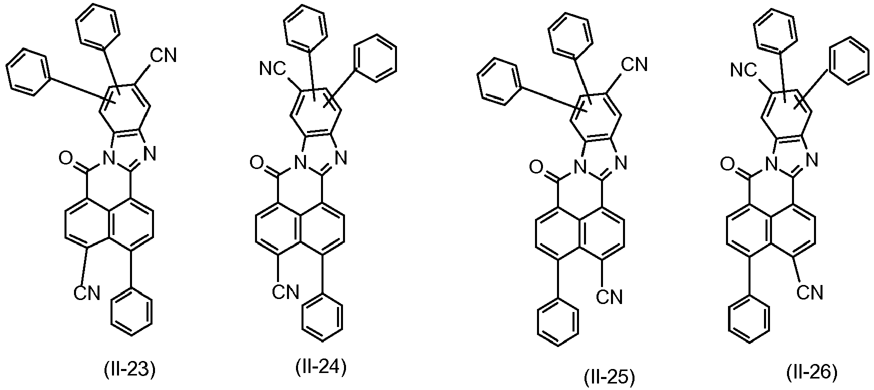

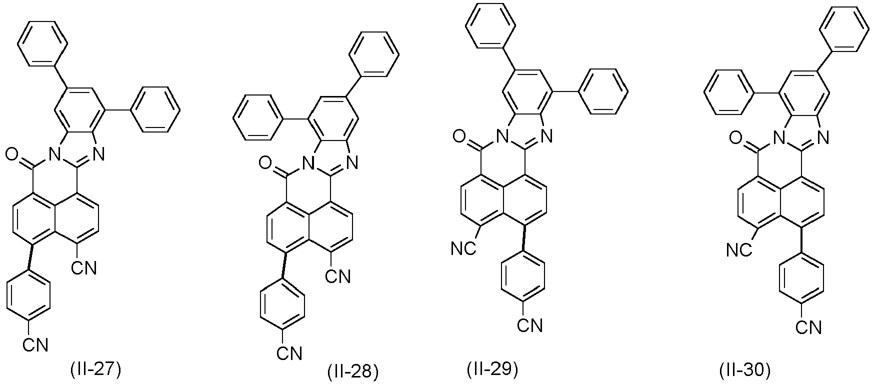

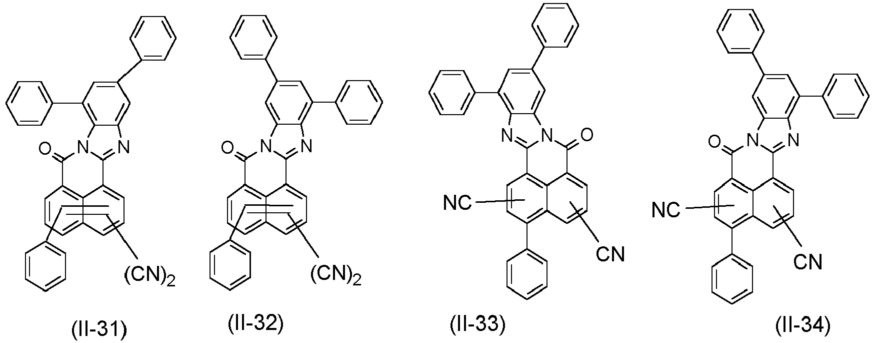

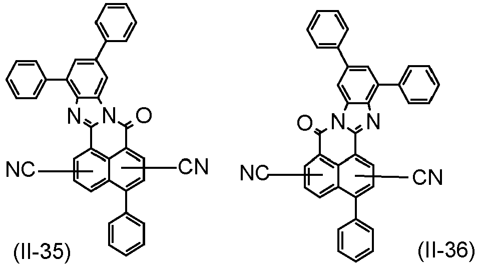

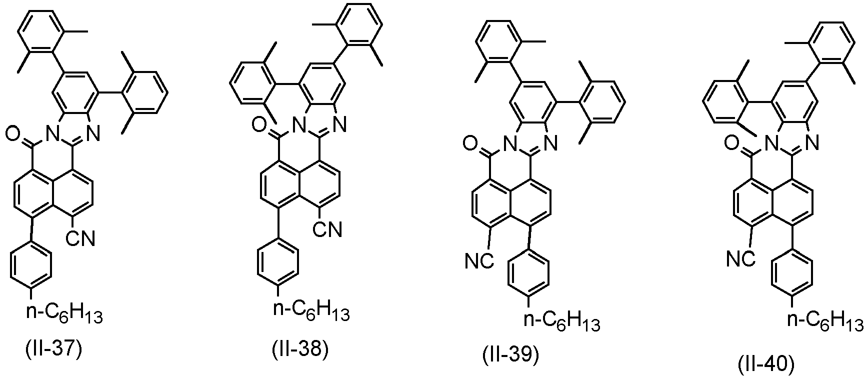

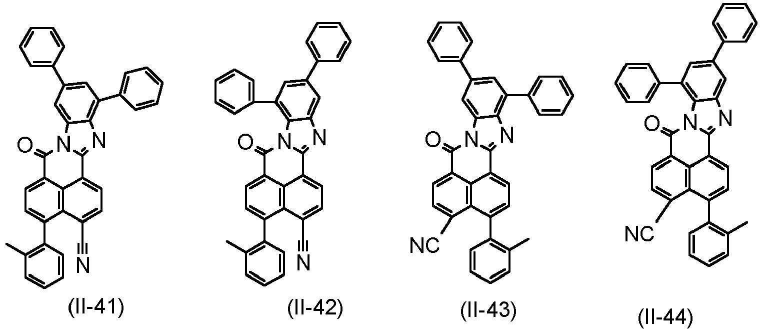

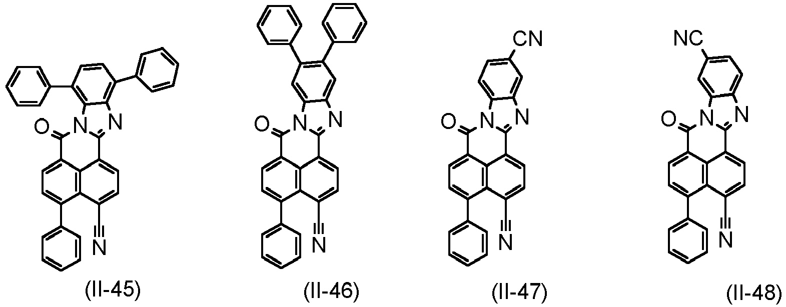

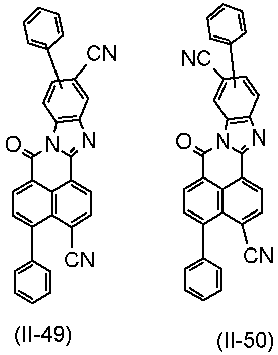

Compounds of the formula (II) are usually green, yellow-green or yellow fluorescent dyes.

Compounds of formula (III) are known from WO 2015/169935 (PCT/EP2015/060137). Compounds of formula (III) encompass the following compounds of the formulae III-a and III-b as well as compounds of formulae III-c and IIII-d: 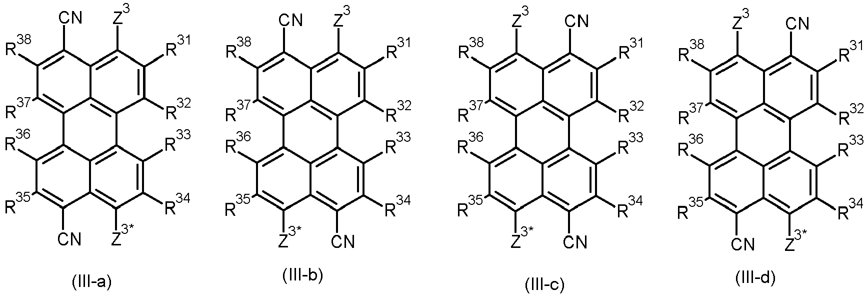 in which
R³¹, R³², R³³, R³⁴, R³⁵, R³⁶, R³⁷, R³⁸, Z³ and Z^{3*} are each as defined above,
individually and mixtures thereof.

More particularly, in compounds of the formulae III-a, III-b, III-c and III-d, the R³¹, R³², R³³, R³⁴, R³⁵, R³⁶, R³⁷, R³⁸ substituents that are not cyano or hydrogen are all chlorine or all bromine.

In the perylene compounds of the formula III and mixtures thereof, 1, 2, 3, 4, 5, 6, 7 or 8 of the R³¹, R³², R³³, R³⁴, R³⁵, R³⁶, R³⁷ and R³⁸ substituents are cyano. The other R³¹, R³², R³³ R³⁴, R³⁵, R³⁶, R³⁷ and R³⁸ substituents are each independently hydrogen, bromine or chlorine. More particularly, the other R³¹, R³², R³³, R³⁴, R³⁵, R³⁶, R³⁷ and R³⁸ substituents are each independently hydrogen or bromine. In a further embodiment, the other R³¹, R³², R³³, R³⁴, R³⁵, R³⁶, R³⁷ and R³⁸ substituents are each independently hydrogen or chlorine.

Preferably, 1, 2, 3 or 4 of the R³¹, R³², R³³, R³⁴, R³⁵, R³⁶, R³⁷ and R³⁸ substituents are cyano. The other R³¹, R³², R³³, R³⁴, R³⁵, R³⁶, R³⁷ and R³⁸ substituents are each independently hydrogen, bromine or chlorine. Specifically, 1, 2, 3 or 4 of the R³², R³³, R³⁶ and R³⁷ substituents are cyano. In a specific embodiment, none of the R³¹, R³², R³³, R34, R³⁵, R³⁶, R³⁷ or R³⁸ substituents is bromine or chlorine.

In a further preferred embodiment, one of the R³¹, R³², R³³, R³⁴, R³⁵, R³⁶, R³⁷ and R³⁸ substituents is cyano, and the other R³¹, R³², R³³, R³⁴, R³⁵, R³⁶, R³⁷ and R³⁸ substituents are hydrogen. In particular, one of the R³², R³³, R³⁶ and R³⁷ substituents is cyano, and the other R³¹, R³², R³³, R³⁴, R³⁵, R³⁶, R³⁷ and R³⁸ substituents are hydrogen.

In a further preferred embodiment, two of the R³¹, R³², R³³, R³⁴, R³⁵, R³⁶, R³⁷ and R³⁸ substituents are cyano, and the other R³¹, R³², R³³, R³⁴, R³⁵, R³⁶, R³⁷ and R³⁸ substituents are hydrogen. In particular, two of the R³², R³³, R³⁶ and R³⁷ substituents are cyano, and the other R³¹, R³², R³³, R³⁴, R³⁵, R³⁶, R³⁷ and R³⁸ substituents are hydrogen.

In the perylene compounds of the formula III and mixtures thereof, one of the Z³ substituents is cyano and one of the Z^{3*} substituents is cyano. The other Z³ substituent and the other Z^{3*} substituent are each as defined above and are preferably selected independently from C₁-C₁₀-alkyl, CO₂R³⁹, phenyl-C₁-C₁₀-alkyl and phenyl, where phenyl and the phenyl moiety of phenyl-C₁-C₁₀-alkyl are unsubstituted or bear one or more, for example 1, 2 or 3, substituents selected from C₁-C₆-alkyl, where R³⁹ is as defined above. Preferably, R³⁹ is linear or branched C₁-C₆-alkyl, specifically methyl, ethyl, n-propyl, isopropyl, n-butyl or isobutyl.

Even more preferably, one of the Z³ substituents is C₁-C₆-alkyl, C₁-C₆-alkoxycarbonyl or phenyl which is unsubstituted or bears 1, 2 or 3 C₁-C₄-alkyl groups. Specifically, one of the Z³ substituents is C₁-C₆-alkyl such as methyl, ethyl, n-propyl, isopropyl, n-butyl, isobutyl, C₁-C₆-alkoxycarbonyl such as methoxycarbonyl, ethoxycarbonyl, n-propoxycarbonyl, isopropoxycarbonyl, n-butoxycarbonyl, isobutoxycarbonyl, phenyl, or phenyl bearing 1, 2 or 3 C₁-C₄-alkyl groups, such as 2-methylphenyl or 2,6-dimethylphenyl.

Even more preferably, one of the Z^{3*} substituents is C₁-C₆-alkyl, C₁-C₆-alkoxycarbonyl or phenyl which is unsubstituted or bears 1, 2 or 3 C₁-C₄-alkyl groups. Specifically, one of the Z^{3*} substituents is C₁-C₆-alkyl such as methyl, ethyl, n-propyl, isopropyl, n-butyl, isobutyl, C₁-C₆-alkoxycarbonyl such as methoxycarbonyl, ethoxycarbonyl, n-propoxycarbonyl, isopropoxycarbonyl, n-butoxycarbonyl, isobutoxycarbonyl, phenyl, 2-methylphenyl or 2,6-dimethylphenyl.

Especially, the perylene compound of the formula IIII is selected from compounds of the formulae (III-1), (III-2), (III-3), (III-4), (III-5), (III-6), (III-7), (III-8), (III-9), (III-10), (III-11), (III-12), (III-13), (III-14), (III-15), (III-16), (III-17), (III-18), (III-19), (III-20) 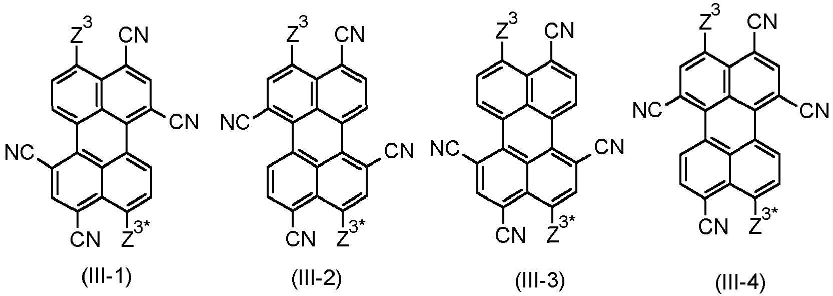 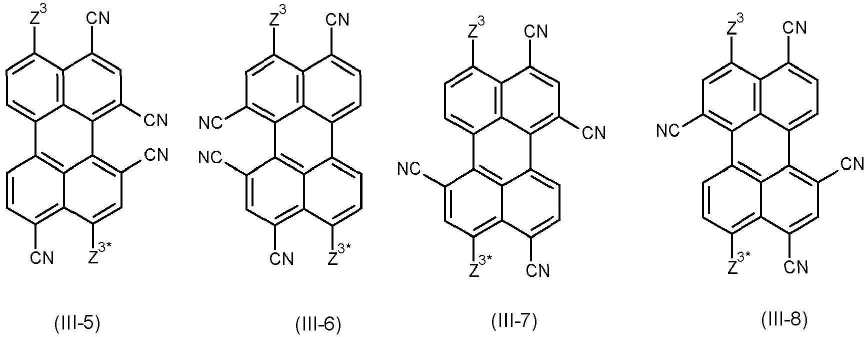 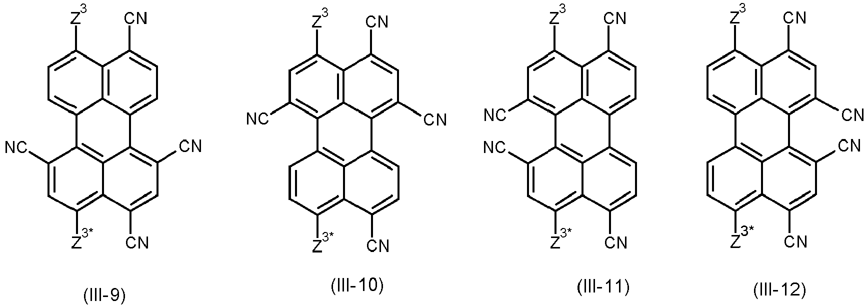 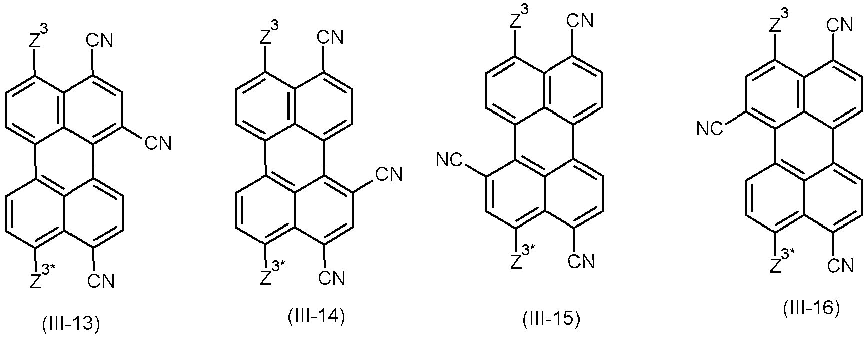 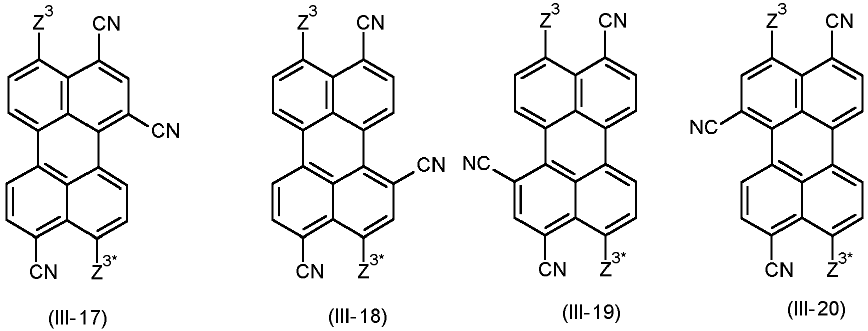 in which
- Z³: is selected from C₁-C₆-alkyl, C₁-C₆-alkoxycarbonyl, phenyl, and phenyl bearing 1, 2 or 3 C₁-C₄-alkyl groups; and
- Z^{3*}: is selected from C₁-C₆-alkyl, C₁-C₆-alkoxycarbonyl, phenyl, and phenyl bearing 1, 2 or 3 C₁-C₄-alkyl groups;
and mixtures thereof.

Among these, specific preference is given to perylene compounds of the formulae (III-1), (III-2), (III-3), (III-4), (III-5), (III-6), (III-7), (III-8), (III-9), (III-10), (III-11), (III-12), (III-13), (III-14), (III-15), (III-16), (III-17), (III-18), (III-19), (III-20) in which Z and Z* have the same definition.

A compound of the formula (III) corresponding to a compound of formula (III-A) 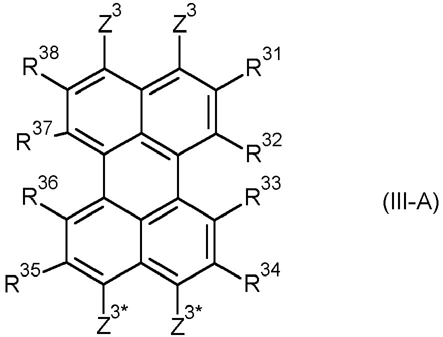 wherein
one of the Z³ substituents is cyano and the other Z³ substituent is C₁-C₁₈-alkyl, C₂-C₁₈-alkenyl, C₂-C₁₈-alkynyl, C₃-C₁₂-cycloalkyl or C₆-C₁₄-aryl, where C₁-C₁₈-alkyl, C₂-C₁₈-alkenyl, C₂-C₁₈-alkynyl are unsubstituted or bear one or more identical or different Z^{3a} substituents, where Z^{3a} is as defined above;
   C₃-C₁₂-cycloalkyl is unsubstituted or bears one or more identical or different Z^{3b} substituents, where Z^{3b} is as defined above; and C₆-C₁₄-aryl is unsubstituted or bears one or more identical or different Z^{3Ar} substituents, where Z^{3Ar} is as defined above;
one of the Z^{3*} substituents is cyano and the other Z^{3*} substituent is C₁-C₁₈-alkyl, C₂-C₁₈-alkenyl, C₂-C₁₈-alkynyl, C₃-C₁₂-cycloalkyl or C₆-C₁₄-aryl, where C₁-C₁₈-alkyl, C₂-C₁₈-alkenyl, C₂-C₁₈-alkynyl are unsubstituted or bear one or more identical or different Z^{3a} substituents, where Z^{3a} is as defined above; C₃-C₁₂-cycloalkyl is unsubstituted or bears one or more identical or different Z^{3b} substituents, where Z^{3b} is as defined above; and C₆-C₁₄-aryl is unsubstituted or bears one or more identical or different Z^{3Ar} substituents, where Z^{3Ar} is as defined above;
R³¹ R³⁴, R³⁵ and R³⁸ are each hydrogen;
two of the R³², R³³, R³⁶ or R³⁷ substituents are hydrogen; and the other R³², R³³, R³⁶ or R³⁷ substituents are cyano;
or mixtures thereof,
is obtainable by a process in which
a) perylene of formula (III-II) 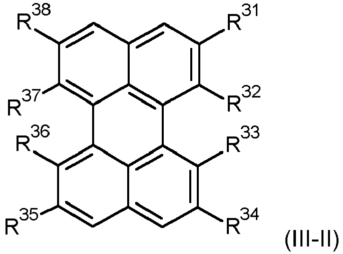 in which
   R³¹, R³², R³³, R³⁴, R³⁵, R³⁶, R³⁷ and R³⁸ are each hydrogen
   is halogenated to obtain a mixture of 3,9-dihaloperylene of the formula (III-IIIa) and 3,10-dihaloperylene of the formula (III-IIIb) 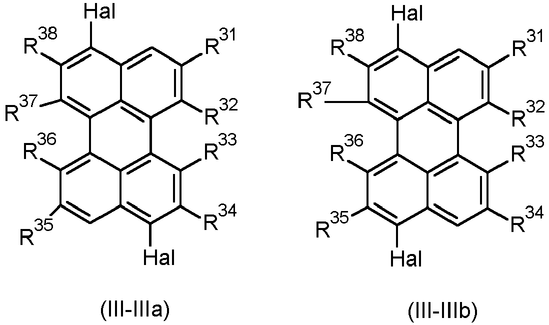 in which
   R³¹, R³², R³³, R³⁴, R³⁵, R³⁶, R³⁷ and R³⁸ are each hydrogen; and
   Hal are each all chlorine or bromine;
b) the mixture of compounds of the formulae IIIa and IIIb obtained in step a) is reacted with an organometallic compound of the formula (III-IV)

   Z³-Met (III-IV)

   and optionally with an organometallic compound of the formula (III-V)

   Z^{3*}-Met (III-V)

   in which
   - Z³: is selected from C₁-C₁₈-alkyl, C₂-C₁₈-alkenyl, C₂-C₁₈-alkynyl, C₃-C₁₂-cycloalkyl and C₆-C₁₄-aryl, where C₁-C₁₈-alkyl, C₂-C₁₈-alkenyl, C₂-C₁₈-alkynyl are unsubstituted or bear one or more identical or different Z^{3a} substituents,
   C₃-C₁₂-cycloalkyl is unsubstituted or bears one or more identical or different Z^{3b} substituents, and C₆-C₁₄-aryl is unsubstituted or bears one or more identical or different Z^{3Ar} substituents;
   - Z^{3*}: is selected from C₁-C₁₈-alkyl, C₂-C₁₈-alkenyl, C₂-C₁₈-alkynyl, C₃-C₁₂-cycloalkyl and C₆-C₁₄-aryl, where C₁-C₁₈-alkyl, C₂-C₁₈-alkenyl, C₂-C₁₈-alkynyl are unsubstituted or bear one or more identical or different Z^{3a} substituents,
   C₃-C₁₂-cycloalkyl is unsubstituted or bears one or more identical or different Z^{3b} substituents, and
   C₆-C₁₄-aryl is unsubstituted or bears one or more identical or different Z^{3Ar} substituents;
   where Z^{3*} may also be as defined for Z³;
   - Met: is B(OH)₂, B(OR^{3'})(OR^{3"}), Zn-Hal or Sn(R^{3*})₃,
   in which
   R^{3'} and R^{3"} are each independently hydrogen, C₁-C₃₀-alkyl, C₅-C₈-cycloalkyl, C₆-C₁₄-aryl or heteroaryl, or R^{3'} and R^{3"} together are C₂-C₄-alkylene which optionally bears 1, 2, 3, 4, 5, 6, 7 or 8 substituents selected from C₁-C₄-alkyl, C₅-C₈-cycloalkyl, C₆-C₁₄-aryl and heteroaryl,
   Hal is chlorine or bromine, and
   R^{3*} is C₁-C₈-alkyl or phenyl,
   to obtain a mixture of compounds of the formulae (III-VIa) and (III-VIb), 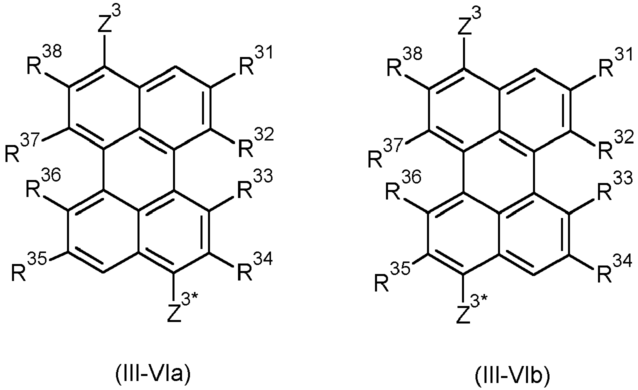 in which
   R³¹, R³², R³³, R³⁴, R³⁵, R³⁶, R³⁷ and R³⁸ are each hydrogen; and
   Z³ and Z^{3*} are each as defined above;
c) the mixture of compounds of the formulae (III-VIa) and (III-VIb) obtained in step b) is halogenated to obtain a reaction mixture which comprises compounds of the formulae (III-VIIa) and (III-VIIb) 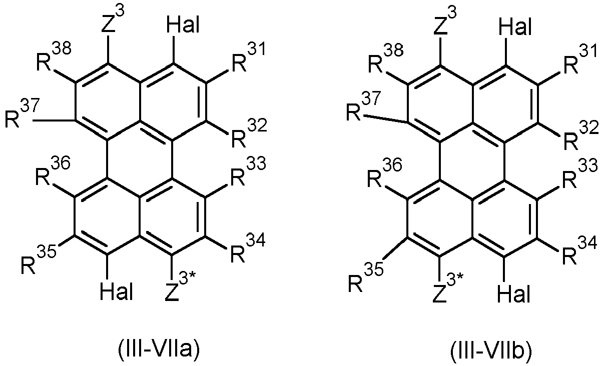 in which
   - Z³ and Z^{3*}: are each as defined above;
   - Hal: is halogen selected from chlorine and bromine, where the Hal substituents are either all chlorine or all bromine,
   - R³¹, R³², R³³, R³⁴, R³⁵, R³⁶, R³⁷ and R³⁸: are each hydrogen or halogen selected from chlorine and bromine, where the R³¹, R²³, R³³, R³⁴, R³⁵, R³⁶, R³⁷ and R³⁸ substituents that are not hydrogen are either all chlorine or all bromine;
d) the compounds of the formulae (III-VIIa) and (III-VIIb) present in the reaction mixture obtained in step c) are subjected to a substitution of halogen for cyano, and optionally partly for hydrogen, to obtain at least one compound of the formula (III-A) or mixtures thereof; and
e) the at least one compound of the formula (III-A) or mixtures thereof present in the reaction mixture obtained in step d) is optionally subjected to at least one separation and/or purification step.

### Step a)

The halogenation of perylene of the formula (III-II) is effected typically with a brominating agent or a chlorinating agent, meaning that either all the Hal substituents in the compounds of the formulae (III-IIIa) or (III-IIIb) are bromine or all the Hal substituents are chlorine.

Typically, elemental bromine in a solvent is used as the brominating agent. Further suitable brominating agents are N-bromosuccinimide and dibromoisocyanuric acid. Suitable solvents are water or aliphatic monocarboxylic acids, and chlorinated hydrocarbons such as chlorobenzene and chloroform. Suitable aliphatic monocarboxylic acids are those having 2 to 6 carbon atoms, such as acetic acid, propionic acid, butyric acid, pentanecarboxylic acid and hexanecarboxylic acid, and mixtures thereof. When an aliphatic monocarboxylic acid is used as a solvent, it may be advantageous to use iodine as a catalyst.

Suitable chlorinating agents are chlorine in a solvent, e.g. tetrachloromethane. Likewise suitable are N-chlorosuccinimide and dichloroisocyanuric acid. Chlorination with dichloroisocyanuric acid is effected preferably in concentrated sulfuric acid.

The molar ratio of brominating agent to perylene of the formula (III-II) is typically about 10:1 to 2.5:1, more preferably 9:1 to 3.0:1. The molar ratio is especially 8.5:1 to 3.5:1.

The molar ratio of chlorinating agent to perylene of the formula (III-II) is typically about 10:1 to 2.5:1, more preferably 9:1 to 3.0:1. The molar ratio is especially 8.5:1 to 3.5:1.

The dihalogenated compounds of the formulae (III-IIIa) and (III-IIIb) obtained in reaction step a) are generally used in step b) without further purification.

### Step b)

In the reaction in step b), the compounds of the formulae (III-IIIa) and (III-IIIb) obtained in step a) are subjected to a cross-coupling with an organometallic compound of the formula (III-IV) and optionally with an organometallic compound of the formula (III-V).

Preference is given to effecting the reaction in the presence of catalytically active amounts of a transition metal of transition group VIII of the Periodic Table (group 10 according to IUPAC), for example nickel, palladium or platinum, especially in the presence of a palladium catalyst. Suitable catalysts are, for example, palladium-phosphine complexes such as tetrakis(triphenylphosphine)palladium(0), PdCl₂(o-tolyl₃P)₂, bis(triphenylphosphine)palladium(II) chloride, the [1,1'-bis(diphenyl-phosphino)ferrocene]palladium(II) chloride-dichloromethane complex, bis[1,2-bis(di-phenylphosphino)ethane]palladium(0) and [1,4-bis(diphenylphosphino)butane]-palladium(II) chloride, palladium on activated carbon in the presence of phosphine compounds, and palladium(II) compounds such as palladium(II) chloride or bis(acetonitrile)palladium(II) chloride in the presence of phosphine compounds such as triphenylphosphine, 1,1'-bis(diphenylphosphino)ferrocene, 1,2-bis(diphenylphosphino)-ethane, 1,3-bis(diphenylphosphino)propane and 1,4-bis(diphenylphosphino)butane. The amount of catalyst is typically 10 to 150 mol%, based on the compounds of the formulae (III-IIIa) and (III-IIIb).

Especially suitable organometallic compounds (III-IV) are an appropriately substituted arylboronic acid and arylboronic esters (compounds III-IV where Met = B(OH)₂ or B(OR^{3'})(OR^{3"}) where R^{3'}, R^{3"} = C₁-C₄-alkyl, or R^{3'} and R^{3"} together are C₂-C₄-alkylene optionally bearing 1, 2, 3 or 4 substituents selected from C₁-C₄-alkyl).

The reaction is effected under the conditions of a Suzuki coupling, as known, for example, from Suzuki et al., Chem. Rev., 1995, 95, 2457-2483 and the literature cited therein. The arylboronic acids and esters thereof are known from the literature, commercially available, or can be prepared from the corresponding arylmagnesium compounds by reaction with appropriate boric esters. Suitable organometallic compounds (III-IV) are additionally alkylboronic acid or alkylboronic esters.

Suitable organometallic compounds (III-IV) are especially also arylstannanes, cycloalkylstannanes, alkynylstannanes, alkenylstannanes or alkylstannanes (compounds (III-IV) where Met = Sn(R^{3*})₃ where R^{3*} = C₁-C₄-alkyl). In that case, the reaction is effected under the conditions of a Stille coupling, as known, for example, from D. Milstein, J. K. Stille, J. Am. Chem. Soc. 1978, 100, p. 3636-3638 or V. Farina, V. Krishnamurthy, W. J. Scott, Org. React. 1997, 50, 1-652. Stannanes of the formula (III-IV) are either known or can be prepared by commonly known processes.

Suitable organometallic compounds (III-IV) are additionally organozinc compounds (compounds (III-IV) where Met = Zn-Hal where Hal = Cl, Br, especially Br). In that case, the reaction is effected under the conditions of a Negishi coupling, as known, for example, from A. Lützen, M. Hapke, Eur. J. Org. Chem., 2002, 2292-2297. Arylzinc compounds of the formula (III-IV) or alkylzinc compounds of the formula (III-IV) are either known or can be prepared by commonly known processes.

The reaction of (III-IIIa) and (III-IIIb) with the organometallic compound (III-IV), especially in the case of the Suzuki coupling, is effected under basic conditions. Suitable bases are alkali metal carbonates and alkali metal hydrogencarbonates such as sodium carbonate, potassium carbonate, cesium carbonate, sodium hydrogencarbonate, alkaline earth metal carbonates and alkaline earth metal hydrogencarbonates such as magnesium carbonate or magnesium hydrogencarbonate, or tertiary amines such as triethylamine, trimethylamine, triisopropylamine or N-ethyl-N-diisopropylamine.

Typically, the coupling of the compounds (III-IIIa) and (III-IIIb) with the compound (III-IV) is effected in a solvent. Suitable solvents are organic solvents such as aromatics, e.g. toluene, mesitylene, acyclic ethers, e.g. 1,2-dimethoxyethane, cyclic ethers such as tetrahydrofuran or 1,4-dioxane, polyalkylene glycols such as diethylene glycol, carbonitriles such as acetonitrile, propionitrile, carboxamides such as dimethylformamide or dimethylacetamide. In the Suzuki coupling, the aforementioned solvents can also be used in a mixture with water; for example, the ratio of organic solvent to water may be in the range from 5:1 to 1:5.

At least one mole of the organometallic compound (III-IV) is used per mole of halogen atom to be exchanged. It may be advantageous to use a 5 to 30% molar excess of organometallic compound of the formula (III-IV) per mole of halogen atom to be exchanged.

If Z³ is different than Z^{3*}, a further coupling with an organometallic compound of the formula V is subsequently conducted. In terms of the process, the procedure is as in the reaction of the compound of the formulae (III-IIIa) and (III-IIIb) with the organometallic compound (III-IV).

### Step c)

The halogenation of compounds of the formulae (III-VIa) and (III-VIb) is typically effected with a brominating agent or a chlorinating agent. Suitable brominating agents or chlorinating agents are those mentioned in step a). In general, the molar ratio of brominating agent to compound of the formulation (III-VIa) and (III-VIb) to be halogenated is 10:1 to 30:1, preferably 15:1 to 25:1.

Step c) of the process according to the invention is typically undertaken in the presence of a solvent at elevated temperatures. Suitable solvents are aprotic solvents such as halogenated aromatics such as chlorobenzene or dichlorobenzenes or halogenated hydrocarbons. Also suitable are aqueous aprotic solvents.

It may be advantageous to conduct step c) in the presence of catalytic amounts of iodine.

The reaction temperature in step c) is typically 50°C up to the boiling temperature of the solvent, in particular 80 to 150°C.

### Step d)

Suitable process conditions for cyano-dehalogenation are described in J. March, Advanced Organic Chemistry, 4th edition, John Wiley & Sons Publishers (1992), p. 660-661, and in WO 2004/029028. One example is the reaction with copper cyanide. Additionally suitable are alkali metal cyanides such as potassium cyanides and sodium cyanide, and also zinc cyanide. Typically, the cyanide source is used in excess. The reaction is generally effected in polar aprotic solvents in the presence of transition metals such as Pd(II) salts or Pd complexes, copper complexes or nickel complexes. The palladium catalyst can be prepared in situ from Pd(0) complexes such as tris(dibenzylideneacetone)dipalladium(0) and 1,1'-bis(diphenylphosphino)ferrocene. Preferred polar aprotic solvents are dimethylformamide, N-methylpyrrolidone, (CH₃)₂SO, dimethyl sulfone and sulfolane. The reaction is performed typically at temperatures of 80 to 160°C, preferably 100 to 140°C, especially preferably 130 to 150°C. The molar ratio of halogen atom to be exchanged to zinc cyanide is typically 1:1 to 1:3, preferably 1.5:2.5. Alternatively, it is also possible to use copper cyanide in N-methylpyrrolidone or sulfolane in the absence of a catalyst.

### Step e)

Optionally, the reaction mixture obtained in step d), comprising at least one perylene compound of the formula III-A or mixtures thereof, is subjected to a partial or complete separation and/or a purifying step. The separation and/or purification in step e) can be effected by customary processes known to those skilled in the art, such as extraction, distillation, recrystallization, separation on suitable stationary phases, and a combination of these measures.

It may be advantageous to undertake a partial or full separation of the isomers obtained after reaction step a) and/or b) and/or c).

A cyanated perylene compound of the formula III corresponding to the formula III-B 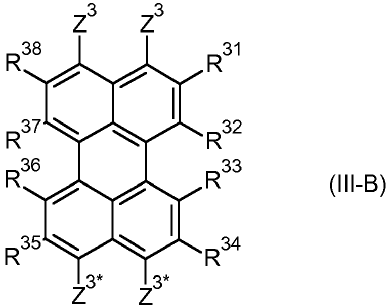 in which
one of the Z³ substituents is cyano and the other Z³ substituent is COOR³⁹;
one of the Z^{3*} substituents is cyano and the other Z^{3*} substituent is COOR³⁹;
R³¹, R³⁴, R³⁵ and R³⁸ are hydrogen;
one of the R³², R³³, R³⁶ or R³⁷ substituents is cyano and the other R³², R³³, R³⁶ and R³⁷ substituents are hydrogen;
R³⁹ is hydrogen, C₁-C₁₀-alkyl, C₂-C₁₀-alkenyl, C₂-C₁₀-alkynyl, C₃-C₁₂-cycloalkyl or C₆-C₁₄-aryl, where
   C₁-C₁₀-alkyl, C₂-C₁₀-alkenyl, C₂-C₁₀-alkynyl are unsubstituted or bear one or more identical or different R^{3a} substituents,
   C₃-C₁₂-cycloalkyl is unsubstituted or bears one or more identical or different R^{3b} substituents and
   C₆-C₁₄-aryl is unsubstituted or bears one or more identical or different R^{3Ar} substituents,
where R^{3a}, R^{3b} and R^{3Ar} are each as defined above,
or mixtures thereof,
is obtainable by a process in which
f) a mixture of perylene compounds of the formulae (III-VIIIa) and (III-Vlllb) 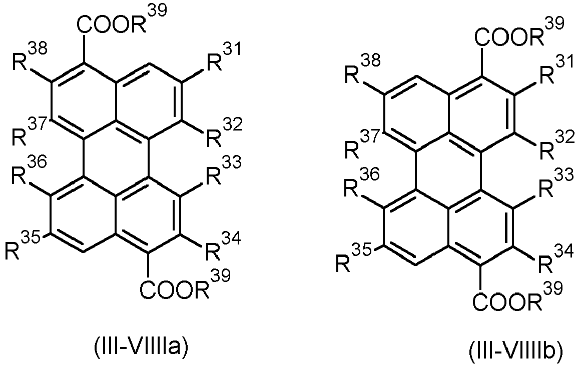 in which
   R³¹, R³², R³³, R³⁴, R³⁵, R³⁶, R³⁷ and R³⁸ are hydrogen; and
   R³⁹ is as defined above
   is halogenated to obtain a reaction mixture which comprises compounds of the formulae (III-IXa) and (III-IXb) 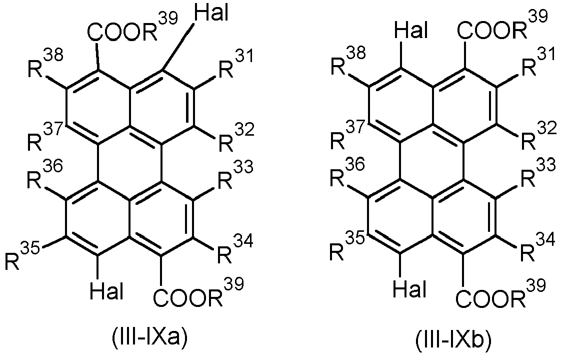 in which
   - R³¹, R³², R³³, R³⁴, R³⁵, R³⁶, R³⁷ and R³⁸: are each hydrogen or halogen selected from chlorine and bromine, where the R³¹, R³², R³³, R³⁴, R³⁵, R³⁶, R³⁷ and R³⁸ substituents that are not hydrogen are either all chlorine or all bromine;
   - Hal: is halogen selected from chlorine and bromine, where the Hal substituents are either all chlorine or all bromine; and
   - R³⁹: is as defined above,
   or mixtures thereof;
g) the compounds of the formulae (III-IXa) and(III-IXb) present in the reaction mixture obtained in step f) are subjected to a substitution of halogen for cyano groups, and optionally partly for hydrogen, to obtain at least one compound of the formula III-B or mixtures thereof; and
h) the at least one compound of the formula III-B or mixtures thereof present in the reaction mixture obtained in step g) is optionally subjected to at least one separation and/or purification step.

### Step f)

In terms of the process, step f) is conducted like step c).

### Step g)

In terms of the process, step g) is conducted like step d).

Compounds of the formula (III) are usually yellow or yellow-green fluorescent dyes.

Compounds of the formula (IV) are subject-matter of unpublished EP 15161081.3. Preferred are compounds of the formula (IV), wherein X⁴⁰ is O. Also preferred are compounds of the formula (IV), wherein X⁴⁰ is S.

Preferred are compounds of the formula (IV), wherein two of the radicals R⁴², R⁴³, R⁴⁴ and R⁴⁵ are cyano and the remaining two radicals R⁴², R⁴³, R⁴⁴ and R⁴⁵ are selected from hydrogen and bromine. Even more preferred are compounds of formula (IV), wherein two of the radicals R⁴², R⁴³, R⁴⁴ and R⁴⁵ are cyano and the remaining two radicals R⁴², R⁴³, R⁴⁴ and R⁴⁵ are each hydrogen. In particular, R⁴² and R⁴⁴ are each cyano and R⁴³ and R⁴⁵ are each hydrogen.

Preferred are compounds of the formula (IV), wherein m4 is 0, i.e R⁴¹ is absent.

Preferred are also compounds of the formula (IV), wherein m4 is 1 or 2. In this context, each R⁴¹ is preferably selected from cyano, bromine, chlorine, C₁-C₄-alkyl such as methyl, ethyl, n-propyl, isopropyl, n-butyl or isobutyl, C₁-C₄-alkoxy such as methoxy, ethoxy, n-propoxy, isopropoxy, n-butoxy or isobutoxy, C₁-C₄-haloalkyl, in particular C₁-C₂-haloalkyl, phenyl and phenyloxy, where the phenyl ring in the two last mentioned radicals is unsubstituted or carries 1, 2 or 3 substituents selected from C₁-C₁₀-alkyl. In particular, R⁴¹ is selected from cyano, bromine, and phenyl which is unsubstituted or carries 1 or 2 radicals selected from C₁-C₄-alkyl; especially R⁴¹ is cyano. Especially, m is 1. More especially, m4 is 1 and R⁴¹ is cyano.

Compounds of the formula (IV) are preferred, wherein group A is a radical of the formula (A.1). Compounds of the formula (IV), where A is a radical of the formula (A.1) are also referred to as compounds of formula (IV-A.1), 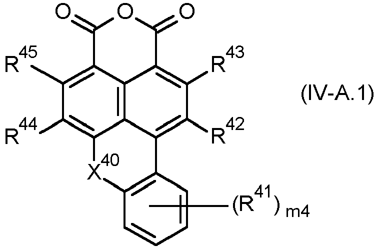 wherein
m4, X⁴⁰, R⁴¹, R⁴², R⁴³, R⁴⁴, and R⁴⁵ are as defined above and especially have one of the preferred meanings.

Examples for preferred inventive compounds of formula (IV-A.1) are shown below:

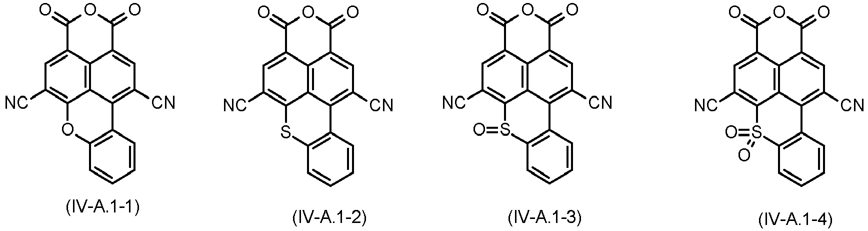

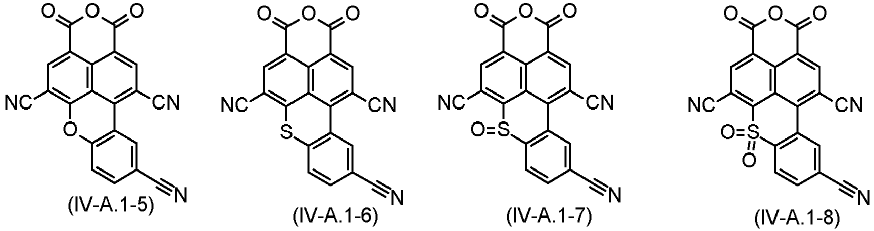

Likewise, compounds of the formula (IV) are preferred, wherein A is a radical of the formula (A.2). Compounds of the formula (IV), where A is a radical of the formula (A.2) are also referred to as compounds of formula (IV-A.2), 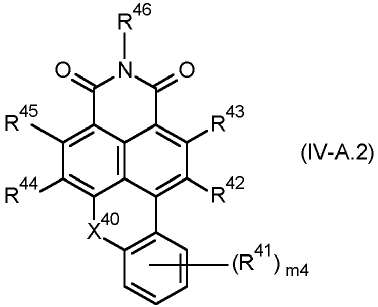 wherein
m4, X⁴⁰, R⁴¹, R⁴², R⁴³, R⁴⁴, R⁴⁵ and R⁴⁶ are as defined above. In particular, m4, X⁴⁰, R⁴¹, R42, R⁴³, R⁴⁴ and R⁴⁵ have one of the preferred meanings mentioned above.

In the compounds of the formula (I-A.2), R⁴⁶ is preferably selected from hydrogen, linear C₁-C₂₄-alkyl, branched C₃-C₂₄-alkyl, C₆-C₁₀-aryl and C₆-C₁₀-aryl-C₁-C₁₀-alkylene, where the aryl ring in the two last mentioned moieties is unsubstituted or substituted with 1, 2, 3, 4 or 5 identical or different radicals R^{46a}. In a special embodiment, R⁴⁶ is selected from linear C₁-C₂₄-alkyl, a radical of the formula (B.1) and a radical of the formula (B.2) 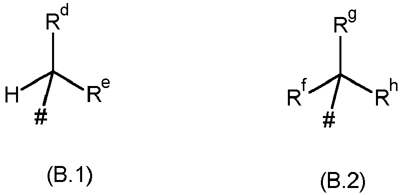 in which
- #: is the bonding site to the nitrogen atom;
- R^{d} and R^{e},: in the formula (B.1), independently from each other are selected from C₁-C₂₃-alkyl, where the sum of the carbon atoms of the R^{d} and R^{e} radicals is an integer from 2 to 23;
- R^{f}, R^{g} and R^{h},: in the formula (B.2) are independently selected from C₁- to C₂₀-alkyl, where the sum of the carbon atoms of the R^{f}, R^{g} and R^{h} radicals is an integer from 3 to 23.

Preferred radicals of the formula (B.1) are: 1-ethylpropyl, 1-methylpropyl, 1-propylbutyl, 1-ethylbutyl, 1-methylbutyl, 1-butylpentyl, 1-propylpentyl, 1-ethylpentyl, 1-methylpentyl, 1-pentylhexyl, 1-butylhexyl, 1-propylhexyl, 1-ethylhexyl, 1-methylhexyl, 1-hexylheptyl, 1-pentylheptyl, 1-butylheptyl, 1-propylheptyl, 1-ethylheptyl, 1-methylheptyl, 1-heptyloctyl, 1-hexyloctyl, 1-pentyloctyl, 1-butyloctyl, 1-propyloctyl, 1-ethyloctyl, 1-methyloctyl, 1-octylnonyl, 1-heptylnonyl, 1-hexylnonyl, 1-pentylnonyl, 1-butylnonyl, 1-propylnonyl, 1-ethylnonyl, 1-methylnonyl, 1-nonyldecyl, 1-octyldecyl, 1-heptyldecyl, 1-hexyldecyl, 1-pentyldecyl, 1-butyldecyl, 1-propyldecyl, 1-ethyldecyl, 1-methyldecyl, 1-decylundecyl, 1-nonylundecyl, 1-octylundecyl, 1-heptylundecyl, 1-hexylundecyl, 1-pentylundecyl, 1-butylundecyl, 1-propylundecyl, 1-ethylundecyl, 1-methylundecyl, 1-undecyldodecyl, 1-decyldodecyl, 1-nonyldodecyl, 1-octyldodecyl, 1-heptyldodecyl, 1-hexyldodecyl, 1-pentyldodecyl, 1-butyldodecyl, 1-propyldodecyl, 1-ethyldodecyl, 1-methyldodecyl, 1-undecyltridecyl, 1-decyltridecyl, 1-nonyltridecyl, 1-octyltridecyl, 1-heptyltridecyl, 1-hexyltridecyl, 1-pentyltridecyl, 1-butyltridecyl, 1-propyltridecyl, 1-ethyltridecyl, 1-methyltridecyl, 1-tridecyltetradecyl, 1-decyltetradecyl, 1-nonyltetradecyl, 1-octyltetradecyl, 1-heptyltetradecyl, 1-hexyltetradecyl, 1-pentyltetradecyl, 1-butyltetradecyl, 1-propyltetradecyl, 1-ethyltetradecyl, 1-methyltetradecyl, 1-octylhexadecyl, 1-heptylhexadecyl, 1-hexylhexadecyl, 1-pentylhexadecyl, 1-butylhexadecyl, 1-propylhexadecyl, 1-ethylhexadecyl, 1-methylhexadecyl, 1-hexyloctadecyl, 1-pentyloctadecyl, 1-butyloctadecyl, 1-propyloctadecyl, 1-ethyloctadecyl, 1-methyloctadecyl, 1-pentadecyleicosanyl, 1-tetradecyleicosanyl, 1-tridecyleicosanyl, 1-dodecyleicosanyl, 1-undecyleicosanyl, 1-butyleicosanyl, 1-propyleicosanyl, 1-ethyleicosanyl, 1-methyleicosanyl.

Particularly preferred radicals of the formula (B.1) are:
1-methylethyl, 1-methylpropyl, 1-methylbutyl, 1-methylpentyl, 1-methylhexyl, 1-methylheptyl, 1-methyloctyl, 1-ethylpropyl, 1-ethylbutyl, 1-ethylpentyl, 1-ethylhexyl, 1-ethylheptyl, 1-ethyloctyl, 1-propylbutyl, 1-propylpentyl, 1-propylhexyl, 1-propylheptyl, 1-propyloctyl, 1-butylpentyl, 1-butylhexyl, 1-butylheptyl, 1-butyloctyl, 1-pentylhexyl, 1-pentylheptyl, 1-pentyloctyl, 1-hexylheptyl, 1-hexyloctyl, 1-heptyloctyl.

A particularly preferred radical of the formula (B.2) is tert.-butyl.

In a further special embodiment, R⁴⁶ is a radical of the formula (C.1), a radical of the formula (C.2) or a radical of the formula (C.3) 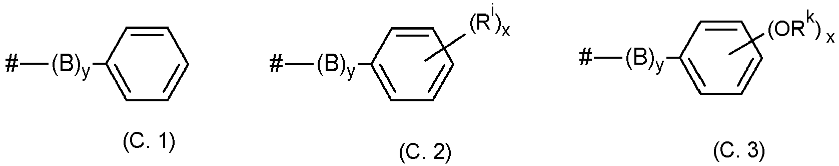 where
- #: represents the bonding side to the nitrogen atom,
- B: where present, is a C₁-C₁₀-alkylene group which may be interrupted by one or more nonadjacent groups selected from -O- and -S-,
- y: is 0 or 1,
- Rⁱ: is independently of one another selected from C₁-C₂₄-alkyl, C₁-C₂₄-fluoroalkyl, fluorine, chlorine or bromine,
- R^{k}: is independently of one another selected from C₁-C₂₄-alkyl,
- x: in formulae C.2 and C.3 is 1, 2, 3, 4 or 5.

Preferably, y is 0, i.e. the variable B is absent.

Irrespectively of its occurrence, Rⁱ is preferably selected from C₁-C₂₄-alkyl, more preferably linear C₁-C₁₀-alkyl or branched C₃-C₁₀-alkyl, especially isopropyl. Irrespectively of its occurrence, R^{k} is preferably selected from C₁-C₃₀-alkyl, more preferably linear C₁-C₁₀-alkyl or branched C₃-C₁₀-alkyl. The variable x in formulae C.2 and C.3 is preferably 1, 2 or 3.

Examples for preferred inventive compounds of formula (IV-A.2) are shown below:

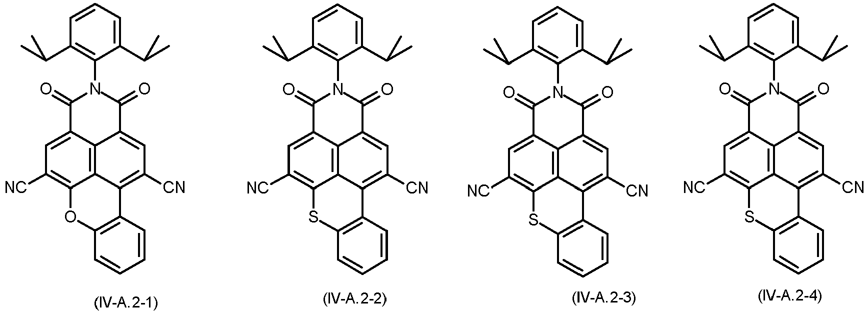

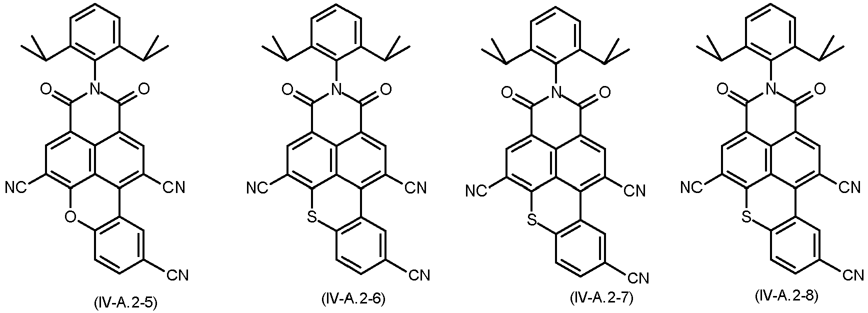

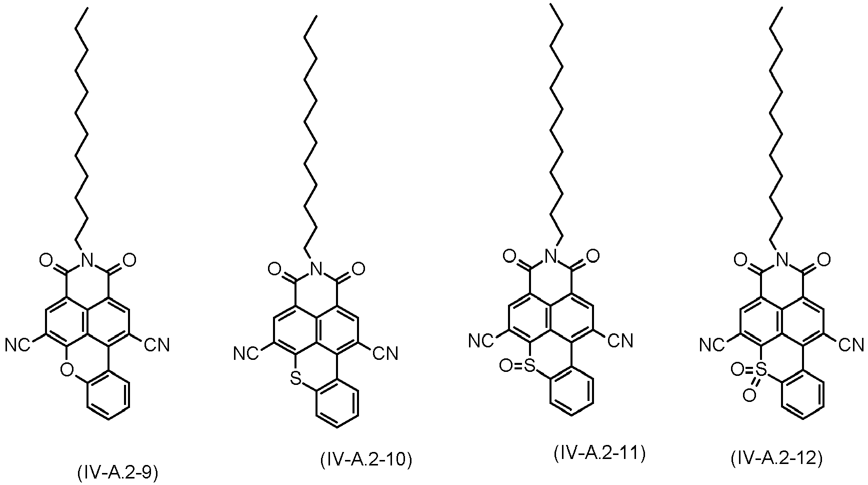

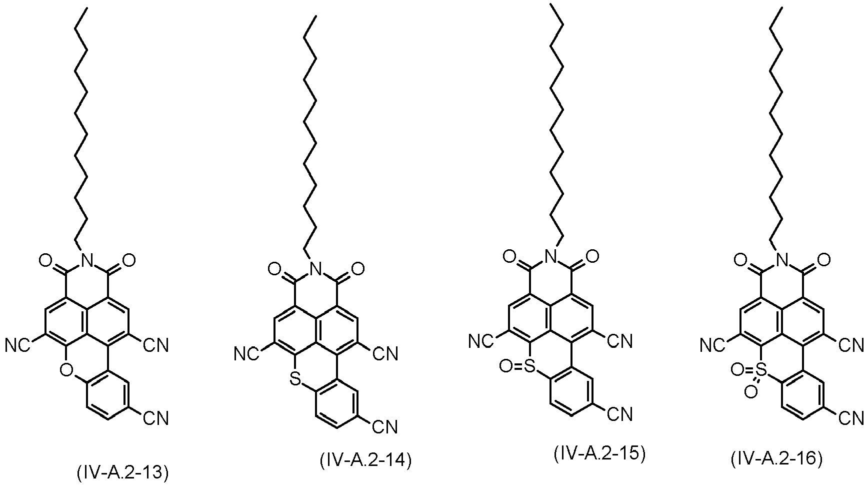

A special group of embodiments relates to compounds of formula (IV-A.2), wherein the variables m4, X⁴⁰, R⁴¹, R⁴², R⁴³ R⁴⁴, and R⁴⁵ independently of each other or in particular in combination, have the following meanings:
- X⁴⁰: is O or S;
- R42 and R⁴⁴: are each cyano;
- R⁴³ and R⁴⁵: are each hydrogen or one of R⁴³ and R⁴⁵ is bromine and the other of R⁴³ and R⁴⁵ is hydrogen;
- R⁴¹: is selected from cyano, bromine, and phenyl which is unsubstituted or carries 1 or 2 radicals selected from C₁-C₄-alkyl;
- R⁴⁶: is selected from hydrogen, C₁-C₂₄-linear alkyl, branched C₃-C₂₄-alkyl, a radical of the formula (C.1), a radical of the formula (C.2) and a radical of the formula (C.3);
- m4: is 0 or 1.

Even more preferably,
- X⁴⁰: is O or S;
- R⁴² and R⁴⁴: are each cyano;
- R⁴³ and R⁴⁵: are each hydrogen;
- R⁴¹: is selected from cyano, bromine, and phenyl which is unsubstituted or carries 1 or 2 radicals selected from C₁-C₄-alkyl; especially cyano;
- R⁴⁶: is selected from linear C₁-C₂₄-alkyl, branched C₃-C₂₄-alkyl, a radical of the formula (C.1), a radical of the formula (C.2) and a radical of the formula (C.3); especially linear C₁-C₂₄-alkyl, branched C₃-C₂₄-alkyl, or phenyl which carries 1 or 2 radicals selected from C₁-C₄-alkyl such as 2,6-diisopropylphenyl ;
- m4: is 0 or 1.

Likewise, compounds of the formula (IV) are preferred, wherein A is a radical of the formula (A.3). This group of embodiments includes the pure regioisomer of the formula (IV-A.3a), the pure regioisomer of the formula (IV-A.3b) and mixtures thereof, 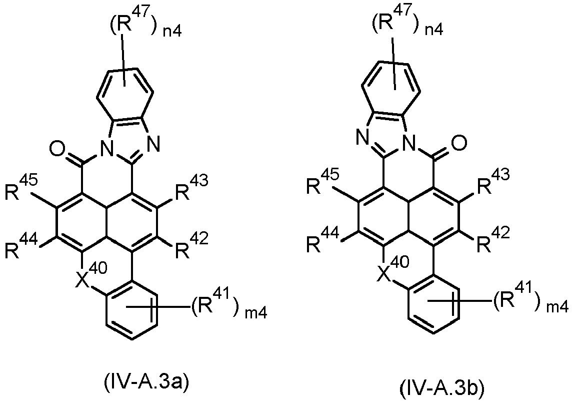 wherein X⁴⁰, R⁴¹ R⁴², Rv³, R⁴⁴, R⁴⁵, R⁴⁷, n4 and m4 are as defined above. In particular, X⁴⁰, R⁴¹ R⁴², R⁴³, R⁴⁴ R⁴⁵, and m4 have one of the preferred meanings mentioned above.

Preferred are compounds of the formulae (IV-A.3a) and (IV-A.3b), wherein n4 is 0, i.e R⁴⁷ is absent. Preferred are also compounds of the formulae (IV-A.3a) and (IV-A.3b), wherein n4 is 1 or 2. In this context, each R⁴⁷ is preferably selected from cyano, bromine, chlorine, C₁-C₄-alkyl such as methyl, ethyl, n-propyl, isopropyl, n-butyl or isobutyl, C₁-C₄-alkoxy such as methoxy, ethoxy, n-propoxy, isopropoxy, n-butoxy or isobutoxy, C₁-C₄-haloalkyl, in particular C₁-C₂-haloalkyl, phenyl and phenyloxy, wherein phenyl in the two last mentioned radicals is unsubstituted or carries 1, 2 or 3 substituents selected from C₁-C₁₀-alkyl. In particular, R⁴⁷ is selected from cyano, bromine and phenyl which is unsubstituted or carries 1 or 2 radicals selected from C₁-C₄-alkyl.

Examples for such preferred compounds are given in tables 1 to 4.

Table 1: Compounds of the formulae (IV-A.3a) or (IV-A.3b) and their mixtures, wherein X⁴⁰ is O, and wherein R⁴², R⁴³, R⁴⁴, R⁴⁵, (R⁴¹)ₘ₄ and (R⁴⁷)ₙ₄ have the meanings given in any of lines 1 to 10 of table A.

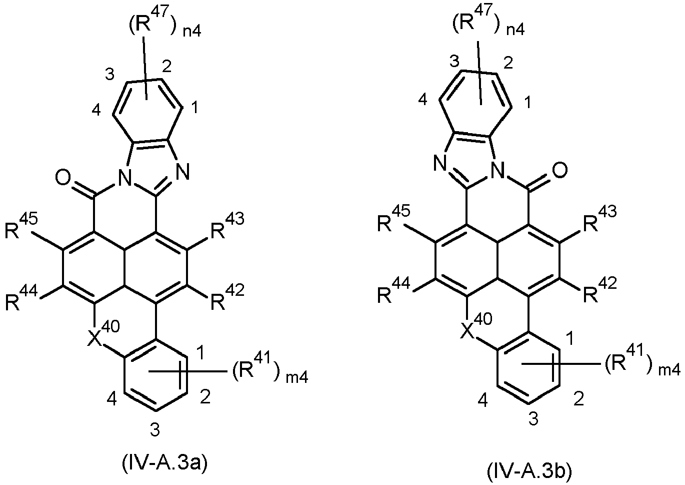

**Table A:**

| | R⁴² | R⁴⁴ | R⁴³ | R⁴⁵ | (R⁴¹)ₘ₄ | (R⁴⁷)ₙ₄ |
|---|---|---|---|---|---|---|
| 1. | CN | CN | H | H | -- | -- |
| 2. | CN | CN | H | H | -- | 1-Ph, 4-Ph |
| 3. | CN | CN | H | H | -- | 1-Ph, 3-Ph |
| 4. | CN | CN | H | H | -- | 2-Ph, 3-Ph |
| 5. | CN | CN | H | H | -- | 2-Ph, 4-Ph |
| 6. | CN | CN | H | H | 2-CN | -- |
| 7. | CN | CN | H | H | 2-CN | 1-Ph, 4-Ph |
| 8. | CN | CN | H | H | 2-CN | 1-Ph, 3-Ph |
| 9. | CN | CN | H | H | 2-CN | 2-Ph, 3-Ph |
| 10. | CN | CN | H | H | 2-CN | 2-Ph, 4-Ph |

In table A the sign "--" in the definition of (R⁴¹)ₘ₄ has the meaning of m4 being 0, i.e. R⁴¹ is absent; the sign "--" in the definition of (R⁴⁷)ₙ₄ has the meaning of n4 being 0, i.e. R⁴⁷ is absent; in case that m4 is different from 0, the number in the definition of (R⁴¹)ₘ₄ indicates the position the radical R⁴¹ is attached to the aromatic ring; in case that n4 is different from 0, the numbers in the definition of (R⁴⁷)ₙ₄ indicate the positions the radicals R⁴⁷ are attached to the benzimidazole ring; Ph is phenyl.

Amongst compounds of the formulae (IV-A.3a) or (IV-A.3b), preference is also given to the compounds defined in the following tables 2, 3 and 4:

### Table 2:

Compounds of the formulae (IV-A.3a) or (IV-A.3b) and their mixtures, wherein X⁴⁰ is S, and wherein R⁴², R⁴³, R⁴⁴, R⁴⁵, (R⁴¹)ₘ₄ and (R⁴⁷)ₙ₄ have the meanings given in any of lines 1 to 10 of table A.

### Table 3:

Compounds of the formulae (IV-A.3a) or (IV-A.3b) and their mixtures, wherein X⁴⁰ is SO, and wherein R⁴², R⁴³, R⁴⁴, R⁴⁵, (R⁴¹)ₘ₄ and (R⁴⁷)ₙ₄ have the meanings given in any of lines 1 to 10 of table A.

### Table 4:

Compounds of the formulae (IV-A.3a) or (IV-A.3b) and their mixtures, wherein X⁴⁰ is SO₂, and wherein R⁴², R⁴³, R⁴⁴, R⁴⁵, (R⁴¹)ₘ₄ and (R⁴⁷)ₙ₄ have the meanings given in any of lines 1 to 10 of table A.

According to a further group of embodiments, compounds of the formula (IV) are preferred, wherein A is a radical of the formula (A.4). This group of embodiments includes the pure regioisomer of the formula (IV-A.4a), the pure regioisomer of the formula (IV-A.4b) and mixtures thereof, 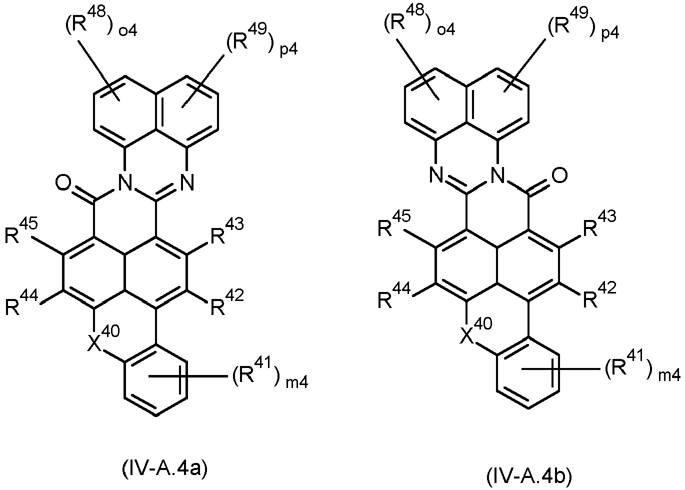 wherein X⁴⁰, R⁴¹ R⁴², R⁴³ R⁴⁴, R⁴⁵, R⁴⁸, R⁴⁹, o4, p4 and m4 are as defined above. In particular, X⁴⁰, R⁴¹, R⁴², R⁴³ R⁴⁴, R⁴⁵, and m4 have one of the preferred meanings mentioned above.

Preferred are compounds of the formulae (IV-A.4a) or (IV-A.4b) and their mixtures, wherein o4 and p4 are 0, i.e R⁴⁸ and R⁴⁹ are absent. Preferred are also compounds of the formulae (IV-A.4a) and (IV-A.4b), wherein the sum of o4 and p4 is 1, 2, 3 or 4. In this context, R⁴⁸ and R⁴⁹ are, independently of each other, preferably selected from cyano, bromine, chlorine, C₁-C₄-alkyl such as methyl, ethyl, n-propyl, isopropyl, n-butyl or isobutyl, C₁-C₄-alkoxy such as methoxy, ethoxy, n-propoxy, isopropoxy, n-butoxy or isobutoxy, C₁-C₄-haloalkyl, in particular C₁-C₂-haloalkyl, phenyl and phenyloxy, wherein phenyl in the two last mentioned radicals is unsubstituted or carries 1, 2 or 3 substituents selected from C₁-C₁₀-alkyl. In particular, R⁴⁸ and R⁴⁹ are, independently of each other, selected from cyano, bromine and phenyl which is unsubstituted or carries 1 or 2 radicals selected from C₁-C₄-alkyl.

Examples for preferred inventive compounds of formulae (IV-A.4a) and (IV-A.4b) are shown below:

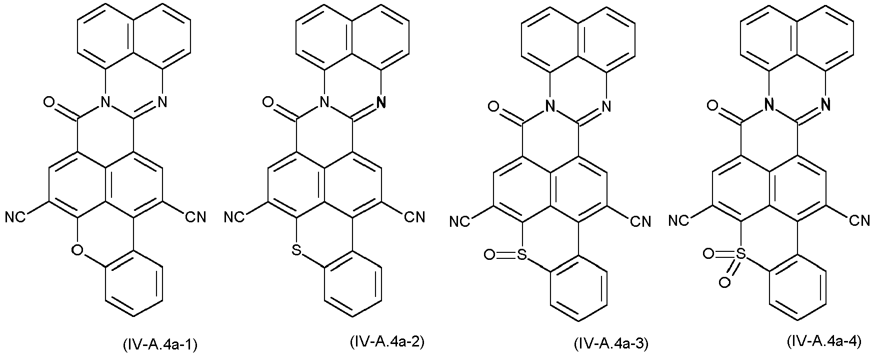

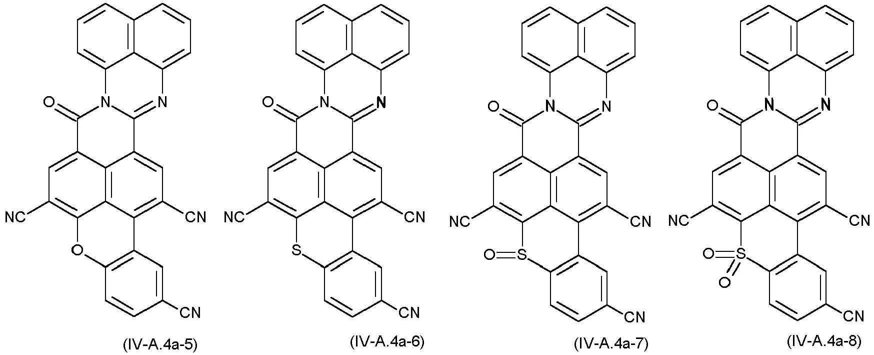

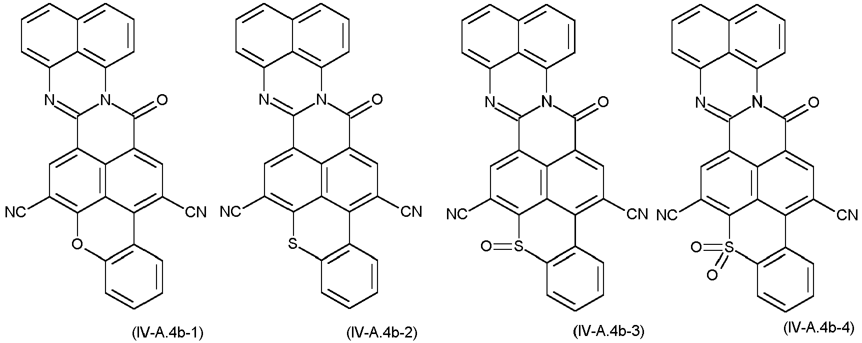

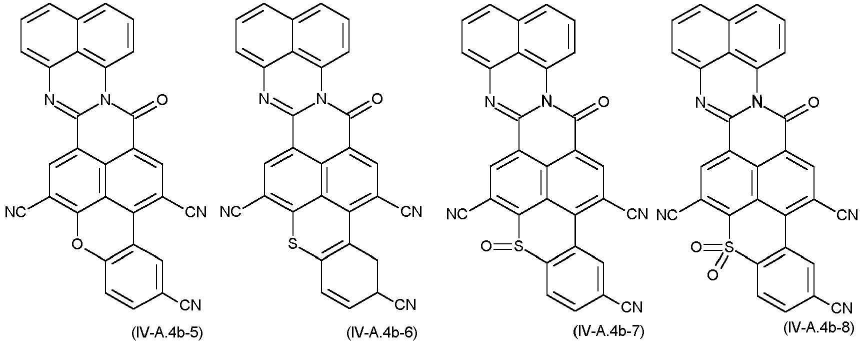

Compounds of the formula (IV) used according to the present invention can be prepared e.g. according to the preparation methods as described below by treating a compound of the formula (IV-II) 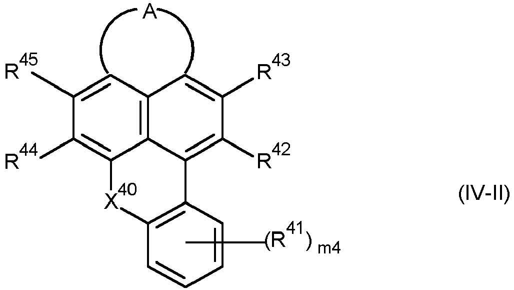 wherein
at least one of the radicals R⁴², R⁴³, R⁴⁴ and R⁴⁵ is selected from bromine and chlorine and the remaining radicals R⁴², R⁴³, R⁴⁴ and R⁴⁵ are hydrogen;
X⁴⁰ is O, S, SO or SO₂;
A is a radical of the general formulae (A.1), (A.2), (A.3), or (A.4), wherein (A.1), (A.2), (A.3), and (A.4) are as defined above;
R⁴¹ is bromine, chlorine, -NR^{4a}R^{4b}, C₁-C₂₄-alkyl, C₁-C₂₄-haloalkyl, C₁-C₂₄-alkoxy, C₁-C₂₄-haloalkoxy, C₃-C₂₄-cycloalkyl, heterocycloalkyl, heteroaryl, C₆-C₂₄-aryl, C₆-C₂₄-aryloxy, C₆-C₂₄-aryl-C₁-C₁₀-alkylene, where the rings of cycloalkyl, heterocycloalkyl, heteroaryl, aryl, aryloxy and-aryl-alkylene in the six last-mentioned radicals are unsubstituted or substituted with 1, 2, 3, 4 or 5 identical or different radicals R^{41a} and where C₁-C₂₄-alkyl, C₁-C₂₄-haloalkyl, C₁-C₂₄-alkoxy, and the alkylene moiety of C₆-C₂₄-aryl-C₁-C₁₀-alkylene may be interrupted by one or more groups selected from O, S and NR^{4c}, wherein R^{4a}, R^{4b} and R^{4c} are as defined above; and
m is 0, 1, 2, 3 or 4,
with a cyanating agent. A suitable cyanating agent is for example copper(I) cyanide.

Suitable process conditions for the exchange of bromine or chlorine with cyano are described e.g. in J. March, Advanced Organic Chemistry, 4th edition, John Wiley & Sons Publishers (1992), p. 660-661, in WO 2004/029028 and WO 2015/019270.

Compounds of the formula (IV-II) can be prepared by treating a compound of the formula (IV-III) 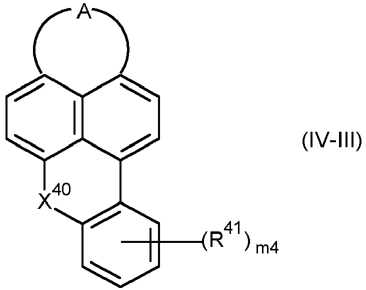 wherein X⁴⁰, A, R⁴¹ and m4 are as defined above
with a halogenating agent selected from brominating agents and chlorinating agents.

Bromination is typically carried out with elemental bromine in a solvent as described e.g. in WO 2014/131628. Further suitable brominating agents are N-bromosuccinimide and dibromoisocyanuric acid. Suitable solvents are water or aliphatic monocarboxylic acids, and chlorinated hydrocarbons such as chlorobenzene and chloroform. Suitable aliphatic monocarboxylic acids are those having 2 to 6 carbon atoms, such as acetic acid, propionic acid, butyric acid, pentanecarboxylic acid and hexanecarboxylic acid, and mixtures thereof. When an aliphatic monocarboxylic acid is used as a solvent, it may be advantageous to use iodine as a catalyst.

Chlorination is typically carried out with elemental chlorine, N-chlorosuccinimide, chlorosulfonic acid, sulfuryl chloride in an inert solvent as described e.g. in US 2011/0068328. A further suitable chlorinating agent is N-chlorosuccinimide.

Depending on the molar ratio of the halogenating agent to compound of the formula (IV-III), a mono-, di- or multihalogene-substituted compound of formula (IV-II), i.e. a mono-, di-, or multibromo-substituted compound of formula (IV-II) and mono-, di- or multichlorine-substituted compound of formula (IV-II), respectively, is obtained which can be separated by column chromatography (SiO₂).

Benzoxanthene compounds or benzothioxanthene compounds of formula (IV), where A is a radical of formulae (A.1) or (A.2) are known in the art and for example described in US 3,748,330, US 3,812,051, GB 1 440 450 or WO 2014/131628. Benzoxanthene compounds or benzothioxanthene compounds of formula (IV), where A is a radical of formulae (A.3) or (A.4) can be prepared in analogy to the method described in WO 2015/019270.

Compounds of the formula (IV), wherein X is SO or SO₂, can be obtained by oxidizing compounds of the formula (IV), wherein X is S. Suitable oxidizing agents are meta-chloroperbenzoic acid, hypochlorite or hydrogen peroxide.

Benzoxanthene compounds of the formula (V) are known from WO 2014/131628. They are usually yellow or yellow-green fluorescent dyes.

Compounds of the formula (VI) are known from WO 2012/168395. Particular preference is given to the compounds specified in WO 2012/168395, at page 28, line14 to page 32, line 5.

Especially preferably, organic fluorescent dyes of formula VI are selected from compounds of formulae (VI-1), (VI-2), (VI-3), (VI-4), (VI-5), (VI-6), (VI-7), (VI-8), (VI-9), (VI-10), (VI-11), (VI-12), (VI-13), (VI-14), (VI-15), (VI-16), (VI-17), (VI-18), (VI-19), (VI-20), (VI-21), (VI-22), (VI-23), (VI-24), (VI-25), (VI-26), (VI-27), (VI-28), (VI-29), (VI-30), (VI-31), (VI-32), (VI-33), (VI-34), (VI-35), (VI-36), (VI-37), (VI-38), (VI-39), (VI-40), (VI-41), (VI-42), (VI-43), (VI-44), (VI-45), (VI-46), (VI-47), (VI-48), (VI-49), (VI-50), (VI-51), (VI-52), (VI-53), (VI-54), (VI-55), and mixtures thereof 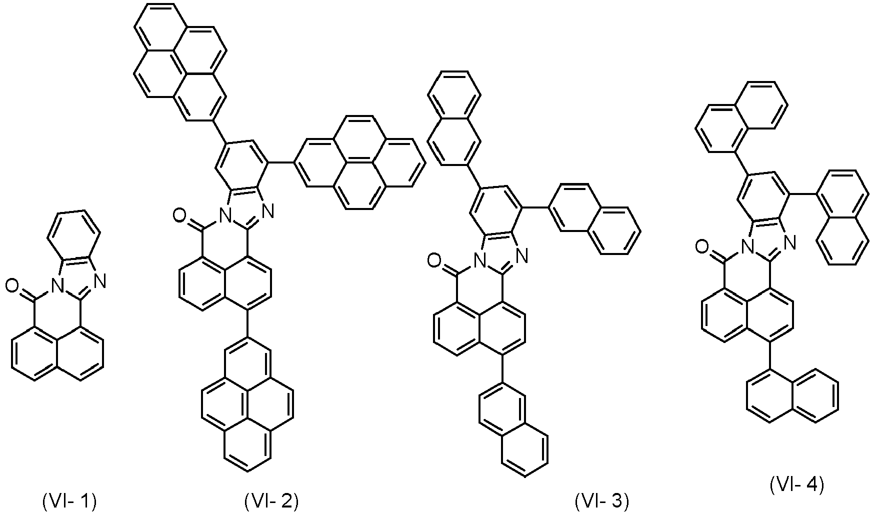 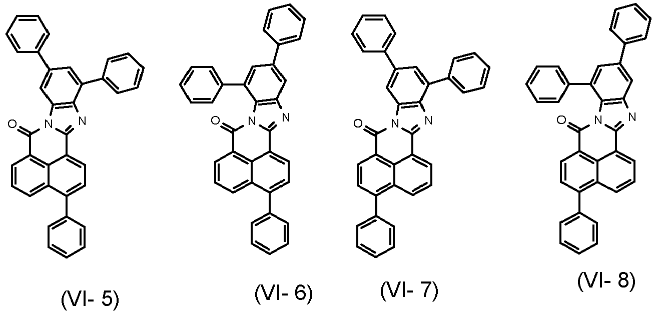 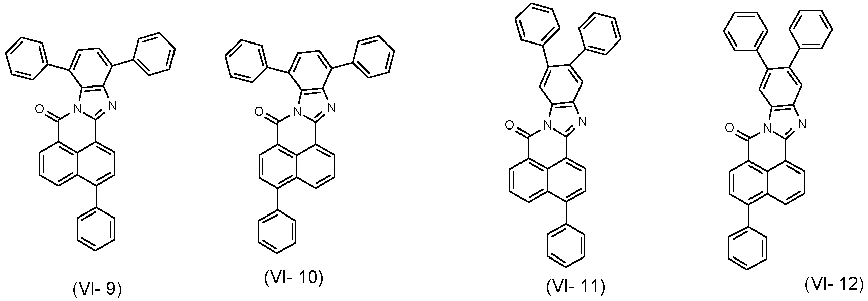 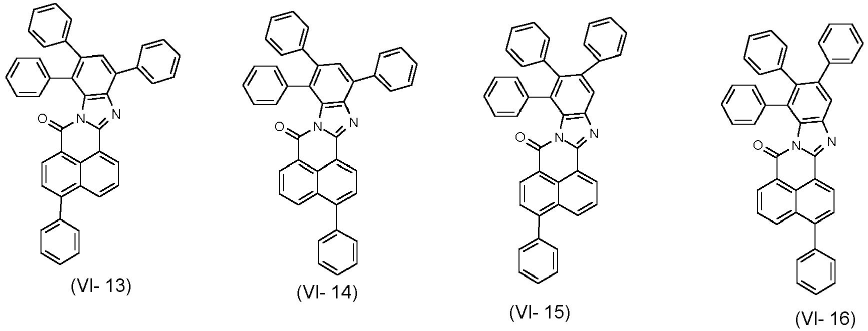 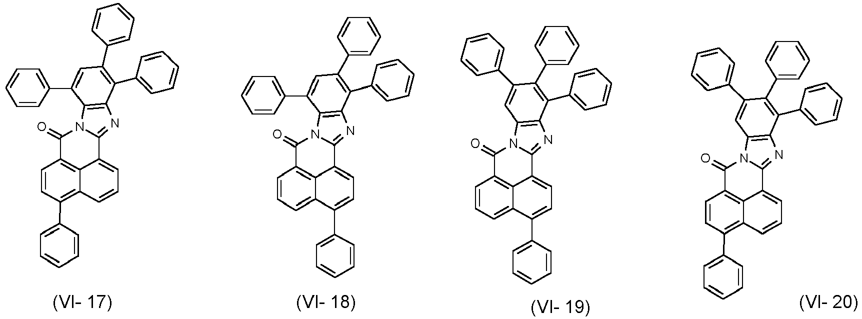 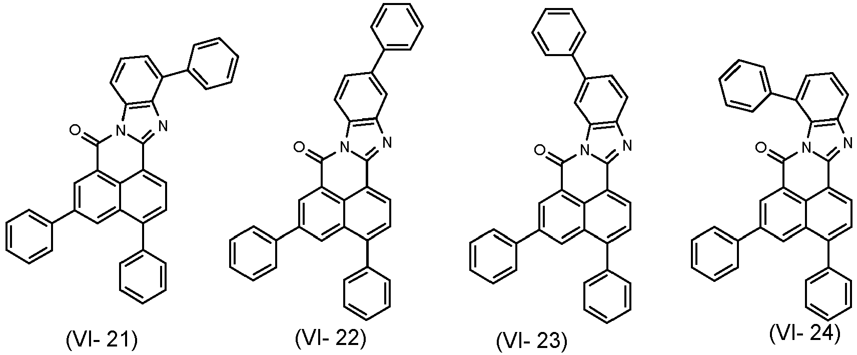 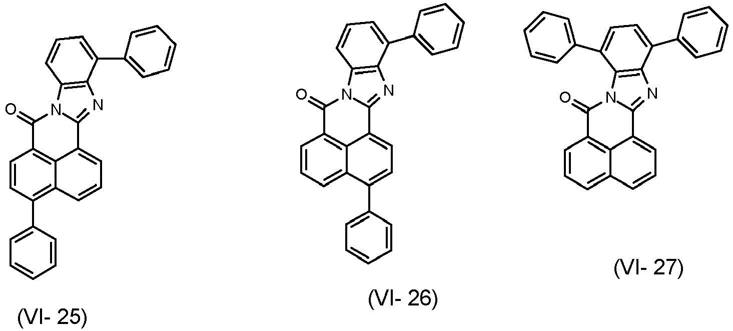 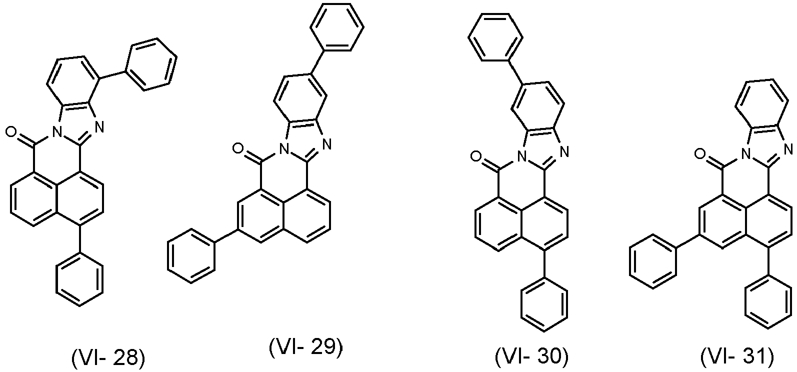 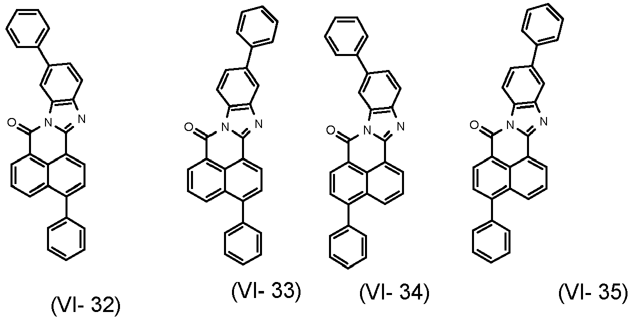 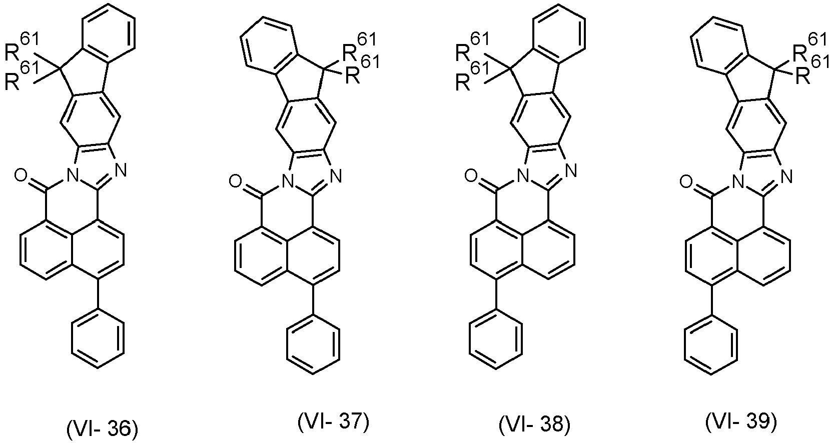 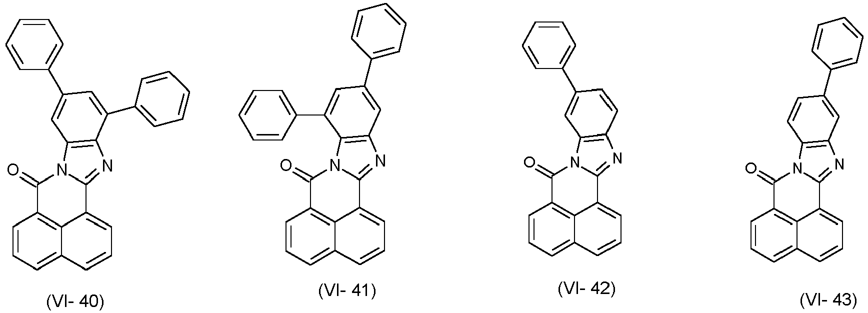 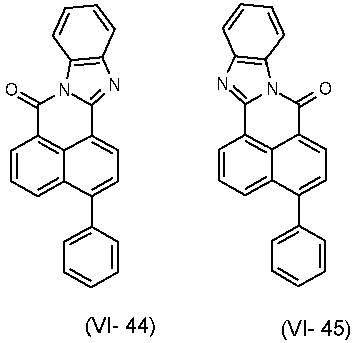 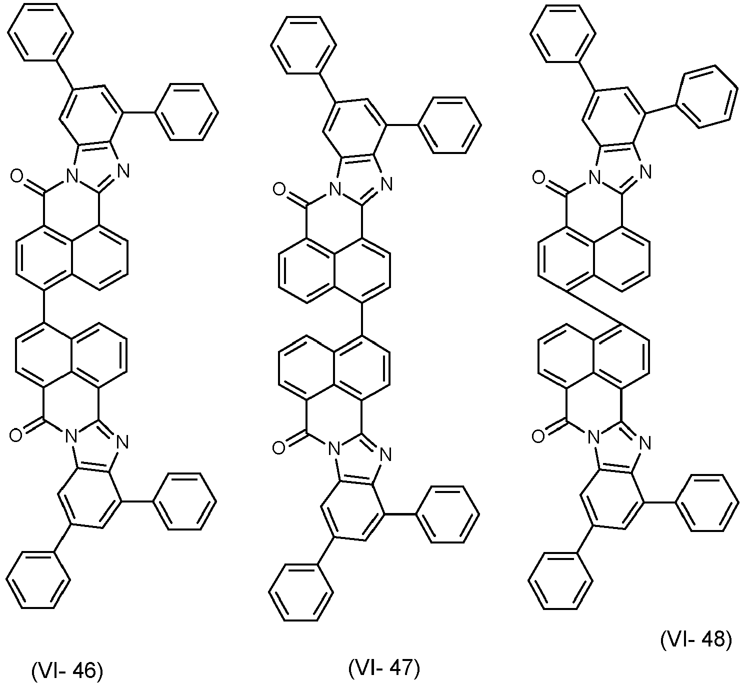 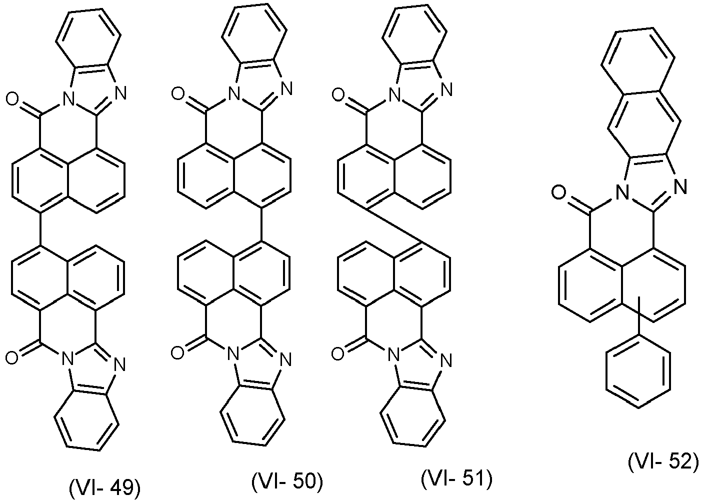 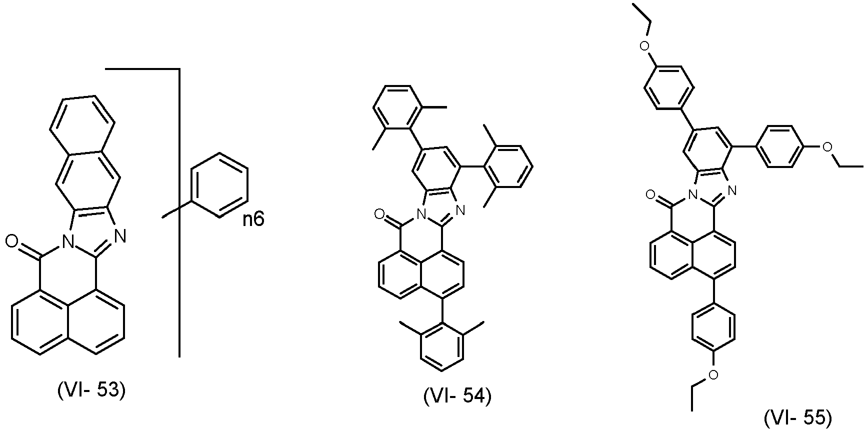 or mixtures thereof,
where n6 is a number from 0, 1, 2, 3, 4, 5, 6, 7, 8, 9 or 10;
R⁶¹ is independently hydrogen, C₁-C₁₈-alkyl or cycloalkyl, the carbon chain of which may comprise one or more -O-, -S-, -CO-, -SO- and/or -SO₂- moieties and which may be mono- or polysubstituted;
   aryl or heteroaryl which may be mono- or polysubstituted.

Compounds of formula (VI) are especially preferred for use in combination with the inventive red-fluorescent dye. Especially preferred are the compounds of formulae (VI-5), (VI-6), (VI-7) and (VI-8) and mixtures thereof.

Compounds of the formula (VI) are usually yellow or yellow-green fluorescent dyes.

Likewise, preferred are compounds of the formula (VII), wherein R⁷¹ and R⁷² are each independently selected from C₁-C₁₀-alkyl, 2,6-di(C₁-C₁₀-alkyl)aryl and 2,4-di(C₁-C₁₀-alkyl)aryl. More preferably, R⁷¹ and R⁷² are identical. Very particularly, R⁷¹ and R⁷² are each 2,6-diisopropylphenyl or 2,4-di-*tert*-butylphenyl.

R⁷³ is preferably phenoxy, or (C₁-C₁₀-alkyl)phenoxy, more preferably 2,6-(dialkyl)phenoxy or 2,4-(dialkyl)phenoxy. Especially preferably R⁷³ is phenoxy, 2,6-diisopropylphenoxy, 2,4-di-*tert-*butylphenoxy or 4-tert-octylphenoxy.

More particularly, suitable further organic fluorescent dyes are selected from the compounds of the formulae VII-1, VII-2 and VII-3 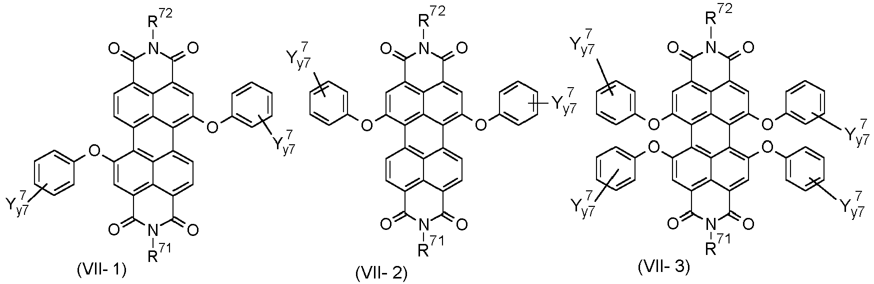 in which
- R⁷¹ and R⁷²: are each as defined above and especially have one of the preferred meanings;
- Y⁷: is linear or branched C₁-C₁₀-alkyl; and
- y7: is 0, 1, 2, or 3.

Further examples of particularly suitable further organic fluorescent dyes are the perylene derivatives specified in WO 2007/006717 at page 1 line 5 to page 22 line 6. Fluorescent dyes of formula (VII) are usually red-fluorescent dyes.

Particularly suitable further organic fluorescent dyes of formula (VII) are N,N'-bis(2,6-diisopropylphenyl)-1,6,7,12-tetraphenoxyperylene-3,4;9,10-tetracarboximide, N,N'-bis(2,6-diisopropylphenyl)-1,7-di(2,6-diisopropylphenoxy)perylene-3,4;9,10-tetracarboximide, N,N'-bis(2,6-diisopropylphenyl)-1,6-di(2,6-diisopropylphenoxy)-perylene-3,4;9,10-tetracarboximide, N,N'-bis(2,6-diisopropylphenyl)-1,7-di(p-tert-octylphenoxy)perylene-3,4;9,10-tetracarboximide, N,N'-bis(2,6-diisopropylphenyl)-1,6-di(p-tert-octylphenoxy)perylene-3,4;9,10-tetracarboximide, N,N'-bis(2,6-diisopropylphenyl)-1,7-diphenoxyperylene-3,4;9,10-tetracarboximide, N,N'-bis(2,6-diisopropylphenyl)-1,6-diphenoxyperylene-3,4;9,10-tetracarboximide, and mixtures thereof.

In a further embodiment, inventive color converters additionally comprise at least one further organic fluorescent dye of the formula (VIII). Fluorescent dyes of the formula (VIII) are usually orange fluorescent dyes. Preferred are compounds of formula (VIII) 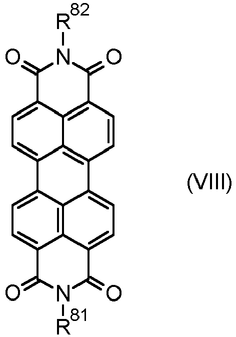 where
R⁸¹ and R⁸² are each independently selected from C₁-C₁₀-alkyl, 2,6-di(C₁-C₁₀-alkyl)aryl and 2,4-di(C₁-C₁₀-alkyl)aryl. More preferably, R⁸¹ and R²² are identical. Very particularly, R⁸¹ and R⁸² are each 2,6-diisopropylphenyl or 2,4-di-*tert*-butylphenyl.

Preferred are also compounds of formula (IX) 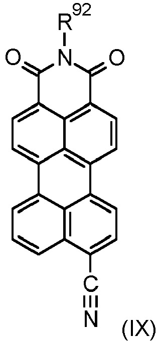 wherein
- R⁹²: is C₁-C₁₀-alkyl, 2,6-di(C₁-C₁₀-alkyl)aryl or 2,4-di(C₁-C₁₀-alkyl)aryl, in particular R⁹² is 2,6-diisopropylphenyl or 2,4-di-*tert*-butylphenyl.

Compounds of the formula (IX) are usually yellow-orange fluorescent dyes.

The number of organic fluorescent dyes to be used in combination with the inventive red-fluorescent dye can be any number, preferably 1, 2, 3 or 4, more preferably 1 or 2.

In a preferred embodiment, inventive color converters comprise, as well as the at least one organic fluorescent dye present in accordance with the invention, at least one further organic fluorescent dye selected from compounds of formulae (II), (IV), (VI) and (VII). In particular, the at least one further organic fluorescent dye is a compound of formula (VI), especially one of the compounds of formulae (VI-1), (VI-2), (VI-3), (VI-4), (VI-5), (VI-6), (VI-7), (VI-8), (VI-9), (VI-10), (VI-11), (VI-12), (VI-13), (VI-14), (VI-15), (VI-16), (VI-17), (VI-18), (VI-19), (VI-20), (VI-21), (VI-22), (VI-23), (VI-24), (VI-25), (VI-26), (VI-27), (VI-28), (VI-29), (VI-30), (VI-31), (VI-32), (VI-33), (VI-34), (VI-35), (VI-36), (VI-37), (VI-38), (VI-39), (VI-40), (VI-41), (VI-42), (VI-43), (VI-44), (VI-45), (VI-46), (VI-47), (VI-48), (VI-49), (VI-50), (VI-51), (VI-52), (VI-53), (VI-54), (VI-55), and mixtures thereof. Among these, especially preferred are the compounds of formulae (VI-5), (VI-6), (VI-7), (VI-8) and mixtures thereof.

The red-fluorescent dye according to the invention used in combination with at least one further organic fluorescent dye, especially a yellow or green-yellow fluorescent dye, leads to excellent properties in terms of energetic and photometric conversion efficacy.

The concentration of the organic fluorescent dyes in the polymer is set as a function of the thickness of the color converter and the type of polymer. If a thin polymer layer is used, the concentration of the organic fluorescent dye(s) is generally higher than in the case of a thick polymer layer.

Typically, the concentration of inventive organic fluorescent dye of the formula la or Ib or mixtures thereof is 0.001 to 0.5% by weight, preferably 0.002 to 0.3% by weight, most preferably 0.003 to 0.1 % by weight, based in each case on the amount of polymer used. Typically, the concentration of the at least one further organic fluorescent dye is 0.001 to 0.5% by weight, preferably 0.002 to 0.3% by weight, based on the amount of the polymer used.

The ratio of at least one further organic fluorescent dye to the inventive red organic fluorescent dye is typically in the range from 4:1 to 25:1, preferably 5:1 to 20:1.

In a further preferred embodiment, the color converter according to the present invention may comprise inorganic fluorescent materials. According to this embodiment, the color converter is preferably used for conversion of light which has been produced by a blue diode, using at least one compound of the formulae la or Ib or a mixture thereof as a fluorescent dye in combination with at least one inorganic fluorescent colorant. In this embodiment, the blue LED and the color converter are in a remote phosphor arrangement.

The at least one inorganic fluorescent material is preferably selected from garnets, silicates, sulfides, nitrides and oxynitrides.

Particularly preferred among these are those selected from garnets, silicates, sulfides, nitrides and oxynitrides. Suitable examples of garnets, silicates, sulfides, nitrides and oxynitrides are compiled in table I below:

**Table I:**

| Class | Compounds | Excitation Peak nm | Emission Peak nm | Reference |
|---|---|---|---|---|
| Garnets | • YAG:Ce | 460-470 | 550 | US 5,998,925 |
| | • (Y₃Al₅O₁₂:Ce) | | | |
| | • (Y, Gd, Tb,Lu)₃Al₅O₁₂:Ce | | | |
| | • TAG:Ce (Tb₃Al₅O₁₂:Ce) | 460-470 | 575 | US 6,669,866, US 6,812,500, US 6,576,930, US 6,060,861, US 6,245,259, US 6,765,237 |
| Silicates | • Eu-doped Silicates | <460 | 510 to 610 | US 7,311,858, US 7,267,787 |
| | - A₂Si(OD)₄:Eu wit A = Sr, Ba, Ca, Mg, Zn and D = F, Cl, S, N, Br | | | |
| | - (SrBaCa)₂SiO₄:Eu | | | US 6,809,347, US 6,943,380 |
| | - Sr₃SiO₅ | | | |
| | - Ba₂MgSi₂O₇:Eu²⁺; | | | US 6,429,583, WO 02/11214 |
| | - Ba₂SiO₄:Eu²⁺ | | | |
| | - (Ca,Ce)₃(Sc,Mg)₂Si₃O₁₂ | | | |
| Sulfides | • (Ca, Sr)S:Eu | <460 | 615-660 | |
| Nitrides | • (CaAlSiN₃:Eu²) | 455 | red | |
| | • (Sr,Ca)AlSiN₃:Eu²⁺ | | orange | WO2005052087 |
| Oxynitrides | • SiAlON:Ce | 300-580 | 490 | |
| | • β-SiAlON:Eu | | 540 | |
| | • Ca-alpha-SiAlON:Eu (Ba₃Si₆O₁₂N₂:Eu) | | 585-595 | |
| | General formula | | | |
| | Ca_{X}Eu_{y}(Si,Al)₁₂(O,N)₁₆ | | | |

According to a further embodiment, inventive color converters comprise, as well as the at least one organic fluorescent dye present in accordance with the invention, at least one further organic fluorescent dye selected from compounds of formulae (II), (III), (IV), (V), (VI), (VII), (VIII), (IX) and mixtures thereof and at least one inorganic fluorescent material as defined above.

According to a further preferred embodiment, the inventive color converter comprises at least one quantum dot. Quantum dots are a nanocrystal of a semiconductor material having a diameter of about 20 nm or less. The quantum dot may include one of a Si-based nanocrystal, a group II-VI compound semiconductor nanocrystal, a group III-V compound semiconductor nanocrystal, a group IV-VI compound nanocrystal and a mixture thereof. The group II-VI compound semiconductor nanocrystal may include one selected from a group consisting of CdS, CdSe, CdTe, ZnS, ZnSe, ZnTe, HgS, HgSe, HgTe, CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HggZnTe, CdZnSeS, CdZnSeTe, CdZnSTe, CdHgSeS, CdHgSeTe, CdHgSTe, HgZnSeS, HgZnSeTe and HgZnSTe. The group III-V compound semiconductor nanocrystal may include one selected from a group consisting of GaN, GaP, GaAs, AIN, AIP, AlAs, InN, InP, InAs, GaNP, GaNAs, GaPAs, AINP, AINAs, AIPAs, InNP, InNAs, InPAs, GaAINP, GaAINAs, GaAIPAs, GalnNP, GalnNAs, GalnPAs, InAINP, InAINAs, and InAIPAs. The IV-VI compound semiconductor nano crystal may be SbTe.

To synthesize a nanocrystal in form of a quantum dot, quantum dots may be prepared by vapor deposition such as metal organic chemical vapor deposition or molecular beam epitaxy, or by a wet chemical process in which a crystal is grown by adding one or more precursors into an organic solvent.

According to a further embodiment, inventive color converters comprise, as well as the at least one organic fluorescent dye present in accordance with the invention, at least one further organic fluorescent dye selected from compounds of formulae (II), (III), (IV), (V), (VI), (VII), (VIII), (IX) and mixtures thereof and at least one quantum dot as defined above.

In a more preferred embodiment of the invention, the inventive color converter does not comprise quantum dots. Likewise, in a more preferred embodiment of the invention, the inventive color converter does not comprise inorganic fluorescent materials.

In one embodiment of the invention, inventive color converters have a laminate structure. They may either have a monolayer structure or a multilayer structure, generally composed of a plurality of polymer layers comprising one or more fluorescent dyes and/or scattering bodies. If the color converter has a multilayer structure, one layer comprises the red fluorescent dye according to the invention and another layer comprises at least one fluorescent dye encompassed by the present invention.

In one embodiment, the at least one red organic fluorescent dye is present in the layer of the color converter facing the LED. In another embodiment, the at least one further fluorescent dye is present in the layer of the color converter facing the LED. According to a preferred embodiment, the color converter additionally comprises at least one inorganic white pigment as a scattering body.

In a preferred embodiment, at least one of the layers or matrices comprising organic fluorescent dye comprises scattering bodies for light.

Suitable scattering bodies are inorganic white pigments, for example titanium dioxide, barium sulphate, lithopone, zinc oxide, zinc sulphide, calcium carbonate with a mean particle size to DIN 13320 of 0.01 to 10 µm, preferably 0.1 to 1 µm, more preferably 0.15 to 0.4 µm, especially scattering bodies based on TiO₂.

Scattering bodies are included typically in an amount of 0.01 to 2.0% by weight, preferably 0.05 to 1 % by weight, more preferably 0.1 to 0.5% by weight, based in each case on the polymer of the layer comprising scattering bodies.

In a preferred embodiment, the color converter has a two-layer structure with a red-fluorescing layer and a green-yellow-fluorescing layer comprising at least one fluorescent dye present in accordance with the invention, with the red layer facing the blue light source. In this embodiment, both layers comprise TiO₂ as a scattering body.

In one embodiment, the color converters consist of a plurality of polymer layers which have been laminated together to form a composite and wherein the various fluorescent dyes/colorants and/or scattering bodies may be present in different polymer layers.

If inventive color converters comprise more than one fluorescent dyes/colorant, it is possible in one embodiment of the invention for a plurality of fluorescent dyes/colorants to be present alongside one another in one layer.

In another embodiment, the various fluorescent dyes/colorants are present in various layers.

In a preferred embodiment, inventive color converters comprise, as well as the at least one organic fluorescent dye present in accordance with the invention, at least one further organic fluorescent dye selected from compounds of formulae (II), (IV), (VI) and (VII), scattering bodies based on TiO₂ and at least one polymer consisting essentially of polystyrene, polyethylene terephthalate or polycarbonate.

In an even more preferred embodiment, inventive color converters comprise, as well as the at least one organic fluorescent dye present in accordance with the invention, at least one further organic fluorescent dye selected from compounds of formulae (II), (IV), (VI) and (VII), scattering bodies based on TiO₂ and at least one polymer consisting essentially of polyethylene terephthalate.

Likewise, in an even more preferred embodiment, inventive color converters comprise, as well as the at least one organic fluorescent dye present in accordance with the invention, at least one further organic fluorescent dye selected from compounds of formulae (II), (IV), (VI) and (VII), scattering bodies based on TiO₂ and at least one polymer consisting essentially of polycarbonate.

In a particularly preferred embodiment, inventive color converters comprise, as well as the at least one organic fluorescent dye present in accordance with the invention, at least one further organic fluorescent dye of formula (VI), scattering bodies based on TiO₂ and at least one polymer consisting essentially of polystyrene, polyethylene terephthalate or polycarbonate.

In an even more particularly preferred embodiment, inventive color converters comprise, as well as the at least one organic fluorescent dye present in accordance with the invention, at least one further organic fluorescent dye of formula (VI), scattering bodies based on TiO₂ and at least one polymer consisting essentially of polyethylene terephthalate.

Likewise, in a particularly preferred embodiment, inventive color converters comprise, as well as the at least one organic fluorescent dye present in accordance with the invention, at least one further organic fluorescent dye of formula (VI), scattering bodies based on TiO₂ and at least one polymer consisting essentially polycarbonate.

In one embodiment, at least one polymer layer of the color converter has been mechanically reinforced with glass fibers.

Inventive color converters may be in any desired geometric arrangement. The color converters may, for example, be in the form of films, sheets or plaques. Equally, the matrix containing organic fluorescent dyes may be in droplet form or hemispherical form or in the form of lenses with convex and/or concave, flat or spherical surfaces.

"Casting" refers to the embodiment where LEDs or components comprising LEDs are cast or enveloped fully with a polymer comprising organic fluorescent dye.

In one embodiment of the invention, the polymer layers (matrices) comprising organic fluorescent dye are 25 to 250 micrometers thick, preferably 35 to 200 µm and particularly 50 to 180 µm.

In another embodiment, the polymer layers comprising organic fluorescent dyes are 0.2 to 5 millimeters thick, preferably 0.3 to 3 mm and more preferably 0.4 to 1 mm.

If the color converters consist of one layer or they have a laminate structure, the individual layers, in a preferred embodiment, are continuous and do not have any holes or interruptions.

Inventive color converters may optionally comprise further constituents such as a backing layer.

Backing layers serve to impart mechanical stability to the color converter. The type of material for the backing layers is not crucial, provided that it is transparent and has the desired mechanical strength. Suitable materials for backing layers are, for example, glass or transparent rigid organic polymers such as polycarbonate, polystyrene or polymethacrylates or polymethylmethacrylates.

Backing layers generally have a thickness of 0.1 mm to 10 mm, preferably 0.2 mm to 5 mm, more preferably 0.3 mm to 2 mm.

In one embodiment of the invention, inventive color converters have at least one barrier layer against oxygen and/or water, as disclosed in WO 2012/152812. Examples of suitable barrier materials for barrier layers are, for example, glass, quartz, metal oxides, SiO₂, a multilayer system composed of alternating layers of Al₂O₃ and SiO₂ layers, titanium nitride, SiO₂/metal oxide multilayer materials, polyvinyl alcohol, polyacrylonitrile, polyvinylidene chloride (PVDC), liquid crystal polymers (LCP), polystyrene-acrylonitrile (SAN), polybutylene terephthalate (PBT), polybutylene naphthalate (PBN), polyethylene terephthalate (PET), polyethylene naphthalate (PEN), polyvinyl butyrate (PBT), polyvinyl chloride (PVC), polyamides, polyoxymethylenes, polyimides, polyetherimides, epoxy resins, polymers which derive from ethylene-vinyl acetate (EVA) and polymers which derive from ethylene-vinyl alcohol (EVOH).

A preferred material for barrier layers is glass or a multilayer system composed of alternating layers of Al₂O₃ and SiO₂ layers.

Preferably, suitable barrier layers have low permeability for oxygen.

More preferably, suitable barrier layers have low permeability for oxygen and water.

Inventive color converters are especially suitable for the conversion of blue light to white light.

More particularly, they are suitable for conversion of light emitted by blue LEDs. Suitable LEDs are, for example, those based on gallium nitride (GaN) or indium gallium nitride (InGaN). Likewise possible is use for conversion of light produced by mercury lamps, by organic light-emitting diodes (OLEDs) or by UV LEDs.

According to a further embodiment, they are suitable for conversion of light emitted by green LEDs. Suitable LEDs are, for example, those based on GalnNAs, such as Te-doped GalnNAs and Mg-doped GalnNAs. Particularly, they are suitable for conversion of light emitted by white LEDs, especially cold-white light of LEDs, into pleasant light with natural-white color temperature or warm-white color temperature with high conversion efficacy.

They are additionally suitable for applications as a light-collecting system (fluorescence collector) in photovoltaics and in fluorescence conversion solar cells.

In a further embodiment, the inventive color converters are used for the conversion of blue light.

Inventive color converters on irradiation with light, especially with blue LED light, exhibit a high quantum yield and overall energy efficiency. In addition, they have a high photostability on illumination with blue light. Moreover, they are stable toward oxygen and water.

Inventive color converters can be produced by different processes.

In one embodiment, the process for producing inventive color converters comprises the dissolution of the at least one polymer and the at least one organic fluorescent dye and, if present, further organic fluorescent dyes, in a solvent and subsequent removal of the solvent.

In another embodiment, the process for producing inventive color converters comprises the extrusion of the at least one organic fluorescent dye and, if present, further organic fluorescent dyes with the at least one polymer.

The invention further provides lighting devices comprising at least one LED and at least one color converter according to the invention. The at least one LED is preferably blue and emits light preferably within a wavelength range from 400 to 500 nm, preferably 420 to 480 nm, most preferably 430 to 470 nm.

In one embodiment, inventive lighting devices comprise exactly one LED. In another embodiment, inventive lighting devices comprise two or more LEDs.

In one embodiment, inventive lighting devices comprise a plurality of LEDs, all of which are blue. In another embodiment, inventive lighting devices comprise a plurality of LEDs, at least one LED being blue and at least one LED not being blue but emitting light in another color, for example red.

Furthermore, the type of LED used is not crucial for the inventive lighting devices. In a preferred embodiment, the power density of the blue LED light impinging the surface of the converter plate is usually less than 200 mW/cm², preferably less than 120 mW/cm², more preferably less than 80 mW/cm². The use of LEDs of higher power densities, such as 150 or 200 mW/cm², is likewise possible. However, a higher power density of the LED at the converter surface can reduce the lifetime of the fluorescent dyes and the color converters.

Inventive color converters can be used in combination with LEDs in virtually any geometric form and irrespective of the construction of the lighting device.

In one embodiment, color converter and LED are in a phosphor on a chip arrangement.

Preferably, inventive color converters are used in a remote phosphor setup. In this case, the color converter is spatially separated from the LED. In general, the distance between LED and color converter is from 0.1 cm to 50 cm, preferably 0.2 to 10 cm and most preferably 0.5 to 3 cm. Between color converter and LED may be different media such as air, noble gases, nitrogen or other gases or mixtures thereof.

The color converter may, for example, be arranged concentrically around the LED or have a planar geometry. It may take the form, for example, of a plaque, sheet or film, be in droplet form or take the form of a casting.

Inventive lighting devices are suitable for lighting in interiors, outdoors, of offices, of vehicles, in torches, games consoles, streetlights, traffic signs.

Inventive lighting devices exhibit a high quantum yield and high energetic and photometric efficacy. In addition, they have a long lifetime, especially a high photostability on illumination with blue light.

The present invention further provides a device producing electric power upon illumination comprising a photovoltaic cell (solar cell) and the color converter as defined above, where at least a part of the light not absorbed by the photovoltaic cell (solar cell) is absorbed by the color converter. The color converter is usually on top of the photovoltaic cell. The color converter is used to modify the spectrum such that UV and visible light are converted to a more bathochromic spectrum that is converted at higher efficiency by the solar cell.

### Examples

### Example 1: Preparation of a mixture of N,N'-bis(2,6-diisopropylphenyl)-1,7-di(2,6-diphenylphenoxy)perylene-3,4;9,10-tetracarboximide (compound of formula la) and N,N'-bis(2,6-diisopropylphenyl)-1,6-di(2,6-diphenylphenoxy)perylene-3,4;9,10-tetracarboximide (compound of formula Ib)

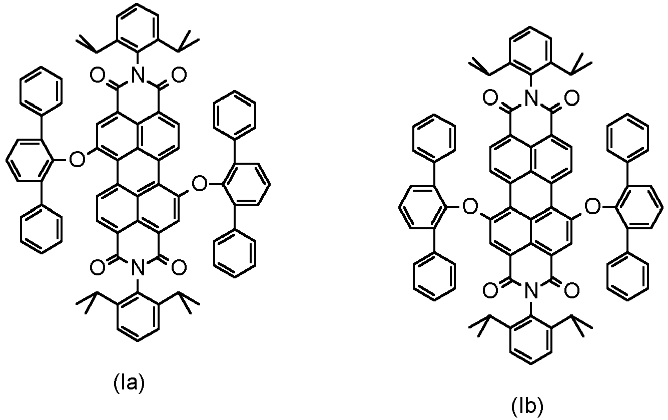

A mixture of 5.0 g (5.7 mmol) of a mixture of N,N'-bis(2,6-diisopropylphenyl)-1,7-dibromo-perylene-3,4;9,10-tetracarboximide and N,N'-bis(2,6-diisopropylphenyl)-1,6-dibromo-perylene-3,4;9,10-tetracarboximide (isomer ratio 75:25), 3.61 g (14.3 mmol) of 2,6-diphenylphenol and 1.99 g (14.3 mmol) of potassium carbonate in 30 mL of N-methylpyrrolidone (NMP) was heated to 50°C and stirred at this temperature for 5 hours. The resulting mixture was filtered, washed two times with 30 mL of NMP and washed three times with 200 mL of water to obtain the pure compound of formula Ia. The resulting purple compound of formula Ia was dried in vacuo at 60°C. The compound of formula Ia was obtained with a purity of 98 % (HPLC) with a yield of 78%.

### Example 2: Preparation of N,N'-bis(2,6-diisopropylphenyl)-1,7-di(2,6-diphenylphenoxy)-perylene-3,4;9,10-tetracarboximide (compound of formula Ia)

N,N'-bis(2,6-diisopropylbenzene)-1,7-di-bromo-3,4,9,10-perylentetracarboxybisimide (prepared according to DE 19547210; 5.07 g, 5.76 mmol), 2,6-diphenylphenol (3.55 g, 14.4 mmol), K₂CO₃ (4.70 g, 34.0 mmol) and NMP (100 mL) were introduced in a three-necks 250 mL flask at room temperature under argon. The mixture was stirred under argon at 200°C for 0.5 hours. The color of the mixture turned from red/pink into green. The heating was turn off.

Ethanol (100 mL) and distilled water (60 mL) were added to the mixture and stirred for 10 minutes at room temperature. The mixture was filtered and the solid was washed with NMP (10 mL), ethanol (20 mLI) and distilled water (20 mL) and dried overnight at 60°C under reduced pressure.

The obtained solid product (6.18 g), NMP (60 mL) and distilled water (30 mL) were introduced in a three necks 150 mL flask and stirred under argon at 100°C for one hour. The heating was turned off and the mixture was slowly cooled to room temperature. When the mixture was at 50°C ethanol (40 mL) was added drop by drop until the mixture was at room temperature. The compound was filtered, washed with NMP (10 mL), ethanol (20 mL) and distilled water (20 mL) and was dried overnight at 60°C under vacuum to afford the title compound as a pink solid (5.34 g, 77%).
Purity (HPLC-MS 254 nm and NMR ¹³C and ¹H) = 97%. R_{f} (DCM) = 0.70.
¹H-NMR (500Mz, CDCl₃, δ(ppm)): 1.13 (4*6H, CH(C*H₃*)₂, d, J=6.95Hz), 2.54 (4*1H, C*H*(CH₃)₂, m, J6.75Hz), 7.00-7.07 (12H, m), 7.33 (4H, d, J=7.85Hz), 7.43 (8H, d, J=4.40Hz), 7.49 (2H, t, J=7.75Hz), 7.55 (6H, aromatic 6*CH diisopropylphenyl, s), 7.98 (2H, 2*CH perylene, s), 8.44 (2H, 2*CH perylene, d, J=8.30Hz), 9.31 (2H, 2*CH perylene, d, J=8.35Hz).

### Materials used:

Polymer 1: transparent polycarbonate based on a polycondensate of bisphenol A and phosgene (Makrolon® 2805 from Bayer MaterialScience AG)
Polymer 2: transparent polystyrene based on a homopolymer of styrene with a density of 1048 kg/m3 and a Vicat softening temperature of 98°C to DIN EN ISO 306 (PS 168 N from BASF SE)
Polymer 3: transparent polyethylene terephthalate PET Terez® 3200, from TER Plastics
Dye 1: Yellow-fluorescent dye 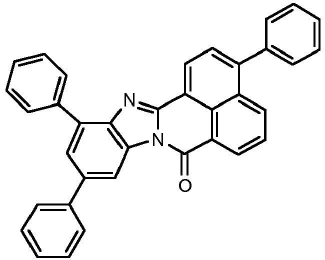 from example 10 of WO 2012/168395 (not according to the invention). The pure compound was obtained by crystallization from toluene.
Dye 2: Red-fluorescent dye N,N'-bis(2,6-diisopropylphenyl)-1,7-di(2,6-diphenyl-phenoxy)perylene-3,4;9,10-tetracarboximide (compound of formula la) according to the present invention 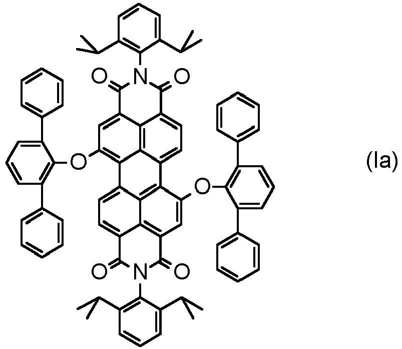
Dye 3 (comparison): Red-fluorescent dye N,N'-bis(2,6-diisopropylphenyl)-1,6,7,12-tetraphenoxyperylene-3,4;9,10-tetracarboxylic acid diimide

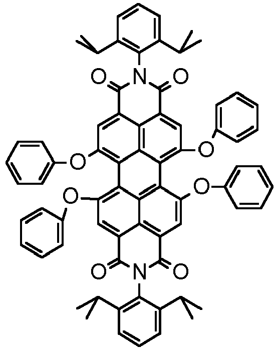

Titanium dioxide: TiO₂ rutile pigment; in the case of polystyrene films: Kronos® 2220; and in the case of polycarbonate films: Kronos® 2233 - both from Kronos Titan

### Spectroscopic measurements

The fluorescence spectra and fluorescence quantum yields were recorded using an absolute quantum yield measurement instrument (C9920-02) manufactured by Hamamatsu, and, if necessary, equipped with cuvettes for liquid samples. The spectra were recorded at an excitation wavelength of 510 nm.

### Sample preparation for dye Ia:

The spectroscopic properties of the dye of the formula la according to the invention were obtained in solution (CH₂Cl₂) and in thin polymer films (polystyrene (PS); polycarbonate (PC)) (thicknesses ca. 60 µm) which comprised the compound of formula la at a concentration of 0.02 % by weight and TiO₂ Kronos 2220 at a concentration of 0.5%, based on the polymer.

The films were prepared by doctor-blading from CH₂Cl₂ solution. About 2.5 g of polymer (PC, PS) and 0.02% by weight of the compound of formula la were dissolved in about 6 - 6.5 mL of methylene chloride, and 0.5% by weight of TiO₂ was dispersed therein, based in each case on the amount of polymer used. The solution/dispersion obtained was coated onto a glass surface using an applicator frame (wet film thickness 400 µm). After the solvent had dried off, the film was detached from the glass and dried in a vacuum drying cabinet at 50°C overnight. Two circular film pieces having a diameter of 22 mm were punched out of each film of thickness, and these served as analysis samples.

### Results of spectroscopic measurements for dye Ia:

| CH₂Cl₂ solution: | |
|---|---|
| Absorption: | λₘₐₓ = 562 nm, εₘₐₓ 98800 kg/mol•cm |
| Emission: | λₘₐₓ = 598 nm, 626.5 nm, FQ = 98 % |

| PS film: | |
|---|---|
| Emission: | λₘₐₓ (PS) = 579 nm, 611 nm, FQ = 97% |

| PC film: | |
|---|---|
| Emission: | λₘₐₓ (PC) = 582 nm, 613 nm, FQ = 95% |

### Production of the color converters:

Fluorescent dye la according to the invention (dye 2) was used in combination with the dye 1 and fluorescent dye 3 (not according to the invention) was used in combination with dye 1, respectively, to produce color converters. For this purpose, the two dyes together with TiO₂ (Kronos 2233) were incorporated into a matrix composed of the polymer PC.

To produce the converter material, polycarbonate, dyes and TiO₂ according to the desired concentrations (see Table II) were mixed with methylene chloride. The solution/dispersion obtained was coated onto a glass surface using an applicator frame (wet film thickness 400 µm). After the solvent had dried off, the film was detached from the glass and dried.

**Table II:**

| Example | Polymer | yellow fluorescent dye 1 [% by wt.] | red fluorescent dye / [% by wt.] | TiO₂ [% by wt.] | Film thickness [µm] |
|---|---|---|---|---|---|
| C1 | PC | 0.13 | dye 3 / 0.009 | 0.80 | 135 |
| C2 | PC | 0.09 | dye 3 / 0.0062 | 0.80 | 130 |
| A1 | PC | 0.13 | dye 2 / 0.0097 | 0.80 | 143 |
| A2 | PC | 0.09 | dye 2 / 0.0060 | 0.80 | 138 |

The amounts of yellow fluorescent dye 1, red-fluorescent dye 2, red-fluorescent dye 3 and TiO₂ are based on the amount of polymer polycarbonate used.

### Lighting devices:

With these color converter films a lighting device was constructed. This device is an arrangement which consists of a cup-like, highly reflective mixing chamber with 16 blue LEDs (peak wavelength 450-452 nm) arranged at the bottom. This mixing chamber is covered by the inventive color converter film on top.

### Characterization of the lighting devices:

The light irradiated from the surface of the converter plaque was subjected to a photometric measurement, where the total light from irradiated from the device was measured by an integral measurement with an Ulbricht sphere, ISP 500-100, and a CAS 140CT-156 CCD detector (from Instrument Systems, Munich). The spectrum was used to determine the relevant photometric parameters such as CIELAB coordinates x, y and the CCT (= correlated color temperature), distance from the Planck curve dᵤᵥ, etc.

The energy efficiency is defined as the integral of the intensities (∼mW/cm²•nm) of the generated white light divided through the integral of the intensities of the blue light which is used for pumping the device. The luminous efficacy (in lumen/Watt) is defined as lumen output of the device divided by the integrated intensities of the blue light which is used for pumping the device.

**Table III**

| Example | CIELAB -x | CIELAB -y | CCT [K] | Planck distance [dᵤᵥ] | energy efficiency [%] | optical efficacy [Lm/ W] |
|---|---|---|---|---|---|---|
| blue LED 450 nm | 0.1539 | 0.0232 | | | | |
| C1 | 0.4337 | 0.4025 | 3042 | -1.82E-04 | 61.83 | 188.4 |
| C2 | 0.3746 | 0.3592 | 4032 | -6.72E-03 | 66.80 | 197.2 |
| A1 | 0.4328 | 0.4006 | 3042 | -8.09E-04 | 62.54 | 211.3 |
| A2 | 0.3828 | 0.3786 | 3951 | 1.82E-04 | 66.15 | 221.0 |

Table III shows that the inventive color converters (examples A1 and A2) as well as the non-inventive color converters (examples C1 and C2) produce a warm-white and neutral-white light of high quality (near to Planck curve). However, color converters comprising the inventive red-fluorescent dye allow to provide white LEDs with a higher luminous efficacy in comparison to color converters comprising a red-fluorescent dye known from prior art.

## Claims

1. Perylene bisimide compound of the formulae Ia or Ib 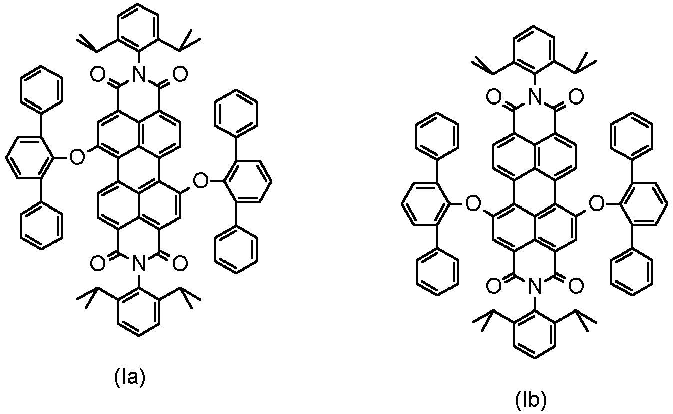 or mixtures thereof.

2. A color converter comprising at least one polymer as a matrix and at least one compound of the formula la or Ib or a mixture of compounds of formulae Ia and Ib as defined in claim 1 as a fluorescent dye, wherein the at least one polymer is selected from polystyrene, polycarbonate, polymethylmethacrylate, polyvinylpyrrolidone, polymethacrylate, polyvinyl acetate, polyvinyl chloride, polybutene, silicone, polyacrylate, epoxy resin, polyvinyl alcohol, poly(ethylene vinylalcohol)-coplymer (EVA, EVOH), polyacrylonitrile, polyvinylidene chloride (PVDC), polystyreneacrylonitrile (SAN), polybutylene terephthalate (PBT), polyethylene terephthalate (PET), polyvinyl butyrate (PVB), polyvinyl chloride (PVC), polyamides, polyoxymethylenes, polyimides, polyetherimides and mixtures thereof.

3. The color converter according to claim 2, wherein the at least one polymer consists essentially of polystyrene, polycarbonate, or polyethylene terephthalate.

4. The color converter according to claim 2 or 3, comprising as fluorescent dye a compound of the formula (Ia).

5. The color converter according to any of claims 2 to 4, wherein the color converter additionally comprises at least one inorganic white pigment as a scattering body.

6. The color converter according to any of claims 2 to 5, comprising at least one further organic fluorescent dye selected from
(i) a cyanated naphthalene benzimidazole compound of the formula II 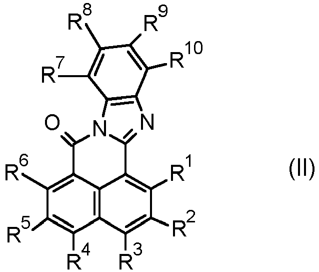 wherein
R¹, R², R³, R⁴, R⁵, R⁶, R⁷, R⁸, R⁹ and R¹⁰ are each independently hydrogen, cyano or aryl which is unsubstituted or has one or more identical or different substituents R^{Ar},
where
each R^{Ar} is independently selected from cyano, hydroxyl, mercapto, halogen, C₁-C₂₀-alkoxy, C₁-C₂₀-alkylthio, nitro, -NR^{Ar2}R^{Ar3}, -NR^{Ar2}COR^{Ar3}, -CONR^{Ar2}R^{Ar3}, -SO₂NR^{Ar2}R^{Ar3}, -COOR^{Ar2}, -SO₃R^{Ar2},
C₁-C₃₀-alkyl, C₂-C₃₀-alkenyl, C₂-C₃₀-alkynyl, where the three latter radicals are unsubstituted or bear one or more R^{a} groups,
C₃-C₈-cycloalkyl, 3- to 8-membered heterocyclyl, where the two latter radicals are unsubstituted or bear one or more R^{b} groups,
aryl, U-aryl, heteroaryl and U-heteroaryl, where the four latter radicals are unsubstituted or bear one or more R^{b} groups,
where
each R^{a} is independently selected from cyano, hydroxyl, oxo, mercapto, halogen, C₁-C₂₀-alkoxy, C₁-C₂₀-alkylthio, nitro, -NR^{Ar2}R^{Ar3}, -NR^{Ar2}COR^{Ar3}, -CONR^{Ar2}R^{Ar3}, -SO₂NR^{Ar2}R^{Ar3}, -COOR^{Ar2}, -SO₃R^{Ar2}, C₃-C₈-cycloalkyl, 3- to 8-membered heterocyclyl, aryl and heteroaryl, where the cycloalkyl, heterocyclyl, aryl and heteroaryl radicals are unsubstituted or bear one or more R^{b} groups;
each R^{b} is independently selected from cyano, hydroxyl, oxo, mercapto, halogen, C₁-C₂₀-alkoxy, C₁-C₂₀-alkylthio, nitro, -NR^{Ar2}R^{Ar3}, -NR^{Ar2}COR^{Ar3}, -CONR^{Ar2}R^{Ar3}, -SO₂NR^{Ar2}R^{Ar3}, -COOR^{Ar2}, -SO₃R^{Ar2}, C₁-C₁₈-alkyl, C₂-C₁₈-alkenyl, C₂-C₁₈-alkynyl, C₃-C₈-cycloalkyl, 3- to 8-membered heterocyclyl, aryl and heteroaryl, where the four latter radicals are unsubstituted or bear one or more R^{b1} groups,
each R^{b1} is independently selected from cyano, hydroxyl, mercapto, oxo, nitro, halogen, -NR^{Ar2}R^{Ar3}, -NR^{Ar2}COR^{Ar3}, -CONR^{Ar2}R^{Ar3}, -SO₂NR^{Ar2}R^{Ar3}, -COOR^{Ar2}, -SO₃R^{Ar2}, -SO₃R^{Ar2}, C₁-C₁₈-alkyl, C₂-C₁₈-alkenyl, C₂-C₁₈-alkynyl, C₁-C₁₂-alkoxy, C₁-C₁₂-alkylthio,
U is an -O-, -S-, -NR^{Ar1}-, -CO-, -SO- or -SO₂- moiety;
R^{Ar1}, R^{Ar2}, R^{Ar3} are each independently hydrogen, C₁-C₁₈-alkyl, 3- to 8-membered cycloalkyl, 3- to 8-membered heterocyclyl, aryl or heteroaryl, where alkyl is unsubstituted or bears one or more R^{a} groups, where 3- to 8-membered cycloalkyl, 3- to 8-membered heterocyclyl, aryl and heteroaryl are unsubstituted or bear one or more R^{b} groups;
with the proviso that the compound of the formula II comprises at least one cyano group,
or mixtures thereof;
(ii) a cyanated perylene compound of the formula (III) 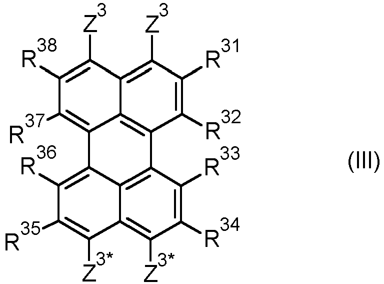 in which
one of the Z³ substituents is cyano and the other Z³ substituent is CO₂R³⁹, CONR³¹⁰R³¹¹, C₁-C₁₈-alkyl, C₂-C₁₈-alkenyl, C₂-C₁₈-alkynyl, C₃-C₁₂-cycloalkyl or C₆-C₁₄-aryl, where
C₁-C₁₈-alkyl, C₂-C₁₈-alkenyl, C₂-C₁₈-alkynyl are unsubstituted or bear one or more identical or different Z^{3a} substituents,
C₃-C₁₂-cycloalkyl is unsubstituted or bears one or more identical or different Z^{3b} substituents, and
C₆-C₁₄-aryl is unsubstituted or bears one or more identical or different Z^{3Ar} substituents;
one of the Z^{3*} substituents is cyano and the other Z^{3*} substituent is CO₂R³⁹, CONR³¹⁰R³¹¹, C₁-C₁₈-alkyl, C₂-C₁₈-alkenyl, C₂-C₁₈-alkynyl, C₃-C₁₂-cycloalkyl or C₆-C₁₄-aryl, where
C₁-C₁₈-alkyl, C₂-C₁₈-alkenyl, C₂-C₁₈-alkynyl are unsubstituted or bear one or more identical or different Z^{3a} substituents,
C₃-C₁₂-cycloalkyl is unsubstituted or bears one or more identical or different Z^{3b} substituents, and
C₆-C₁₄-aryl is unsubstituted or bears one or more identical or different Z^{3Ar} substituents;
R³¹, R³², R³³, R³⁴,R³⁵, R³⁶, R³⁷ and R³⁸ are each independently selected from hydrogen, cyano, bromine and chlorine,
with the proviso that 1, 2, 3, 4, 5, 6, 7 or 8 of the R³¹, R³², R³³, R³⁴, R³⁵, R³⁶, R³⁷ or R³⁸ substituents are cyano;
where
R³⁹ is hydrogen, C₁-C₁₀-alkyl, C₂-C₁₀-alkenyl, C₂-C₁₀-alkynyl, C₃-C₁₂-cycloalkyl or C₆-C₁₄-aryl, where
C₁-C₁₀-alkyl, C₂-C₁₀-alkenyl, C₂-C₁₀-alkynyl are unsubstituted or bear one or more identical or different R^{3a} substituents, C₃-C₁₂-cycloalkyl is unsubstituted or bears one or more identical or different R^{3b} substituents and
C₆-C₁₄-aryl is unsubstituted or bears one or more identical or different R^{3Ar} substituents;
R³¹⁰ and R³¹¹ are each independently hydrogen, C₁-C₁₀-alkyl, C₂-C₁₀-alkenyl, C₂-C₁₀-alkynyl, C₃-C₁₂-cycloalkyl or C₆-C₁₄-aryl, where C₁-C₁₀-alkyl, C₂-C₁₀-alkenyl, C₂-C₁₀-alkynyl are unsubstituted or bear one or more identical or different R^{3a} substituents,
C₃-C₁₂-cycloalkyl is unsubstituted or bears one or more identical or different R^{3b} substituents and C₆-C₁₄-aryl is unsubstituted or bears one or more identical or different R^{3Ar} substituents;
each Z^{3a} is independently halogen, hydroxyl, NR^{310a}R^{311a}, C₁-C₁₀-alkoxy, C₁-C₁₀-haloalkoxy, C₁-C₁₀-alkylthio, C₃-C₁₂-cycloalkyl, C₆-C₁₄-aryl, C(=O)R^{39a}; C(=O)OR^{39a} or C(O)NR^{310a}R^{311a}, where
C₃-C₁₂-cycloalkyl is unsubstituted or bears one or more identical or different R^{3b} substituents and
C₆-C₁₄-aryl is unsubstituted or bears one or more identical or different R^{3Ar} substituents;
each Z^{3b} and each Z^{3Ar} is independently halogen, hydroxyl, NR^{310a}R^{311a}, C₁-C₁₀-alkyl, C₁-C₁₀-alkoxy, C₁-C₁₀-haloalkoxy, C₁-C₁₀-alkylthio, C(=O)R^{39a}; C(=O)OR^{39a} or C(O)NR^{310a}R^{311a};
each R^{3a} is independently halogen, hydroxyl, C₁-C₁₀-alkoxy, C₃-C₁₂-cycloalkyl or C₆-C₁₄-aryl;
each R^{3b} is independently halogen, hydroxyl, C₁-C₁₀-alkyl, C₁-C₁₀-alkoxy, C₁-C₁₀-haloalkoxy, C₁-C₁₀-alkylthio, C₂-C₁₀-alkenyl, C₂-C₁₀-alkynyl, C₃-C₁₂-cycloalkyl or C₆-C₁₄-aryl;
each R^{3Ar} is independently halogen, hydroxyl, C₁-C₁₀-alkyl, C₁-C₁₀-alkoxy, C₁-C₁₀-haloalkoxy, C₁-C₁₀-alkylthio, C₂-C₁₀-alkenyl, C₂-C₁₀-alkynyl, C₃-C₁₂-cycloalkyl or C₆-C₁₄-aryl;
R^{39a} is hydrogen, C₁-C₁₀-alkyl, C₂-C₁₀-alkenyl, C₂-C₁₀-alkynyl, C₃-C₁₂-cycloalkyl or C₆-C₁₄-aryl; and
R^{310a}, R^{311a} are each independently hydrogen, C₁-C₁₀-alkyl, C₂-C₁₀-alkenyl, C₂-C₁₀-alkynyl, C₃-C₁₂-cycloalkyl or C₆-C₁₄-aryl,
and mixtures thereof.
(iii) a cyanated compound of the formula (IV) 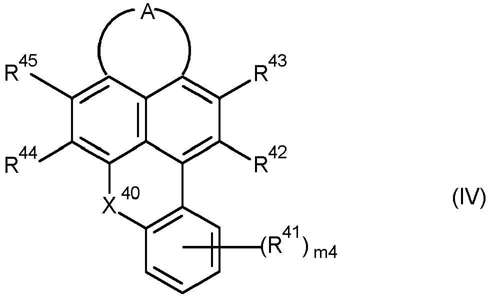 wherein
m4 is 0, 1, 2, 3 or 4;
each R⁴¹ independently from each other is selected from bromine, chlorine, cyano, -NR^{4a}R^{4b}, C₁-C₂₄-alkyl, C₁-C₂₄-haloalkyl, C₁-C₂₄-alkoxy, C₁-C₂₄-haloalkoxy, C₃-C₂₄-cycloalkyl, heterocycloalkyl, heteroaryl, C₆-C₂₄-aryl, C₆-C₂₄-aryloxy, C₆-C₂₄-aryl-C₁-C₁₀-alkylene, where the rings of cycloalkyl, heterocycloalkyl, heteroaryl, aryl, aryloxy in the six last-mentioned radicals are unsubstituted or substituted with 1, 2, 3, 4 or 5 identical or different radicals R^{41a} and where C₁-C₂₄-alkyl, C₁-C₂₄-haloalkyl, C₁-C₂₄-alkoxy, and the alkylene moiety of C₆-C₂₄-aryl-C₁-C₁₀-alkylene may be interrupted by one or more groups selected from O, S and NR^{4c};
at least one of the radicals R⁴², R⁴³, R⁴⁴ and R⁴⁵ is CN, and the remaining radicals, independently from each other, are selected from hydrogen, chlorine and bromine;
X⁴⁰ is O, S, SO or SO₂;
A is a diradical selected from diradicals of the general formulae (A.1), (A.2), (A.3), and (A.4) 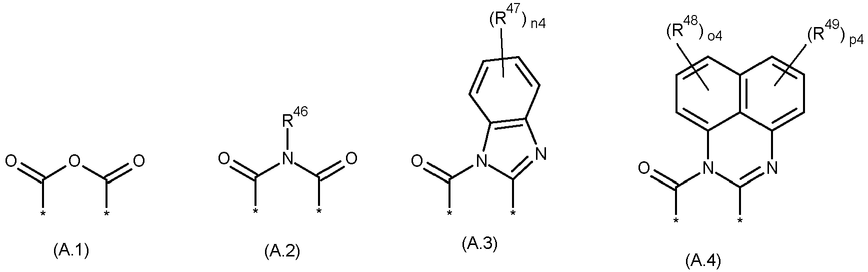
wherein
* in each case denotes the point of attachments to the remainder of the molecule;
n4 is 0, 1, 2, 3, or 4;
o4 is 0, 1, 2, or 3;
p4 is 0, 1, 2, or 3;
R⁴⁶ is hydrogen, C₁-C₂₄-alkyl, C₁-C₂₄-haloalkyl, C₃-C₂₄-cycloalkyl, C₆-C₂₄-aryl or C₆-C₂₄-aryl-C₁-C₁₀-alkylene, where the rings of cycloalkyl, aryl, and aryl-alkylene in the three last-mentioned radicals are unsubstituted or substituted with 1, 2, 3, 4 or 5 identical or different radicals R^{46a}, and where C₁-C₂₄-alkyl, C₁-C₂₄-haloalkyl and the alkylene moiety of C₆-C₂₄-aryl-C₁-C₁₀-alkylene may be interrupted by one or more heteroatoms or heteroatomic groups selected from O, S and NR^{4c};
each R⁴⁷ independently from each other is selected from bromine, chlorine, cyano, -NR^{4a}R^{4b}, C₁-C₂₄-alkyl, C₁-C₂₄-haloalkyl, C₁-C₂₄-alkoxy, C₁-C₂₄-haloalkoxy, C₃-C₂₄-cycloalkyl, heterocycloalkyl, heteroaryl, C₆-C₂₄-aryl, C₆-C₂₄-aryloxy, C₆-C₂₄-aryl-C₁-C₁₀-alkylene, where the rings of cycloalkyl, heterocycloalkyl, heteroaryl, aryl and aryl-alkylene in the six last-mentioned radicals are unsubstituted or substituted with 1, 2, 3, 4 or 5 identical or different radicals R^{47a} and where C₁-C₂₄-alkyl, C₁-C₂₄-haloalkyl, C₁-C₂₄-alkoxy, C₁-C₂₄-haloalkoxy, and the alkylene moiety of C₆-C₂₄-aryl-C₁-C₁₀-alkylene may be interrupted by one or more groups selected from O, S and NR^{4c};
each R⁴⁸ independently from each other is selected from bromine, chlorine, cyano, NR^{4a}R^{4b}, C₁-C₂₄-alkyl, C₁-C₂₄-haloalkyl, C₁-C₂₄-alkoxy, C₁-C₂₄-haloalkoxy, C₃-C₂₄-cycloalkyl, heterocycloalkyl, heteroaryl, C₆-C₂₄-aryl, C₆-C₂₄-aryloxy, C₆-C₂₄-aryl-C₁-C₁₀-alkylene, where the rings of cycloalkyl, heterocycloalkyl, heteroaryl, aryl and aryl-alkylene in the six last-mentioned radicals are unsubstituted or substituted with 1, 2, 3, 4 or 5 identical or different radicals R^{48a} and where C₁-C₂₄-alkyl, C₁-C₂₄-haloalkyl, C₁-C₂₄-alkoxy, C₁-C₂₄-haloalkoxy, and the alkylene moiety of C₆-C₂₄-aryl-C₁-C₁₀-alkylene may be interrupted by one or more groups selected from O, S and NR^{4c};
each R⁴⁹ independently from each other is selected from bromine, chlorine, cyano, NR^{4a}R^{4b}, C₁-C₂₄-alkyl, C₁-C₂₄-haloalkyl, C₁-C₂₄-alkoxy, C₁-C₂₄-haloalkoxy, C₃-C₂₄-cycloalkyl, heterocycloalkyl, heteroaryl, C₆-C₂₄-aryl, C₆-C₂₄-aryloxy, C₆-C₂₄-aryl-C₁-C₁₀-alkylene, where the rings of cycloalkyl, heterocycloalkyl, heteroaryl, aryl and aryl- alkylene in the six last-mentioned radicals are unsubstituted or substituted with 1, 2, 3, 4 or 5 identical or different radicals R^{49a} and where C₁-C₂₄-alkyl, C₁-C₂₄-haloalkyl, C₁-C₂₄-alkoxy, C₁-C₂₄-haloalkoxy, and the alkylene moiety of C₆-C₂₄-aryl-C₁-C₁₀-alkylene may be interrupted by one or more groups selected from O, S and NR^{4c};
R^{41a}, R^{46a}, R^{47a} R^{48a}, R^{49a} are independently of one another selected from C₁-C₂₄-alkyl, C₁-C₂₄-fluoroalkyl, C₁-C₂₄-alkoxy, fluorine, chlorine and bromine;
R^{4a}, R^{4b}, R^{4c} are independently of one another are selected from hydrogen, C₁-C₂₀-alkyl, C₃-C₂₄-cycloalkyl, heterocycloalkyl, heteroaryl and C₆-C₂₄-aryl;
and mixtures thereof;
(iv) a benzoxanthene compound of the formula (V) 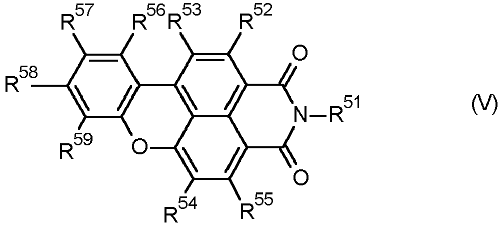 wherein
R⁵¹ is phenyl which is unsubstituted or carries 1, 2, 3, 4, or 5 substituents selected from halogen, R⁵¹¹, OR⁵⁵², NHR⁵⁵² and NR⁵⁵²R⁵⁵⁷;
R⁵², R⁵³, R⁵⁴, R⁵⁵, R⁵⁶, R⁵⁷, R⁵⁸ and R⁵⁹ are independently of each other selected from hydrogen, halogen, R⁵⁵³, OR⁵⁵³, NHR⁵⁵³ and NR⁵⁵³R⁵⁵⁴, wherein
R⁵¹¹ is selected from C₂-C₁₈-alkyl, C₆-C₂₄-aryl and heteroaryl;
R⁵⁵² and R⁵⁵⁷ are independently of each other selected from C₁-C₁₈-alkyl, C₆-C₂₄-aryl and heteroaryl; and
R⁵⁵³ and R⁵⁵⁴ are independently of each other selected from C₁-C₁₈-alkyl, C₆-C₂₄-aryl and heteroaryl;
and mixtures thereof;
(v) a fluorescent compound comprising at least one structural unit of formula (VI) 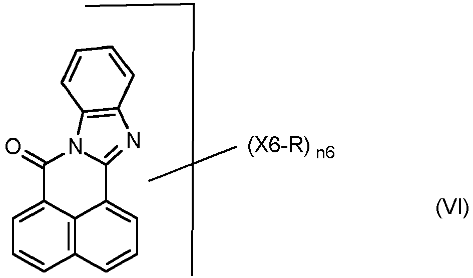 where one or more CH groups of the six-membered ring of the benzimidazole structure shown may be replaced by nitrogen and where the symbols are each defined as follows:
n6 is a number from 0 to (10-p6) for each structural unit of the formula (VI); where p6 is the number of CH units which have been replaced by nitrogen in the six-membered ring of the benzimidazole structure shown
X6 is a chemical bond, O, S, SO, SO₂, NR⁶¹; and
R is an aliphatic radical, cycloaliphatic radical, aryl, heteroaryl, each of which may bear any desired substituents,
an aromatic or heteroaromatic ring or ring system, each of which is fused to other aromatic rings of the structural unit of the formula (VI) is F, Cl, Br, CN, H when X6 is not a chemical bond;
where two R radicals may be joined to give one cyclic radical and where X6 and R, when n6 > one, may be the same or different;
R⁶¹ is each independently hydrogen, C₁-C₁₈-alkyl or cycloalkyl, the carbon chain of which may comprise one or more -O-, -S-, -CO-, -SO- and/or -SO₂- moieties and which may be mono- or polysubstituted; aryl or heteroaryl which may be mono- or polysubstituted;
and mixtures thereof;
and
(vi) a perylene compound of the formulae (VII), (VIII) or (IX) 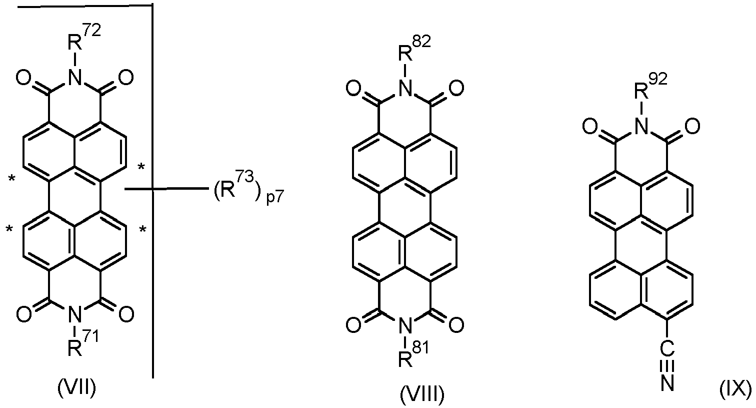 where
p7 is 1 to 4,
R⁷¹, R⁷² are each independently C₁-C₃₀-alkyl, C₃-C₈-cycloalkyl, aryl, heteroaryl, aryl-C₁-C-₁₀-alkylene, where the aromatic ring in the three latter radicals is unsubstituted or mono- or polysubstituted by C₁-C₁₀-alkyl;
R⁷³ is aryloxy which is unsubstituted or mono- or polysubstituted by C₁-C₁₀-alkyl, where the R⁷³ radicals are at one or more of the positions indicated by *;
R⁸¹, R⁸² are each independently C₁-C₃₀-alkyl, C₃-C₈-cycloalkyl, aryl, heteroaryl, aryl-C₁-C₁₀-alkylene, where the aromatic ring in the three latter radicals is unsubstituted or mono- or polysubstituted by C₁-C₁₀-alkyl;
R⁹² is C₁-C₃₀-alkyl, C₃-C₈-cycloalkyl, aryl, heteroaryl, aryl-C₁-C₁₀-alkylene, where the aromatic ring in the three latter radicals is unsubstituted or mono- or polysubstituted by C₁-C₁₀-alkyl
and mixtures thereof.

7. The color converter according to claim 6, wherein the at least one further organic fluorescent dye is selected from compounds of formula (II), compounds of formula (IV), compounds of formula (V), compounds comprising at least one structural unit of formula (VI), compounds of formula (VII) and mixtures thereof.

8. The color converter according to any of claims 2 to 7, comprising as further fluorescent material at least one inorganic fluorescent material selected from garnets, silicates, sulfides, nitrides and oxynitrides.

9. The color converter according to any of claims 2 to 8 comprising at least one quantum dot from a crystalline semiconductor material.

10. The use of a color converter as defined in any of claims 2 to 9, for conversion of light generated by LEDs or OLEDs.

11. The use of a color converter as defined in claim 10 for conversion of light generated by a UV LED, by a blue LED or by a white LED.

12. The use of a color converter as defined in any of claims 2 to 9 in displays.

13. A lighting device comprising at least one LED and at least one color converter according to any of claims 2 to 9, the LED and color converter being preferably in a remote phosphor arrangement.

14. A device producing electric power upon illumination comprising a photovoltaic cell and the color converter as defined in any of claims 2 to 9, where at least a part of the light not absorbed by the photovoltaic cell is absorbed by the color converter.

15. A method for preparing compounds of the formulae la and lb 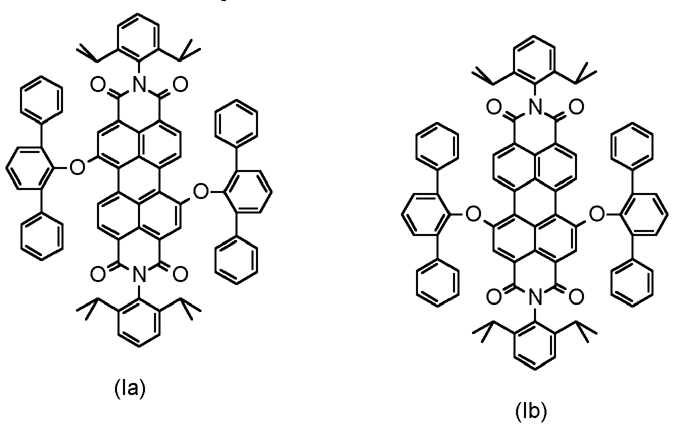 or mixtures thereof,
comprising
a) providing a compound of formulae (1) or (2) 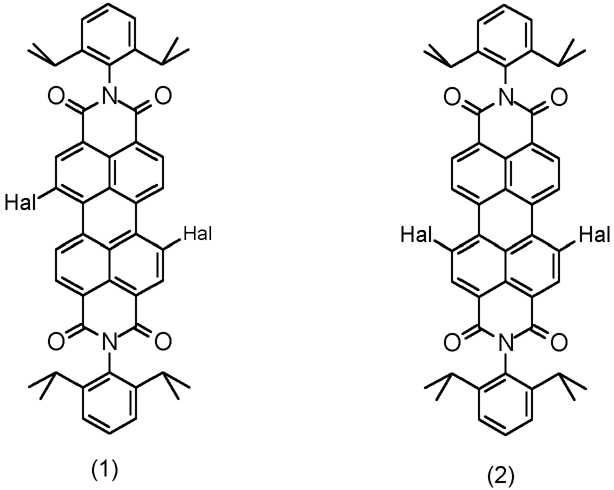 or a mixture thereof
wherein
Hal are each all chlorine or each all bromine;
b) reacting the compound of formula (1) or the compound of formula (2) or a mixture of compounds of formulae (1) or (2) provided in step (a) with 2,6-diphenylphenol in the presence of an inorganic base and in the presence of a polar aprotic solvent to obtain a compound of formula (Ia) or (Ib) or a mixture thereof;
(c) optionally, subjecting the reaction mixture obtained in step b) to at least one work-up step.

16. The use of a perylene bisimide compound of the formula la or lb or a mixture thereof as defined in claim 1, in color converters for converting light emitted from a light source, in particular a light source selected from LEDs and OLEDs, into light of a second, longer wavelength, for coloring coatings, printing inks and plastics, producing aqueous polymer dispersions which absorb and/or emit electromagnetic radiation, for data storage, for optical labels, for security labels in documents and for brand protection or as a fluorescent label for biomolecules.

## Patentansprüche

1. Perylenbisimid-Verbindung der Formeln Ia oder Ib 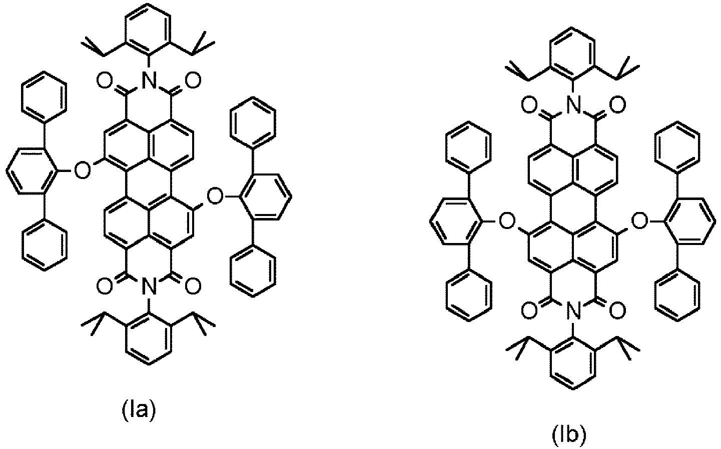 oder Mischungen davon.

2. Farbkonverter, umfassend mindestens ein Polymer als Matrix und mindestens eine Verbindung der Formel Ia oder Ib oder eine Mischung von Verbindungen der Formeln Ia und Ib gemäß Anspruch 1 als Fluoreszenzfarbstoff, wobei das mindestens eine Polymer aus Polystyrol, Polycarbonat, Polymethylmethacrylat, Polyvinylpyrrolidon, Polymethacrylat, Polyvinylacetat, Polyvinylchlorid, Polybuten, Silikon, Polyacrylat, Epoxidharz, Polyvinylalkohol, Poly(ethylen-vinylalkohol)-Copolymer (EVA, EVOH), Polyacrylnitril, Polyvinylidenchlorid (PVDC), Polystyrolacrylnitril (SAN), Polybutylenterephthalat (PBT), Polyethylenterephthalat (PET), Polyvinylbutyrat (PVB), Polyvinylchlorid (PVC), Polyamiden, Polyoxymethylenen, Polyimiden, Polyetherimiden und Mischungen davon ausgewählt ist.

3. Farbkonverter nach Anspruch 2, wobei das mindestens eine Polymer im Wesentlichen aus Polystyrol, Polycarbonat oder Polyethylenterephthalat besteht.

4. Farbkonverter nach Anspruch 2 oder 3, der als Fluoreszenzfarbstoff eine Verbindung der Formel (Ia) umfasst.

5. Farbkonverter nach einem der Ansprüche 2 bis 4, wobei der Farbkonverter zusätzlich mindestens ein anorganisches Weißpigment als Streukörper umfasst.

6. Farbkonverter nach einem der Ansprüche 2 bis 5, umfassend mindestens einen weiteren organischen Fluoreszenzfarbstoff, der ausgewählt ist aus
(i) einer cyanierten Naphthalinbenzimidazol-Verbindung der Formel II 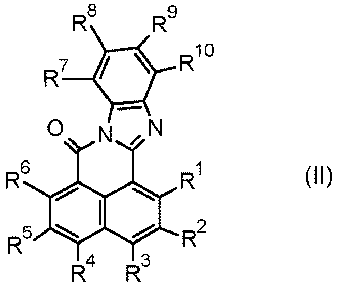 worin
R¹, R², R³, R⁴, R⁵, R⁶, R⁷, R⁸, R⁹ und R¹⁰ jeweils unabhängig voneinander für Wasserstoff, Cyano oder Aryl, das unsubstituiert ist oder einen oder mehrere gleiche oder verschiedene Substituenten R^{Ar} aufweist,
wobei
jedes R^{Ar} unabhängig voneinander aus Cyano, Hydroxy, Mercapto, Halogen, C₁-C₂₀-Alkoxy, C₁-C₂₀-Alkylthio, Nitro,-NR^{Ar2}R^{Ar3}, -NR^{Ar2}COR^{Ar3}, -CONR^{Ar2}R^{Ar3}, -SO₂NR^{Ar2}R^{Ar3}, -COOR^{Ar2} und -SO₃R^{Ar2} ausgewählt ist,
C₁-C₃₀-Alkyl, C₂-C₃₀-Alkenyl, C₂-C₃₀-Alkinyl, wobei die drei letztgenannten Reste unsubstituiert sind oder eine oder mehrere Gruppen R^{a} tragen,
C₃-C₈-Cycloalkyl, 3- bis 8-gliedriges Heterocyclyl, wobei die zwei letztgenannten Reste unsubstituiert sind oder eine oder mehrere Gruppen R^{b} tragen, Aryl, U-Aryl, Heteroaryl und U-Heteroaryl, wobei die vier letztgenannten Reste unsubstituiert sind oder eine oder mehrere Gruppen R^{b} tragen, stehen,
wobei
jedes R^{a} unabhängig voneinander aus Cyano, Hydroxy, Oxo, Mercapto, Halogen, C₁-C₂₀-Alkoxy, C₁-C₂₀-Alkyl-thio, Nitro, -NR^{Ar2}R^{Ar3}, -NR^{Ar2}COR^{Ar3}, -CONR^{Ar2}R^{Ar3}, -SO₂NR^{Ar2}R^{Ar3}, -COOR^{Ar2}, -SO₃R^{Ar2}, C₃-C₈-Cycloalkyl, 3- bis 8-gliedrigem Heterocyclyl, Aryl und Heteroaryl ausgewählt ist, wobei die Cycloalkyl-, Heterocyclyl-, Aryl- und Heteroarylreste unsubstituiert sind oder eine oder mehrere Gruppen R^{b} tragen;
jedes R^{b} unabhängig voneinander aus Cyano, Hydroxy, Oxo, Mercapto, Halogen, C₁-C₂₀-Alkoxy, C₁-C₂₀-Alkyl-thio, Nitro, -NR^{Ar2}R^{Ar3}, -NR^{Ar2}COR^{Ar3}, -CONR^{Ar2}R^{Ar3}, - SO₂NR^{Ar2}R^{Ar3}, -COOR^{Ar2}, -SO₃R^{Ar2}, C₁-C₁₈-Alkyl, C₂-C₁₈-Alkenyl, C₂-C,₈-Alkinyl, C₃-C₈-Cycloalkyl, 3-bis 8-gliedrigem Heterocyclyl, Aryl und Heteroaryl ausgewählt ist, wobei die sieben letztgenannten Reste unsubstituiert sind oder eine oder mehrere Gruppen R^{b1} tragen,
jedes R^{b1} unabhängig voneinander aus Cyano, Hydroxy, Mercapto, Oxo, Nitro, Halogen, -NR^{Ar2}R^{Ar3}, -NR^{Ar2}COR^{Ar3}, -CONR^{Ar2}R^{Ar3}, - SO₂NR^{Ar2}R^{Ar3}, -COOR^{Ar2}, -SO₃R^{Ar2}, -SO₃R^{Ar2}, C₁-C₁₈-Alkyl, C₂-C₁₈-Alkenyl, C₂-C,₈-Alkinyl, C₁-C₁₂-Alkoxy und C₁-C₁₂-Alkylthio ausgewählt ist,
U für eine Gruppierung -0-, -S-, -NR^{Ar1}-, -CO-, -SO- oder -SO₂- steht;
R^{Ar1}, R^{Ar2}, R^{Ar3} unabhängig voneinander für Wasserstoff, C₁-C₁₈-Alkyl, 3- bis 8-gliedriges Cycloalkyl, 3- bis 8-gliedriges Heterocyclyl, Aryl oder Heteroaryl stehen, wobei Alkyl unsubstituiert ist oder eine oder mehrere Gruppen R^{a} trägt, wobei 3- bis 8-gliedriges Cycloalkyl, 3- bis 8-gliedriges Heterocyclyl, Aryl und Heteroaryl unsubstituiert sind oder eine oder mehrere Gruppen R^{b} tragen;
mit der Maßgabe, dass die Verbindung der Formel II mindestens eine Cyanogruppe enthält,
oder Mischungen davon;
(ii) einer cyanierten Perylen-Verbindung der Formel (III) 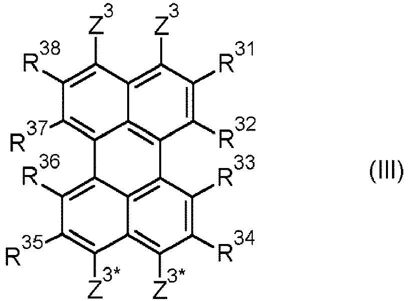 worin
einer der Substituenten Z³ für Cyano steht und der andere Substituent Z³ für CO₂R³⁹, CONR³¹⁰R³¹¹, C₁-C₁₈-Alkyl, C₂-C₁₈-Alkenyl, C₂-C₁₈-Alkinyl, C₃-C₁₂-Cycloalkyl oder C₆-C₁₄-Aryl steht, wobei
C₁-C₁₈-Alkyl, C₂-C₁₈-Alkenyl, C₂-C₁₈-Alkinyl unsubstituiert sind oder einen oder mehrere, gleiche oder verschiedene Substituenten Z^{3a} tragen,
C₃-C₁₂-Cycloalkyl unsubstituiert ist oder einen oder mehrere gleiche oder verschiedene Substituenten Z^{3b} trägt und C₆-C₁₄-Aryl unsubstituiert ist oder einen oder mehrere gleiche oder verschiedene Substituenten Z^{3Ar} trägt;
einer der Substituenten Z^{3*} für Cyano steht und der andere Substituent Z^{3*} für CO₂R³⁹, CONR³¹⁰R³¹¹, C₁-C₁₈-Alkyl, C₂-C₁₈-Alkenyl, C₂-C,₈-Alkinyl, C₃-C₁₂-Cycloalkyl oder C₆-C₁₄-Aryl steht, wobei
C₁-C₁₈-Alkyl, C₂-C₁₈-Alkenyl, C₂-C₁₈-Alkinyl unsubstituiert sind oder einen oder mehrere, gleiche oder verschiedene Substituenten Z^{3a} tragen,
C₃-C₁₂-Cycloalkyl unsubstituiert ist oder einen oder mehrere gleiche oder verschiedene Substituenten Z^{3b} trägt und C₆-C₁₄-Aryl unsubstituiert ist oder einen oder mehrere gleiche oder verschiedene Substituenten Z^{3Ar} trägt;
R³¹, R³², R³³, R³⁴, R³⁵, R³⁶, R³⁷ und R³⁸ jeweils unabhängig voneinander aus Wasserstoff, Cyano, Brom und Chlor ausgewählt sind, mit der Maßgabe, dass 1, 2, 3, 4, 5, 6, 7 oder 8 der Substituenten R³¹, R³², R³³, R³⁴, R³⁵, R³⁶, R³⁷ oder R³⁸ für Cyano stehen;
wobei
R³⁹ für Wasserstoff, C₁-C₁₀-Alkyl, C₂-C₁₀-Alkenyl, C₂-C₁₀-Alkinyl, C₃-C₁₂-Cycloalkyl oder C₆-C₁₄-Aryl steht, wobei
C₁-C₁₀-Alkyl, C₂-C₁₀-Alkenyl, C₂-C₁₀-Alkinyl unsubstituiert sind oder einen oder mehrere gleiche oder verschiedene Substituenten R^{3a} tragen, C₃-C₁₂-Cycloalkyl unsubstituiert ist oder einen oder mehrere gleiche oder verschiedene Substituenten R^{3b} trägt und
C₆-C₁₄-Aryl unsubstituiert ist oder einen oder mehrere gleiche oder verschiedene Substituenten R^{3Ar} trägt;
R³¹⁰ und R³¹¹ unabhängig voneinander für Wasserstoff, C₁-C₁₀-Alkyl, C₂-C₁₀-Alkenyl, C₂-C₁₀-Alkinyl, C₃-C₁₂-Cycloalkyl oder C₆-C₁₄-Aryl stehen, wobei
C₁-C₁₀-Alkyl, C₂-C₁₀-Alkenyl, C₂-C₁₀-Alkinyl, unsubstituiert sind oder einen oder mehrere gleiche oder verschiedene Substituenten R^{3a} tragen,
C₃-C₁₂-Cycloalkyl unsubstituiert ist oder einen oder mehrere gleiche oder verschiedene Substituenten R^{3b} trägt und
C₆-C₁₄-Aryl unsubstituiert ist oder einen oder mehrere gleiche oder verschiedene Substituenten R^{3Ar} trägt;
jedes Z^{3a} unabhängig voneinander für Halogen, Hydroxy, NR^{310a}R^{311a}, C₁-C₁₀-Alkoxy, C₁-C₁₀-Halogenalkoxy, C₁-C₁₀-Alkylthio, C₃-C₁₂-Cycloalkyl, C₆-C₁₄-Aryl, C(=O)R^{39a}; C(=O)OR^{39a} oder C(O)NR^{310a}R^{311a} steht, wobei
C₃-C₁₂-Cycloalkyl unsubstituiert ist oder einen oder mehrere gleiche oder verschiedene Substituenten R^{3b} trägt und C₆-C₁₄-Aryl unsubstituiert ist oder einen oder mehrere gleiche oder verschiedene Substituenten R^{3Ar} trägt;
jedes Z^{3b} und jedes Z^{3Ar} unabhängig voneinander für Halogen, Hydroxy, NR^{310a}R^{311a}, C₁-C₁₀-Alkyl, C₁-C₁₀-Alkoxy, C₁-C₁₀-Halogenalkoxy, C₁-C₁₀-Alkylthio, C(=O)R^{39a}; C(=O)OR^{39a} oder C(O)NR^{310a}R^{311a} steht;
jedes R^{3a} unabhängig voneinander für Halogen, Hydroxy, C₁-C₁₀-Alkoxy C₃-C₁₂-Cycloalkyl oder C₆-C₁₄-Aryl steht;
jedes R^{3b} unabhängig voneinander für Halogen, Hydroxy, C₁-C₁₀-Alkyl, C₁-C₁₀-Alkoxy, C₁-C₁₀-Halogenalkoxy, C₁-C₁₀-Alkylthio, C₂-C₁₀-Alkenyl, C₂-C₁₀-Alkinyl, C₃-C₁₂-Cycloalkyl oder C₆-C₁₄-Aryl steht;
jedes R^{3Ar} unabhängig voneinander für Halogen, Hydroxy, C₁-C₁₀-Alkyl, C₁-C₁₀-Alkoxy, C₁-C₁₀-Halogenalkoxy, C₁-C₁₀-Alkylthio, C₂-C₁₀-Alkenyl, C₂-C₁₀-Alkinyl, C₃-C₁₂-Cycloalkyl oder C₆-C₁₄-Aryl steht;
R^{39a} für Wasserstoff, C₁-C₁₀-Alkyl, C₂-C₁₀-Alkenyl, C₂-C₁₀-Alkinyl, C₃-C₁₂-Cycloalkyl oder C₆-C₁₄-Aryl steht; und
R^{310a}, R^{311a} unabhängig voneinander für Wasserstoff, C₁-C₁₀-Alkyl, C₂-C₁₀-Alkenyl, C₂-C₁₀-Alkinyl, C₃-C₁₂-Cycloalkyl oder C₆-C₁₄-Aryl stehen,
und Mischungen davon;
(iii) einer cyanierten Verbindung der Formel (IV) 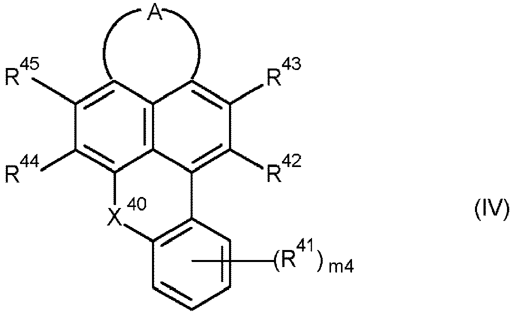 wobei
m4 für 0, 1, 2, 3 oder 4 steht;
jedes R⁴¹ unabhängig voneinander aus Brom, Chlor, Cyano, -NR^{4a}R^{4b}, C₁-C₂₄-Alkyl, C₁-C₂₄-Halogenalkyl, C₁-C₂₄-Alkoxy, C₁-C₂₄-Halogenalkoxy, C₃-C₂₄-Cycloalkyl, Heterocycloalkyl, Heteroaryl, C₆-C₂₄-Aryl, C₆-C₂₄-Aryloxy und C₆-C₂₄-Aryl-C₁-C₁₀-alkylen ausgewählt ist, wobei die Ringe von Cycloalkyl, Heterocycloalkyl, Heteroaryl, Aryl und Aryloxy in den sechs letztgenannten Resten unsubstituiert oder durch 1, 2, 3, 4 oder 5 gleiche oder verschiedene Reste R^{41a} substituiert sind und wobei C₁-C₂₄-Alkyl, C₁-C₂₄-Halogenalkyl, C₁-C₂₄-Alkoxy und die Alkylengruppierung von C₆-C₂₄-Aryl-C₁-C₁₀-alkylen durch eine oder mehrere Gruppen, die aus 0, S und NR^{4c} ausgewählt sind, unterbrochen sein kann;
mindestens einer der Reste R⁴², R⁴³, R⁴⁴ und R⁴⁵ für CN steht und die übrigen Reste unabhängig voneinander aus Wasserstoff, Chlor und Brom ausgewählt sind;
X⁴⁰ für 0, S, SO oder SO₂ steht;
A für einen zweiwertigen Rest steht, der aus zweiwertigen Resten der allgemeinen Formeln (A.1), (A.2), (A.3) und (A.4) ausgewählt ist 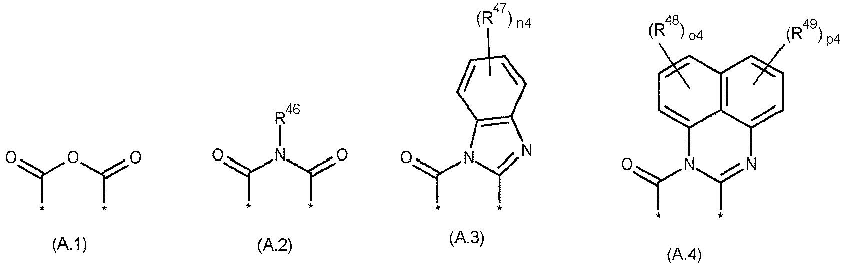 wobei
* in jedem Fall den Punkt von Verknüpfungen mit dem Rest des Moleküls anzeigt;
n4 für 0, 1, 2, 3 oder 4 steht;
o4 für 0, 1, 2 oder 3 steht;
p4 für 0, 1, 2 oder 3 steht;
R⁴⁶ für Wasserstoff, C₁-C₂₄-Alkyl, C₁-C₂₄-Halogenalkyl; C₃-C₂₄-Cycloalkyl, C₆-C₂₄-Aryl oder C₆-C₂₄-Aryl-C₁-C₁₀-alkylen steht, wobei die Ringe von Cycloalkyl, Aryl und Arylalkylen in den drei letztgenannten Resten unsubstituiert oder durch 1, 2, 3, 4 oder 5 gleiche oder verschiedene Reste R^{46a} substituiert sind und wobei C₁-C₂₄-Alkyl, C₁-C₂₄-Halogenalkyl und die Alkylengruppierung von C₆-C₂₄-Aryl-C₁-C₁₀-alkylen durch eine oder mehrere Heteroatome oder Heteroatomgruppen, die aus 0, S und NR^{4c} ausgewählt sind, unterbrochen sein kann;
jedes R⁴⁷ unabhängig voneinander aus Brom, Chlor, Cyano, -NR^{4a}R^{4b}, C₁-C₂₄-Alkyl, C₁-C₂₄-Halogenalkyl, C₁-C₂₄-Alkoxy, C₁-C₂₄-Halogenalkoxy, C₃-C₂₄-Cycloalkyl, Heterocycloalkyl, Heteroaryl, C₆-C₂4-Aryl, C₆-C₂₄-Aryloxy und C₆-C₂₄-Aryl-C₁-C₁₀-alkylen ausgewählt ist, wobei die Ringe von Cycloalkyl, Heterocycloalkyl, Heteroaryl, Aryl und Arylalkylen in den sechs letztgenannten Resten unsubstituiert oder durch 1, 2, 3, 4 oder 5 gleiche oder verschiedene Reste R^{47a} substituiert sind und wobei C₁-C₂₄-Alkyl, C₁-C₂₄-Halogenalkyl, C₁-C₂₄-Alkoxy, C₁-C₂₄-Halogenalkoxy und die Alkylengruppierung von C₆-C₂₄-Aryl-C₁-C₁₀-alkylen durch eine oder mehrere Gruppen, die aus 0, S und NR^{4c} ausgewählt sind, unterbrochen sein kann;
jedes R⁴⁸ unabhängig voneinander aus Brom, Chlor, Cyano, NR^{4a}R^{4b}, C₁-C₂₄-Alkyl, C₁-C₂₄-Halogenalkyl, C₁-C₂₄-Alkoxy, C₁-C₂₄-Halogenalkoxy, C₃-C₂₄-Cycloalkyl, Heterocycloalkyl, Heteroaryl, C₆-C₂4-Aryl, C₆-C₂₄-Aryloxy und C₆-C₂₄-Aryl-C₁-C₁₀-alkylen ausgewählt ist, wobei die Ringe von Cycloalkyl, Heterocycloalkyl, Heteroaryl, Aryl und Arylalkylen in den sechs letztgenannten Resten unsubstituiert oder durch 1, 2, 3, 4 oder 5 gleiche oder verschiedene Reste R^{48a} substituiert sind und wobei C₁-C₂₄-Alkyl, C₁-C₂₄-Halogenalkyl, C₁-C₂₄-Alkoxy, C₁-C₂₄-Halogenalkoxy und die Alkylengruppierung von C₆-C₂₄-Aryl-C₁-C₁₀-alkylen durch eine oder mehrere Gruppen, die aus 0, S und NR^{4c} ausgewählt sind, unterbrochen sein kann;
jedes R⁴⁹ unabhängig voneinander aus Brom, Chlor, Cyano, NR^{4a}R^{4b}, C₁-C₂₄-Alkyl, C₁-C₂₄-Halogenalkyl, C₁-C₂₄-Alkoxy, C₁-C₂₄-Halogenalkoxy, C₃-C₂₄-Cycloalkyl, Heterocycloalkyl, Heteroaryl, C₆-C₂4-Aryl, C₆-C₂₄-Aryloxy und C₆-C₂₄-Aryl-C₁-C₁₀-alkylen ausgewählt ist, wobei die Ringe von Cycloalkyl, Heterocycloalkyl, Heteroaryl, Aryl und Arylalkylen in den sechs letztgenannten Resten unsubstituiert oder durch 1, 2, 3, 4 oder 5 gleiche oder verschiedene Reste R^{49a} substituiert sind und wobei C₁-C₂₄-Alkyl, C₁-C₂₄-Halogenalkyl, C₁-C₂₄-Alkoxy, C₁-C₂₄-Halogenalkoxy und die Alkylengruppierung von C₆-C₂₄-Aryl-C₁-C₁₀-alkylen durch eine oder mehrere Gruppen, die aus 0, S und NR^{4c} ausgewählt sind, unterbrochen sein kann;
R^{41a}, R^{46a}, R^{47a}, R^{48a} und R^{49a} unabhängig voneinander aus C₁-C₂₄-Alkyl, C₁-C₂₄-Fluoralkyl, C₁-C₂₄-Alkoxy, Fluor, Chlor und Brom ausgewählt sind;
R^{4a}, R^{4b} und R^{4c} unabhängig voneinander aus Wasserstoff, C₁-C₂₀-Alkyl, C₃-C₂₄-Cycloalkyl, Heterocycloalkyl, Heteroaryl und C₆-C₂₄-Aryl ausgewählt sind;
und Mischungen davon;
(iv) einer Benzoxanthenverbindung der Formel (V) 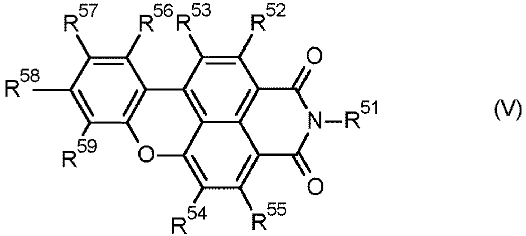 worin
R⁵¹ für Phenyl, das unsubstituiert ist oder 1, 2, 3, 4 oder 5 Substituenten, die aus Halogen, R⁵¹¹, OR⁵⁵², NHR⁵⁵² und NR⁵⁵²R⁵⁵⁷ ausgewählt sind, trägt, steht;
R⁵², R⁵³, R⁵⁴ , R⁵⁵, R⁵⁶, R⁵⁷, R⁵⁸ und R⁵⁹ unabhängig voneinander aus Wasserstoff, Halogen, R⁵⁵³, OR⁵⁵³, NHR⁵⁵³ und NR⁵⁵³R⁵⁵⁴ ausgewählt sind, wobei
R⁵¹¹ aus C₂-C₁₈-Alkyl, C₆-C₂₄-Aryl und Heteroaryl ausgewählt ist;
R⁵⁵² und R⁵⁵⁷ unabhängig voneinander aus C₁-C₁₈-Alkyl, C₆-C₂₄-Aryl und Heteroaryl ausgewählt sind; und
R⁵⁵³ und R⁵⁵⁴ unabhängig voneinander aus C₁-C₁₈-Alkyl, C₆-C₂₄-Aryl und Heteroaryl ausgewählt sind;
und Mischungen davon;
(v) einer fluoreszierenden Verbindung mit mindestens einer Struktureinheit der Formel (VI) 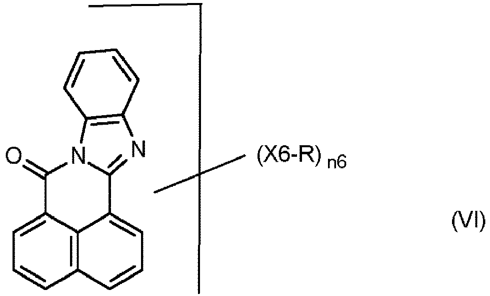 worin eine oder mehrere CH-Gruppen des sechsgliedrigen Rings der dargestellten Benzimidazolstruktur durch Stickstoff ersetzt sein kann und wobei die Symbole folgende Bedeutung haben:
n6 für jede Struktureinheit der Formel (VI) eine Zahl von 0 bis (10-p6); wobei p6 die Zahl der CH-Einheiten ist, die in dem sechsgliedrigen Ring der dargestellten Benzimidazolstruktur durch Stickstoff ersetzt wurden;
X6 eine chemische Bindung, 0, S, SO, SO₂, NR⁶¹; und
R aliphatischer Rest, cycloaliphatischer Rest, Aryl, Heteroaryl, die jeweils beliebige Substituenten tragen können, aromatischer oder heteroaromatischer Ring oder Ringsystem, das jeweils an andere aromatische Ringe der Struktureinheit der Formel (VI) annelliert ist;
F, Cl, Br, CN, H, wenn X6 keine chemische Bindung ist;
wobei zwei Reste R zu einem cyclischen Rest verbunden sein können und
wobei X6 und R für n6 > eins gleich oder verschieden sein können;
R⁶¹ unabhängig voneinander Wasserstoff, C₁-C₁₈-Alkyl oder Cycloalkyl, dessen Kohlenstoffkette eine oder mehrere Gruppierungen -O-, -S-, -CO-, -SO-und/oder -SO₂- enthalten kann und das ein- oder mehrfach substituiert sein kann; Aryl oder Heteroaryl, das ein- oder mehrfach substituiert sein kann;
und Mischungen davon;
und
(vi) einer Perylenverbindung der Formeln (VII), (VIII) oder (IX) 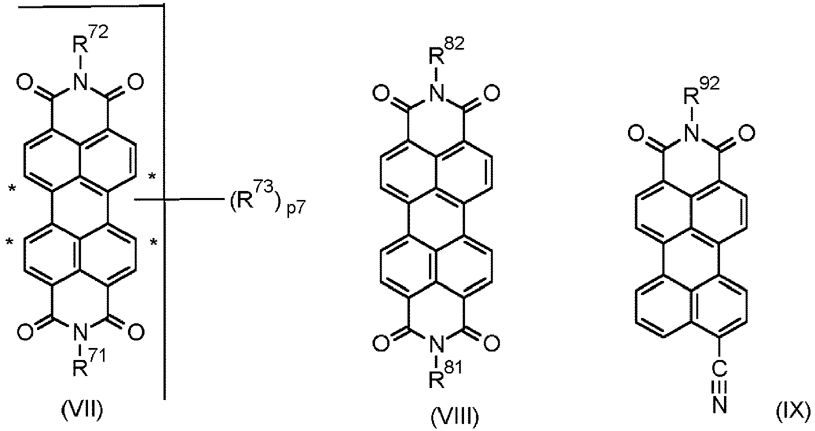 wobei
p7 für 1 bis 4 steht,
R⁷¹ und R⁷² unabhängig voneinander für C₁-C₃₀-Alkyl, C₃-C₈-Cycloalkyl, Aryl, Heteroaryl oder Aryl-C₁-C₁₀-alkylen stehen, wobei der aromatische Ring in den drei letztgenannten Resten unsubstituiert ist oder ein- oder mehrfach durch C₁-C₁₀-Alkyl substituiert ist;
R⁷³ für Aryloxy steht, das unsubstituiert ist oder ein- oder mehrfach durch C₁-C₁₀-Alkyl substituiert ist, wobei die Reste R⁷³ sich an einer oder mehreren der mit * gekennzeichneten Positionen befinden;
R⁸¹ und R⁸² unabhängig voneinander für C₁-C₃₀-Alkyl, C₃-C₈-Cycloalkyl, Aryl, Heteroaryl oder Aryl-C₁-C₁₀-alkylen stehen, wobei der aromatische Ring in den drei letztgenannten Resten unsubstituiert ist oder ein- oder mehrfach durch C₁-C₁₀-Alkyl substituiert ist;
R⁹² für C₁-C₃₀-Alkyl, C₃-C₈-Cycloalkyl, Aryl, Heteroaryl oder Aryl-C₁-C₁₀-alkylen steht, wobei der aromatische Ring in den drei letztgenannten Resten unsubstituiert ist oder ein- oder mehrfach durch C₁-C₁₀-Alkyl substituiert ist;
und Mischungen davon.

7. Farbkonverter nach Anspruch 6, wobei der mindestens eine weitere organische Fluoreszenzfarbstoff aus Verbindungen der Formel (II), Verbindungen der Formel (IV), Verbindungen der Formel (V), Verbindungen mit mindestens einer Struktureinheit der Formel (VI), Verbindungen der Formel (VII) und Mischungen davon ausgewählt ist.

8. Farbkonverter nach einem der Ansprüche 2 bis 7, umfassend als weiteres fluoreszierendes Material mindestens ein anorganisches fluoreszierendes Material, das aus Granaten, Silicaten, Sulfiden, Nitriden und Oxidnitriden ausgewählt ist.

9. Farbkonverter nach einem der Ansprüche 2 bis 8, umfassend mindestens einen Quantenpunkt aus einem kristallinen Halbleitermaterial.

10. Verwendung eines Farbkonverters gemäß einem der Ansprüche 2 bis 9 zur Konversion von durch LEDs oder OLEDs erzeugtem Licht.

11. Verwendung eines Farbkonverters gemäß Anspruch 10 zur Konversion von durch eine UV-LED, durch eine blaue LED oder durch eine weiße LED erzeugtem Licht.

12. Verwendung eines Farbkonverters gemäß einem der Ansprüche 2 bis 9 in Displays.

13. Beleuchtungsvorrichtung mit mindestens einer LED und mindestens einem Farbkonverter nach einem der Ansprüche 2 bis 9, wobei die LED und der Farbkonverter vorzugsweise in einer Remote-Phosphor-Anordnung vorliegen.

14. Vorrichtung, die bei Beleuchtung Strom erzeugt, mit einer Photovoltaikzelle und dem Farbkonverter gemäß einem der Ansprüche 2 bis 9, wobei mindestens ein Teil des nicht von der Photovoltaikzelle absorbierten Lichts von dem Farbkonverter absorbiert wird.

15. Verfahren zur Herstellung von Verbindungen der Formeln Ia und Ib 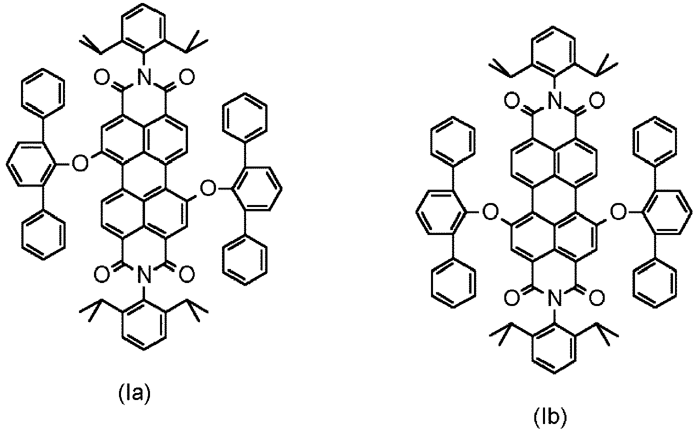 oder Mischungen davon,
bei dem man
a) eine Verbindung der Formeln (1) oder (2) 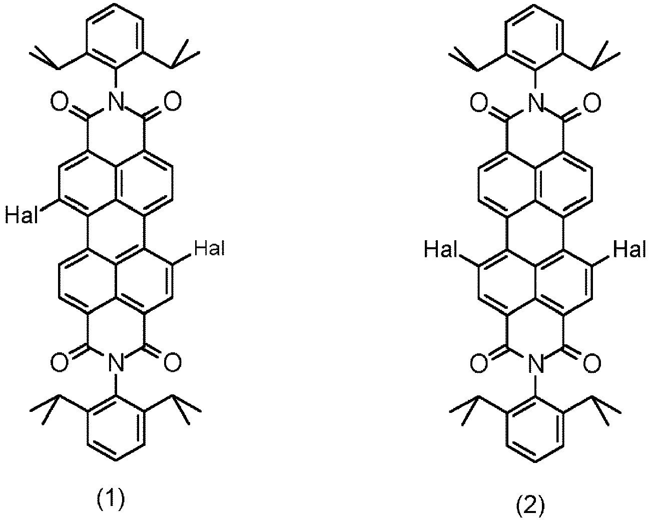 oder eine Mischung davon bereitstellt,
wobei
Hal jeweils alle für Chlor oder jeweils alle für Brom stehen;
b) die Verbindung der Formel (1) oder die Verbindung der Formel (2) oder eine Mischung der Verbindungen der Formeln (1) oder (2), die in Schritt (a) bereitgestellt wurde, in Gegenwart einer anorganischen Base und in Gegenwart eines polaren aprotischen Lösungsmittels mit 2,6-Diphenylphenol zu einer Verbindung der Formel (Ia) oder (Ib) oder einer Mischung davon umsetzt;
c) gegebenenfalls die in Schritt b) erhaltene Reaktionsmischung mindestens einem Aufarbeitungsschritt unterwirft.

16. Verwendung einer Perylenbisimid-Verbindung der Formel Ia oder Ib oder einer Mischung davon gemäß Anspruch 1 in Farbkonvertern zur Konvertierung von von einer Lichtquelle, insbesondere einer aus LEDs und OLEDs ausgewählten Lichtquelle, emittiertem Licht in Licht einer zweiten, längeren Wellenlänge, zur Einfärbung von Lacken, Druckfarben und Kunststoffen, zur Herstellung von elektromagnetische Strahlung absorbierenden und/oder emittierenden wässrigen Polymerdispersionen, zur Datenspeicherung, für optische Label, für Sicherheitslabel in Dokumenten und für den Markenschutz oder als Fluoreszenzlabel für Biomoleküle.

## Revendications

1. Composé bisimide de pérylène représenté par les formules Ia ou Ib 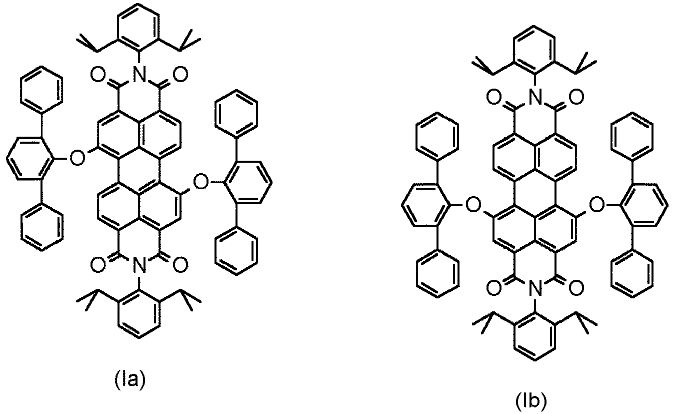 ou mélanges de tels composés.

2. Convertisseur de couleur comprenant au moins un polymère en tant que matrice et au moins un composé représenté par la formule Ia ou Ib ou un mélange de composés représentés par les formules Ia et Ib tels que définis dans la revendication 1 en tant que colorant fluorescent, dans lequel l'au moins un polymère est choisi parmi le polystyrène, le polycarbonate, le poly(méthacrylate de méthyle), la polyvinylpyrrolidone, un polyméthacrylate, le poly(acétate de vinyle), le poly(chlorure de vinyle), le polybutène, une silicone, un polyacrylate, une résine époxyde, le poly(alcool vinylique), un copolymère poly(éthylène-alcool vinylique) (EVA, EVOH), le polyacrylonitrile, le poly(chlorure de vinylidène) (PVDC), le poly(styrène-acrylonitrile) (SAN), le poly(téréphtalate de butylène) (PBT), le poly(téréphtalate d'éthylène) (PET), le poly(butyrate de vinyle) (PVB), le poly(chlorure de vinyle) (PVC), les polyamides, les polyoxyméthylènes, les polyimides, les polyétherimides et les mélanges de ceux-ci.

3. Convertisseur de couleur selon la revendication 2, dans lequel l'au moins un polymère est constitué essentiellement de polystyrène, de polycarbonate ou de poly(téréphtalate d'éthylène).

4. Convertisseur de couleur selon la revendication 2 ou 3, comprenant en tant que colorant fluorescent un composé représenté par la formule (Ia).

5. Convertisseur de couleur selon l'une quelconque des revendications 2 à 4, le convertisseur de couleur comprenant de plus au moins un pigment blanc inorganique en tant que corps de diffusion.

6. Convertisseur de couleur selon l'une quelconque des revendications 2 à 5, comprenant au moins un autre colorant fluorescent organique choisi entre
(i) un composé benzimidazole de naphtalène portant un groupe cyanate représenté par la formule II 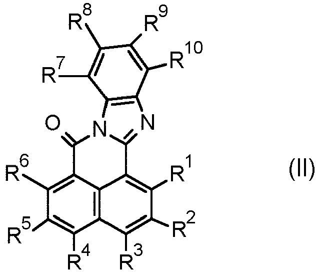 dans lequel
R¹, R², R³, R⁴, R⁵, R⁶, R⁷, R⁸, R⁹ et R¹⁰ sont chacun indépendamment l'atome d'hydrogène, un groupe cyano ou un groupe aryle qui est non substitué ou qui a un ou plusieurs substituants R^{Ar} identiques ou différents, chaque R^{Ar} étant indépendamment choisi parmi les groupes cyano, hydroxyle, mercapto, halogéno, alcoxy en C₁-C₂₀, alkylthio en C₁-C₂₀, nitro, -NR^{Ar2}R^{Ar3}, -NR^{Ar2}COR^{Ar3}, -CONR^{Ar2}R^{Ar3}, -SO₂NR^{Ar2}R^{Ar3}, -COOR^{Ar2}, -SO₃R^{Ar2},
alkyle en C₁-C₃₀, alcényle en C₂-C₃₀, alcynyle en C₂-C₃₀, ces trois derniers radicaux étant non substitués ou portant un ou plusieurs groupes R^{a},
cycloalkyle en C₃-C₈, hétérocyclyle à 3 à 8 chaînons, ces deux derniers radicaux étant non substitués ou portant un ou plusieurs groupes R^{b},
aryle, U-aryle, hétéroaryle et U-hétéroaryle, ces quatre derniers radicaux étant non substitués ou portant un ou plusieurs groupes R^{b},
chaque R^{a} étant indépendamment choisi parmi les groupes cyano, hydroxyle, oxo, mercapto, halogéno, alcoxy en C₁-C₂₀, alkylthio en C₁-C₂₀, nitro, -NR^{Ar2}R^{Ar3}, -NR^{Ar2}COR^{Ar3}, -CONR^{Ar2}R^{Ar3} , -SO₂NR^{Ar2}R^{Ar3}, -COOR^{Ar2}, -SO₃R^{Ar2}, cycloalkyle en C₃-C₈, hétérocyclyle à 3 à 8 chaînons, aryle et hétéroaryle,
les radicaux cycloalkyle, hétérocyclyle, aryle et hétéroaryle étant non substitués ou portant un ou plusieurs groupes R^{b} ;
chaque R^{b} étant indépendamment choisi parmi les groupes cyano, hydroxyle, oxo, mercapto, halogéno, alcoxy en C₁-C₂₀, alkylthio en C₁-C₂₀, nitro, -NR^{Ar2}R^{Ar3}, -NR^{Ar2}COR^{Ar3}, -CONR^{Ar2}R^{Ar3} , -SO₂NR^{Ar2}R^{Ar3}, -COOR^{Ar2}, -SO₃R^{Ar2}, alkyle en C₁-C₁₈, alcényle en C₂-C₁₈, alcynyle en C₂-C₁₈, cycloalkyle en C₃-C₈, hétérocyclyle à 3 à 8 chaînons, aryle et hétéroaryle,
ces quatre derniers radicaux étant non substitués ou portant un ou plusieurs groupes R^{b1},
chaque R^{b1} étant indépendamment choisi parmi les groupes cyano, hydroxyle, mercapto, oxo, nitro, halogéno, -NR^{Ar2}R^{Ar3}, -NR^{Ar2}COR^{Ar3}, -CONR^{Ar2}R^{Ar3}, -SO₂NR^{Ar2}R^{Ar3}, -COOR^{Ar2}, -SO₃R^{Ar2}, -SO₃R^{Ar2}, alkyle en C₁-C₁₈, alcényle en C₂-C₁₈, alcynyle en C₂-C₁₈, alcoxy en C₁-C₁₂, alkylthio en C₁-C₁₂,
U étant une fraction -O-, -S-, -NR^{Ar1}-, -CO-, -SO- ou -SO₂- ;
R^{Ar1}, R^{Ar2}, R^{Ar3} étant chacun indépendamment l'atome d'hydrogène ou un groupe alkyle en C₁-C₁₈, cycloalkyle à 3 à 8 chaînons, hétérocyclyle à 3 à 8 chaînons, aryle ou hétéroaryle, le groupe alkyle étant non substitué ou portant un ou plusieurs groupes R^{a},
les groupes cycloalkyle à 3 à 8 chaînons, hétérocyclyle à 3 à 8 chaînons, aryle et hétéroaryle étant non substitués ou portant un ou plusieurs groupes R^{b} ;
à condition que le composé représenté par la formule II comprenne au moins un groupe cyano,
ou des mélanges de tels composés ;
(ii) un composé de pérylène portant un groupe cyanate représenté par la formule (III) 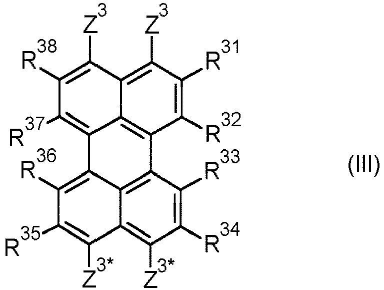 dans lequel
l'un des substituants Z³ est un groupe cyano et l'autre substituant Z³ est un groupe CO₂R³⁹, CONR³¹⁰R³¹¹, alkyle en C₁-C₁₈, alcényle en C₂-C₁₈, alcynyle en C₂-C₁₈, cycloalkyle en C₃-C₁₂ ou aryle en C₆-C₁₄,
les groupes alkyle en C₁-C₁₈, alcényle en C₂-C₁₈, alcynyle en C₂-C₁₈ étant non substitués ou portant un ou plusieurs substituants Z^{3a} identiques ou différents,
le groupe cycloalkyle en C₃-C₁₂ étant non substitué ou portant un ou plusieurs substituants Z^{3b} identiques ou différents et le groupe aryle en C₆-C₁₄ étant non substitué ou portant un ou plusieurs substituants Z^{3Ar} identiques ou différents ;
l'un des substituants Z^{3*} est un groupe cyano et l'autre substituant Z^{3*} est un groupe CO₂R³⁹, CONR³¹⁰R³¹¹, alkyle en C₁-C₁₈, alcényle en C₂-C₁₈, alcynyle en C₂-C₁₈, cycloalkyle en C₃-C₁₂ ou aryle en C₆-C₁₄,
les groupes alkyle en C₁-C₁₈, alcényle en C₂-C₁₈, alcynyle en C₂-C₁₈ étant non substitués ou portant un ou plusieurs substituants Z^{3a} identiques ou différents,
le groupe cycloalkyle en C₃-C₁₂ étant non substitué ou portant un ou plusieurs substituants Z^{3b} identiques ou différents et le groupe aryle en C₆-C₁₄ étant non substitué ou portant un ou plusieurs substituants Z^{3Ar} identiques ou différents ;
R³¹, R³², R³³, R³⁴, R³⁵, R³⁶, R³⁷ et R³⁸ sont chacun indépendamment choisis parmi l'atome d'hydrogène et les groupes cyano, bromo et chloro,
à condition que 1, 2, 3, 4, 5, 6, 7 ou 8 des substituants R³¹, R³², R³³, R³⁴, R³⁵, R³⁶, R³⁷ ou R³⁸ soient des groupes cyano ;
R³⁹ étant l'atome d'hydrogène ou un groupe alkyle en C₁-C₁₀, alcényle en C₂-C₁₀, alcynyle en C₂-C₁₀, cycloalkyle en C₃-C₁₂ ou aryle en C₆-C₁₄,
les groupes alkyle en C₁-C₁₀, alcényle en C₂-C₁₀, alcynyle en C₂-C₁₀ étant non substitués ou portant un ou plusieurs substituants R^{3a} identiques ou différents,
le groupe cycloalkyle en C₃-C₁₂ étant non substitué ou portant un ou plusieurs substituants R^{3b} identiques ou différents et
le groupe aryle en C₆-C₁₄ étant non substitué ou portant un ou plusieurs substituants R^{3Ar} identiques ou différents ;
R³¹⁰ et R³¹¹ étant chacun indépendamment l'atome d'hydrogène ou un groupe alkyle en C₁-C₁₀, alcényle en C₂-C₁₀, alcynyle en C₂-C₁₀, cycloalkyle en C₃-C₁₂ ou aryle en C₆-C₁₄,
les groupes alkyle en C₁-C₁₀, alcényle en C₂-C₁₀, alcynyle en C₂-C₁₀ étant non substitués ou portant un ou plusieurs substituants R^{3a} identiques ou différents,
le groupe cycloalkyle en C₃-C₁₂ étant non substitué ou portant un ou plusieurs substituants R^{3b} identiques ou différents et
le groupe aryle en C₆-C₁₄ étant non substitué ou portant un ou plusieurs substituants R^{3Ar} identiques ou différents ;
chaque Z^{3a} étant indépendamment un groupe halogéno, hydroxyle, NR^{310a}R^{311a}, alcoxy en C₁-C₁₀, halogénoalcoxy en C₁-C₁₀, alkylthio en C₁-C₁₀, cycloalkyle en C₃-C₁₂, aryle en C₆-C₁₄, C(=O)R^{39a} ; C(=O)OR^{39a} ou C (0) NR^{310a}R^{311a},
le groupe cycloalkyle en C₃-C₁₂ étant non substitué ou portant un ou plusieurs substituants R^{3b} identiques ou différents et
le groupe aryle en C₆-C₁₄ étant non substitué ou portant un ou plusieurs substituants R^{3Ar} identiques ou différents ;
chaque Z^{3b} et chaque Z^{3Ar} étant indépendamment un groupe halogéno, hydroxyle, NR^{310a}R^{311a}, alkyle en C₁-C₁₀, alcoxy en C₁-C₁₀, halogénoalcoxy en C₁-C₁₀, alkylthio en C₁-C₁₀, C(=O)R^{39a} ; C(=O)OR^{39a} ou C(O)NR^{310a}R^{311a} ;
chaque R^{3a} étant indépendamment un groupe halogéno, hydroxyle, alcoxy en C₁-C₁₀, cycloalkyle en C₃-C₁₂ ou aryle en C₆-C₁₄ ;
chaque R^{3b} étant indépendamment un groupe halogéno, hydroxyle, alkyle en C₁-C₁₀, alcoxy en C₁-C₁₀, halogénoalcoxy en C₁-C₁₀, alkylthio en C₁-C₁₀, alcényle en C₂-C₁₀, alcynyle en C₂-C₁₀, cycloalkyle en C₃-C₁₂ ou aryle en C₆-C₁₄ ;
chaque R^{3Ar} étant indépendamment un groupe halogéno, hydroxyle, alkyle en C₁-C₁₀, alcoxy en C₁-C₁₀, halogénoalcoxy en C₁-C₁₀, alkylthio en C₁-C₁₀, alcényle en C₂-C₁₀, alcynyle en C₂-C₁₀, cycloalkyle en C₃-C₁₂ ou aryle en C₆-C₁₄ ;
R^{39a} étant l'atome d'hydrogène ou un groupe alkyle en C₁-C₁₀, alcényle en C₂-C₁₀, alcynyle en C₂-C₁₀, cycloalkyle en C₃-C₁₂ ou aryle en C₆-C₁₄ ; et
R^{310a}, R^{311a} étant chacun indépendamment l'atome d'hydrogène ou un groupe alkyle en C₁-C₁₀, alcényle en C₂-C₁₀, alcynyle en C₂-C₁₀, cycloalkyle en C₃-C₁₂ ou aryle en C₆-C₁₄,
et des mélanges de tels composés,
(iii) un composé portant un groupe cyanate représenté par la formule (IV) 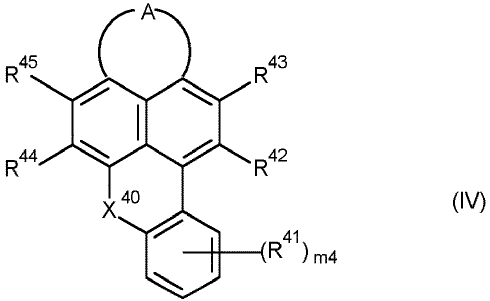 dans lequel
m4 vaut 0, 1, 2, 3 ou 4 ;
chaque R⁴¹ indépendamment des autres est choisi parmi les groupes bromo, chloro, cyano, -NR^{4a}R^{4b}, alkyle en C₁-C₂₄, halogénoalkyle en C₁-C₂₄, alcoxy en C₁-C₂₄, halogénoalcoxy en C₁-C₂₄, cycloalkyle en C₃-C₂₄, hétérocycloalkyle, hétéroaryle, aryle en C₆-C₂₄, aryloxy en C₆-C₂₄, (aryl en C₆-C₂₄)-(alkylène en C₁-C₁₀) ,
les cycles des groupes cycloalkyle, hétérocycloalkyle, hétéroaryle, aryle, aryloxy dans les six derniers radicaux mentionnés étant non substitués ou substitués par 1, 2, 3, 4 ou 5 radicaux R^{41a} identiques ou différents et
les groupes alkyle en C₁-C₂₄, halogénoalkyle en C₁-C₂₄, alcoxy en C₁-C₂₄ et la fraction alkylène du groupe (aryl en C₆-C₂₄)-(alkylène en C₁-C₁₀) pouvant être interrompus par un ou plusieurs groupes choisis entre 0, S et NR^{4c} ;
au moins l'un des radicaux R⁴², R⁴³, R⁴⁴ et R⁴⁵ est CN et les radicaux restants, indépendamment les uns des autres, sont choisis parmi les atomes d'hydrogène, de chlore et de brome ;
X⁴⁰ est 0, S, SO ou SO₂ ;
A est un radical divalent choisi parmi les radicaux divalents représentés par les formules générales (A.1), (A.2), (A.3) et (A.4) 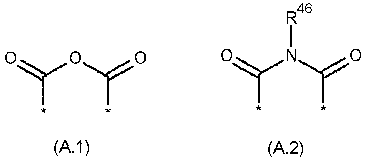 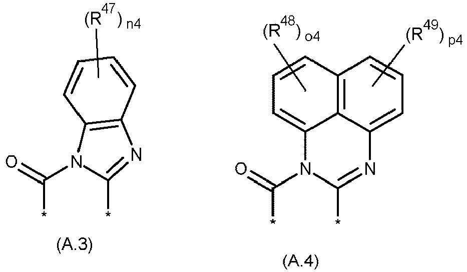 dans lesquels
* dans chaque cas désigne le point des liaisons au reste de la molécule ;
n4 vaut 0, 1, 2, 3 ou 4 ;
o4 vaut 0, 1, 2 ou 3 ;
p4 vaut 0, 1, 2 ou 3 ;
R⁴⁶ est l'atome d'hydrogène ou un groupe alkyle en C₁-C₂₄, halogénoalkyle en C₁-C₂₄, cycloalkyle en C₃-C₂₄, aryle en C₆-C₂₄ ou (aryl en C₆-C₂₄)-(alkylène en C₁-C₁₀) , les cycles des groupes cycloalkyle, aryle et aryl-alkylène dans les trois derniers radicaux mentionnés étant non substitués ou substitués par 1, 2, 3, 4 ou 5 radicaux R^{46a} identiques ou différents et
les groupes alkyle en C₁-C₂₄, halogénoalkyle en C₁-C₂₄ et la fraction alkylène du groupe (aryl en C₆-C₂₄)-(alkylène en C₁-C₁₀) pouvant être interrompus par un ou plusieurs hétéroatomes ou groupes hétéroatomiques choisis entre 0, S et NR^{4c} ;
chaque R⁴⁷ indépendamment des autres est choisi parmi les groupes bromo, chloro, cyano, -NR^{4a}R^{4b}, alkyle en C₁-C₂₄, halogénoalkyle en C₁-C₂₄, alcoxy en C₁-C₂₄, halogénoalcoxy en C₁-C₂₄, cycloalkyle en C₃-C₂₄, hétérocycloalkyle, hétéroaryle, aryle en C₆-C₂₄, aryloxy en C₆-C₂₄, (aryl en C₆-C₂₄)-(alkylène en C₁-C₁₀) ,
les cycles des groupes cycloalkyle, hétérocycloalkyle, hétéroaryle, aryle et aryl-alkylène dans les six derniers radicaux mentionnés étant non substitués ou substitués par 1, 2, 3, 4 ou 5 radicaux R^{47a} identiques ou différents et les groupes alkyle en C₁-C₂₄, halogénoalkyle en C₁-C₂₄, alcoxy en C₁-C₂₄, halogénoalcoxy en C₁-C₂₄ et la fraction alkylène du groupe (aryl en C₆-C₂₄) - (alkylène en C₁-C₁₀) pouvant être interrompus par un ou plusieurs groupes choisis entre 0, S et NR^{4c} ;
chaque R⁴⁸ indépendamment des autres est choisi parmi les groupes bromo, chloro, cyano, NR^{4a}R^{4b}, alkyle en C₁-C₂₄, halogénoalkyle en C₁-C₂₄, alcoxy en C₁-C₂₄, halogénoalcoxy en C₁-C₂₄, cycloalkyle en C₃-C₂₄, hétérocycloalkyle, hétéroaryle, aryle en C₆-C₂₄, aryloxy en C₆-C₂₄, (aryl en C₆-C₂₄)-(alkylène en C₁-C₁₀),
les cycles des groupes cycloalkyle, hétérocycloalkyle, hétéroaryle, aryle et aryl-alkylène dans les six derniers radicaux mentionnés étant non substitués ou substitués par 1, 2, 3, 4 ou 5 radicaux R^{48a} identiques ou différents et les groupes alkyle en C₁-C₂₄, halogénoalkyle en C₁-C₂₄, alcoxy en C₁-C₂₄, halogénoalcoxy en C₁-C₂₄ et la fraction alkylène du groupe (aryl en C₆-C₂₄)-(alkylène en C₁-C₁₀) pouvant être interrompus par un ou plusieurs groupes choisis entre 0, S et NR^{4c} ;
chaque R⁴⁹ indépendamment des autres est choisi parmi les groupes bromo, chloro, cyano, NR^{4a}R^{4b}, alkyle en C₁-C₂₄, halogénoalkyle en C₁-C₂₄, alcoxy en C₁-C₂₄, halogénoalcoxy en C₁-C₂₄, cycloalkyle en C₃-C₂₄, hétérocycloalkyle, hétéroaryle, aryle en C₆-C₂₄, aryloxy en C₆-C₂₄, (aryl en C₆-C₂₄) - (alkylène en C₁-C₁₀),
les cycles des groupes cycloalkyle, hétérocycloalkyle, hétéroaryle, aryle et aryl-alkylène dans les six derniers radicaux mentionnés étant non substitués ou substitués par 1, 2, 3, 4 ou 5 radicaux R^{49a} identiques ou différents et les groupes alkyle en C₁-C₂₄, halogénoalkyle en C₁-C₂₄, alcoxy en C₁-C₂₄, halogénoalcoxy en C₁-C₂₄ et la fraction alkylène du groupe (aryl en C₆-C₂₄) - (alkylène en C₁-C₁₀) pouvant être interrompus par un ou plusieurs groupes choisis entre 0, S et NR^{4c} ;
R^{41a}, R^{46a}, R^{47a}, R^{48a}, R^{49a} sont indépendamment les uns des autres choisis parmi les groupes alkyle en C₁-C₂₄, fluoroalkyle en C₁-C₂₄, alcoxy en C₁-C₂₄, fluoro, chloro et bromo ;
R^{4a}, R^{4b}, R^{4c} sont indépendamment les uns des autres choisis parmi l'atome d'hydrogène et les groupes alkyle en C₁-C₂₀, cycloalkyle en C₃-C₂₄, hétérocycloalkyle, hétéroaryle et aryle en C₆-C₂₄ ;
et des mélanges de tels composés ;
(iv) un composé de benzoxanthène représenté par la formule (V) 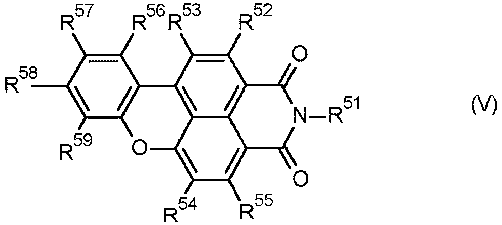 dans lequel
R⁵¹ est un groupe phényle qui est non substitué ou qui porte 1, 2, 3, 4 ou 5 substituants choisis parmi les groupes halogéno, R⁵¹¹, OR⁵⁵², NHR⁵⁵² et NR⁵⁵²R⁵⁵⁷ ;
R⁵², R⁵³, R⁵⁴, R⁵⁵, R⁵⁶, R⁵⁷, R⁵⁸ et R⁵⁹ sont indépendamment les uns des autres choisis parmi l'atome d'hydrogène et les groupes halogéno, R⁵⁵³, OR⁵⁵³, NHR⁵⁵³ et NR⁵⁵³R⁵⁵⁴,
R⁵¹¹ étant choisi parmi les groupes alkyle en C₂-C₁₈, aryle en C₆-C₂₄ et hétéroaryle ;
R⁵⁵² et R⁵⁵⁷ étant indépendamment l'un de l'autre choisis parmi les groupes alkyle en C₁-C₁₈, aryle en C₆-C₂₄ et hétéroaryle ; et
R⁵⁵³ et R⁵⁵⁴ étant indépendamment l'un de l'autre choisis parmi les groupes alkyle en C₁-C₁₈, aryle en C₆-C₂₄ et hétéroaryle ;
et des mélanges de tels composés ;
(v) un composé fluorescent comprenant au moins un motif de structure de formule (VI) 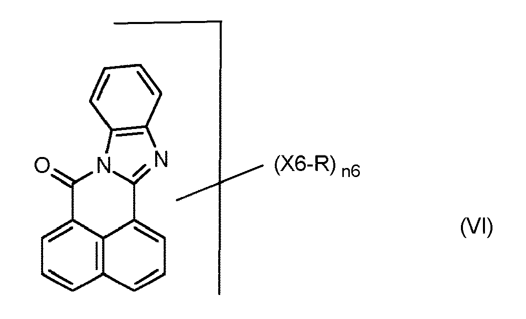 un ou plusieurs groupes CH du cycle à six chaînons de la structure benzimidazole représentée pouvant être remplacés par un atome d'azote et les symboles étant chacun définis de la façon suivants :
n6 est un nombre de 0 à (10-p6) pour chaque motif de structure représenté par la formule (VI) ; p6 étant le nombre de motifs CH qui ont été remplacés par des atomes d'azote dans le cycle à six chaînons de la structure benzimidazole représentée
X6 est une liaison chimique, 0, S, SO, SO₂, NR⁶¹ ; et
R est un radical aliphatique, un radical cycloaliphatique, un groupe aryle, un groupe hétéroaryle, chacun de ceux-ci pouvant porter n'importe quels substituants souhaités, un noyau ou système cyclique aromatique ou hétéroaromatique, chacun de ceux-ci étant condensé à d'autres noyaux aromatiques du motif de structure représenté par la formule (VI)
est F, Cl, Br, CN, H lorsque X6 n'est pas une liaison chimique ;
deux radicaux R pouvant être unis pour donner un radical cyclique et
X6 et R, lorsque n6 > un, pouvant être identiques ou différents ;
R⁶¹ est indépendamment l'atome d'hydrogène ou un groupe alkyle en C₁-C₁₈ ou cycloalkyle, dont la chaîne carbonée peut comprendre une ou plusieurs fractions -0-, -S-, -CO-, -SO-et/ou -SO₂- et qui peut être monosubstitué ou polysubstitué ; un groupe aryle ou hétéroaryle qui peut être monosubstitué ou polysubstitué ;
et des mélanges de tels composés ;
et
(vi) un composé de pérylène représenté par les formules (VII), (VIII) ou (IX) 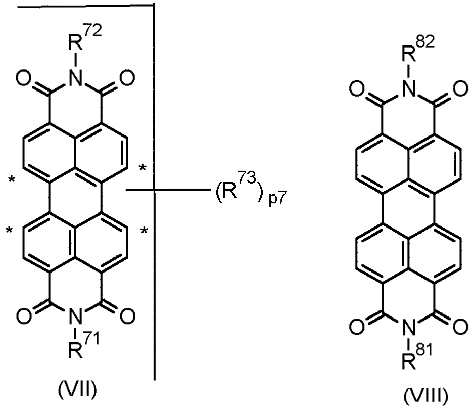 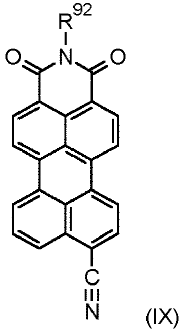
p7 valant 1 à 4,
R⁷¹, R⁷² étant chacun indépendamment un groupe alkyle en C₁-C₃₀, cycloalkyle en C₃-C₈, aryle, hétéroaryle, aryl- (alkylène en C₁-C₁₀), le noyau aromatique dans ces trois derniers radicaux étant non substitué ou monosubstitué ou polysubstitué par alkyle en C₁-C₁₀ ;
R⁷³ étant un groupe aryloxy qui est non substitué ou monosubstitué ou polysubstitué par alkyle en C₁-C₁₀, les radicaux R⁷³ étant en une ou plusieurs des positions indiquées par * ;
R⁸¹, R⁸² étant chacun indépendamment un groupe alkyle en C₁-C₃₀, cycloalkyle en C₃-C₈, aryle, hétéroaryle, aryl- (alkylène en C₁-C₁₀), le noyau aromatique dans ces trois derniers radicaux étant non substitué ou monosubstitué ou polysubstitué par alkyle en C₁-C₁₀ ;
R⁹² étant un groupe alkyle en C₁-C₃₀, cycloalkyle en C₃-C₈, aryle, hétéroaryle, aryl-(alkylène en C₁-C₁₀) , le noyau aromatique dans ces trois derniers radicaux étant non substitué ou monosubstitué ou polysubstitué par alkyle en C₁-C₁₀ ;
et des mélanges de tels composés.

7. Convertisseur de couleur selon la revendication 6, dans lequel l'au moins un autre colorant fluorescent organique est choisi parmi les composés de formule (II), les composés de formule (IV), les composés de formule (V), les composés comprenant au moins un motif de structure de formule (VI), les composés de formule (VII) et les mélanges de ceux-ci.

8. Convertisseur de couleur selon l'une quelconque des revendications 2 à 7, comprenant en tant qu'autre matériau fluorescent au moins un matériau fluorescent inorganique choisi parmi les grenats, les silicates, les sulfures, les nitrures et les oxynitrures.

9. Convertisseur de couleur selon l'une quelconque des revendications 2 à 8 comprenant au moins un point quantique d'un matériau semi-conducteur cristallin.

10. Utilisation d'un convertisseur de couleur tel que défini dans l'une quelconque des revendications 2 à 9, pour la conversion de lumière produite par des DEL ou des DELO.

11. Utilisation d'un convertisseur de couleur tel que défini dans la revendication 10 pour la conversion de lumière produite par une DEL UV, par une DEL bleue ou par une DEL blanche.

12. Utilisation d'un convertisseur de couleur tel que défini dans l'une quelconque des revendications 2 à 9 dans des afficheurs.

13. Dispositif d'éclairage comprenant au moins une DEL et au moins un convertisseur de couleur selon l'une quelconque des revendications 2 à 9, la DEL et le convertisseur de couleur étant de préférence en un agencement de luminophore à distance.

14. Dispositif produisant de l'énergie électrique lorsqu'il est éclairé comprenant une cellule photovoltaïque et le convertisseur de couleur tel que défini dans l'une quelconque des revendications 2 à 9, au moins une partie de la lumière non absorbée par la cellule photovoltaïque étant absorbée par le convertisseur de couleur.

15. Procédé pour la préparation de composés représentés par les formules la et Ib 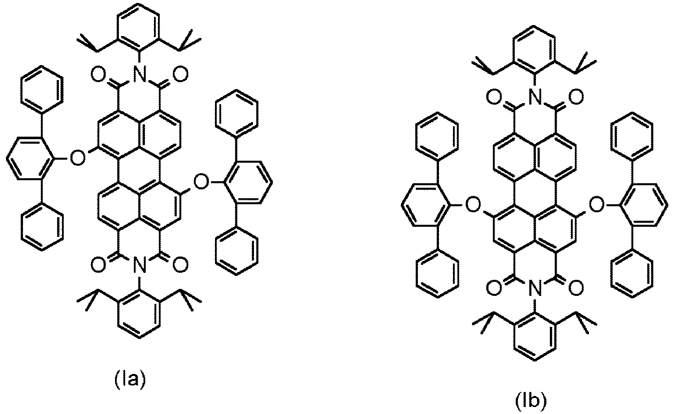 ou de mélanges de ceux-ci,
consistant à
a) fournir un composé représenté par les formules (1) ou (2) 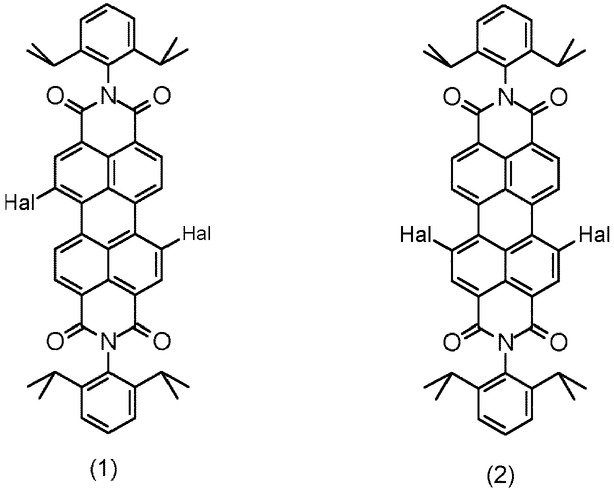 ou un mélange de tels composés
les Hal étant tous des atomes de chlore ou tous des atomes de brome ;
b) faire réagir le composé de formule (1) ou le composé de formule (2) ou un mélange de composés représentés par les formules (1) ou (2) fournis dans l'étape (a) avec du 2,6-diphénylphénol en présence d'une base inorganique et en présence d'un solvant polaire aprotique pour obtenir un composé de formule (Ia) ou (Ib) ou un mélange de tels composés ;
c) éventuellement, soumettre le mélange réactionnel obtenu dans l'étape b) à au moins une étape de traitement conclusif.

16. Utilisation d'un composé bisimide de pérylène représenté par la formule la ou Ib ou d'un mélange de tels composés tels que définis dans la revendication 1, dans des convertisseurs de couleur pour la conversion de lumière émise à partir d'une source de lumière, en particulier une source de lumière choisie parmi les DEL et les DELO, en lumière ayant une seconde longueur d'onde plus grande, pour la coloration de revêtements, d'encres d'impression et de plastiques, la production de dispersions aqueuses de polymère qui absorbent et/ou émettent un rayonnement électromagnétique, pour le stockage de données, pour des étiquettes otiques, pour des étiquettes de sécurité dans des documents et pour la protection de marque ou en tant que marqueur fluorescent pour des biomolécules.
